(19) Europäisches Patentamt
European Patent Office
Office européen des brevets

(11) EP 2 531 484 B1

(12) **EUROPEAN PATENT SPECIFICATION**

(45) Date of publication and mention of the grant of the patent:
**17.08.2016 Bulletin 2016/33**

(21) Application number: **11701517.2**

(22) Date of filing: **01.02.2011**

(51) Int Cl.:
***C07C 211/52*** (2006.01) ***C07C 237/42*** (2006.01)
***C07C 237/44*** (2006.01) ***C07C 255/58*** (2006.01)
***C07C 255/60*** (2006.01) ***A01N 37/18*** (2006.01)
***A01N 43/06*** (2006.01) ***A01N 43/08*** (2006.01)
***A01N 43/78*** (2006.01) ***C07D 277/44*** (2006.01)
***C07D 307/68*** (2006.01) ***C07D 333/20*** (2006.01)
***A01P 5/00*** (2006.01) ***A01P 7/00*** (2006.01)
***A01P 9/00*** (2006.01)

(86) International application number:
**PCT/EP2011/051342**

(87) International publication number:
**WO 2011/095462 (11.08.2011 Gazette 2011/32)**

(54) **INSECTICIDAL COMPOUNDS**
INSEKTIZIDVERBINDUNGEN
COMPOSÉS INSECTICIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.02.2010 EP 10152530**

(43) Date of publication of application:
**12.12.2012 Bulletin 2012/50**

(73) Proprietor: **Syngenta Participations AG**
**4058 Basel (CH)**

(72) Inventors:
- **JUNG, Pierre Joseph Marcel**
  **CH-4332 Stein (CH)**
- **GODFREY, Christopher Richard Ayles**
  **CH-4332 Stein (CH)**
- **HUETER, Ottmar Franz**
  **CH-4332 Stein (CH)**
- **RENOLD, Peter**
  **CH-4332 Stein (CH)**

(74) Representative: **Syngenta International AG**
**WRO B8-Z1-26**
**Schwarzwaldallee 215**
**4058 Basel (CH)**

(56) References cited:
**WO-A1-2008/000438** **WO-A2-2009/049845**
**WO-A2-2010/015355**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 531 484 B1

## Description

The present invention relates to certain bisamide derivatives, to processes and intermediates for preparing them, to insecticidal, acaricidal, nematicidal or molluscicidal compositions comprising them and to methods of using them to combat and control insect, acarine, nematode or mollusc pests.

Bisamide derivatives having insecticidal properties are disclosed in WO2010015355, EP1714958, JP2006306771, WO06137376, WO06137395, WO07017075 and WO2009049845. There exists a need for alternative methods of control of pests. Preferably, new compounds may possess improved insecticidal properties, such as improved efficacy, improved selectivity, lower tendency to generate resistance or activity against a broader range of pests. Compounds may be more advantageously formulated or provide more efficient delivery and retention at sites of action, or may be more readily biodegradable.

It has surprisingly been found that certain bisamide derivatives which are substituted by an arylperfluoroalkyl group or a heterocyclylperfluoroalkyl group have beneficial insecticidal properties.

Accordingly, the present invention provides a compound of formula (I):

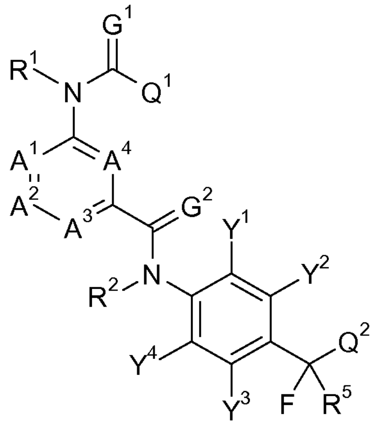

(I)

wherein

$A^1$, $A^2$, $A^3$ and $A^4$ are each independently C-X or nitrogen, wherein each X may be the same or different, and provided that no more than two of $A^1$, $A^2$, $A^3$ and $A^4$ are nitrogen;
X is hydrogen, halogen, cyano, $C_1$-$C_4$alkyl, $C_1$-$C_4$haloalkyl or $C_1$-$C_4$alkoxy;
$G^1$ and $G^2$ are each independently oxygen or sulfur;
$Y^1$ and $Y^4$ are each independently halogen, cyano, $C_1$-$C_4$alkyl, $C_1$-$C_4$haloalkyl, $C_1$-$C_4$alkoxy-$C_1$-$C_4$alkyl, $C_1$-$C_3$alkylthio, $C_1$-$C_3$haloalkylthio, $C_1$-$C_3$alkylsulfinyl, $C_1$-$C_3$haloalkylsulfinyl, $C_1$-$C_3$alkylsulfonyl or $C_1$-$C_3$haloalkylsulfonyl;
$Y^2$ and $Y^3$ are each independently hydrogen, halogen or $C_1$-$C_4$alkyl;
$Q^1$ is aryl or heterocyclyl, each optionally substituted by one to five $R^3$ substituents, which may be the same or different;
$Q^2$ is aryl or heterocyclyl, each optionally substituted by one to five $R^4$ substituents, which may be the same or different;
$R^1$ and $R^2$ are each independently hydrogen, $C_1$-$C_4$alkyl or $C_1$-$C_4$alkylcarbonyl;
$R^3$ is halogen, cyano, nitro, amino, $C_1$-$C_4$alkyl, $C_1$-$C_4$haloalkyl, $C_2$-$C_4$alkenyl, $C_2$-$C_4$haloalkenyl, $C_2$-$C_4$alkynyl, $C_2$-$C_4$haloalkynyl, $C_3$-$C_8$cycloalkyl, $C_3$-$C_8$halocycloalkyl, hydroxy, $C_1$-$C_3$alkoxy, $C_1$-$C_3$haloalkoxy, $C_1$-$C_3$alkylthio, $C_1$-$C_3$haloalkylthio, $C_1$-$C_3$alkylsulfinyl, $C_1$-$C_3$haloalkylsulfinyl, $C_1$-$C_3$alkylsulfonyl, $C_1$-$C_3$haloalkylsulfonyl, *N*-$C_1$-$C_4$alkylamino, *N*,*N*-di-($C_1$-$C_4$alkyl)amino, $C_1$-$C_4$alkylcarbonyl, $C_1$-$C_4$alkylcarbonyloxy, $C_1$-$C_4$alkoxycarbonyl, $C_1$-$C_4$alkylcarbonylamino, arylcarbonylamino or phenyl;
$R^4$ is halogen, cyano, nitro, $C_1$-$C_4$alkyl, $C_1$-$C_4$haloalkyl, $C_2$-$C_4$alkenyl, $C_2$-$C_4$haloalkenyl, $C_2$-$C_4$alkynyl, $C_2$-$C_4$haloalkynyl, $C_3$-$C_8$cycloalkyl, $C_3$-$C_8$halocycloalkyl, hydroxy, $C_1$-$C_3$alkoxy, $C_1$-$C_3$haloalkoxy, $C_1$-$C_3$alkylthio, $C_1$-$C_3$haloalkylthio, $C_1$-$C_3$alkylsulfinyl, $C_1$-$C_3$haloalkylsulfinyl, $C_1$-$C_3$alkylsulfonyl, $C_1$-$C_3$haloalkylsulfonyl, amino, $C_1$-$C_4$alkylamino, *N*,*N*-di-($C_1$-$C_4$alkyl)amino, carboxyl (-COOH), $C_1$-$C_4$alkoxycarbonyl, $C_1$-$C_4$alkylcarbonylamino, N-$C_1$-$C_4$alkylaminocarbonyl, N,N-di($C_1$-$C_4$alkyl)aminocarbonyl; and
$R^5$ is $C_1$-$C_6$perfluoroalkyl;

or a salt or *N*-oxide thereof.
provided that the compound of formula (I) is not

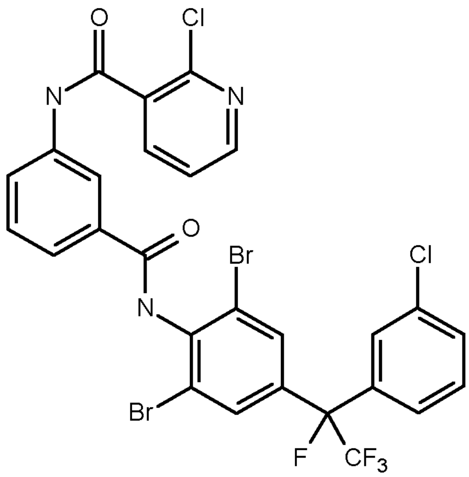

or

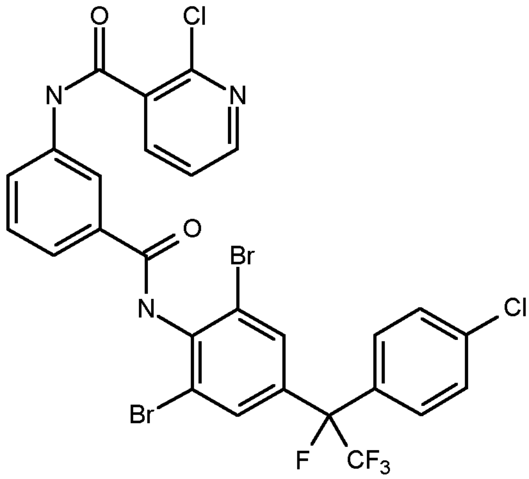

The compounds of formula (I) may exist in different geometric or optical isomers (enantiomers and/or diastereoisomers) or tautomeric forms. This invention covers all such isomers and tautomers and mixtures thereof in all proportions as well as isotopic forms such as deuterated compounds.

Unless otherwise indicated, alkyl, on its own or as part of another group, such as alkoxy, alkylcarbonyl or alkoxycarbonyl, may be straight or branched chain and may preferably contain from 1 to 6 carbon atoms, more preferably 1 to 4, and most preferably 1 to 3. Examples of alkyl include methyl, ethyl, *n*-propyl, *iso*-propyl, *n*-butyl, *sec*-butyl, *iso*-butyl and *tert*-butyl.

Unless otherwise indicated, alkenyl and alkynyl, on their own or as part of another substituent, may be straight or branched chain and may preferably contain 2 to 6 carbon atoms, preferably 2 to 4, more preferably 2 to 3, and where appropriate, may be in either the (E)- or (Z)-configuration. Examples include vinyl, allyl and propargyl.

Halogen means fluorine, chlorine, bromine or iodine.

Haloalkyl groups may contain one or more identical or different halogen atoms, and includes, for example, trifluoromethyl, chlorodifluoromethyl, 2,2,2-trifluoroethyl or 2,2-difluoroethyl. Perfluoroalkyl groups are alkyl groups which are completely substituted with fluorine atoms and include, for example, trifluoromethyl, pentafluoroethyl and heptafluoroprop-2-yl.

Haloalkenyl and haloalkynyl groups may contain one or more identical or different halogen atoms, and include, for example, 2,2-difluorovinyl, 1,2-dichloro-2-fluorovinyl or 1-chloroprop-2-yn-1-yl.

Unless otherwise indicated, cycloalkyl may be mono- or bi-cyclic, may be optionally substituted by one or more $C_1$-$C_6$alkyl groups, and preferably contain 3 to 8 carbon atoms, more preferably 3 to 6 carbon atoms. Examples of cycloalkyl include cyclopropyl, 1-methylcyclopropyl, 2-methylcyclopropyl, cyclobutyl, cyclopentyl and cyclohexyl.

Halocycloalkyl groups may contain one or more identical or different halogen atoms, and includes, for example, 2,2-dichlorocyclopropyl, 2,2-dichloro-1-methylcyclopropyl and 2-chloro-4-fluorocyclohexyl.

Aryl includes phenyl, naphthyl, anthracenyl, indenyl, phenanthrenyl and biphenyl, with phenyl being preferred.

Heteroaryl means a mono-, bi- or tricyclic, aromatic hydrocarbon, containing 3 to 14, preferably 5 to 10, more preferably 6 to 8, ring-atoms, including 1 to 6, preferably 1 to 4, heteroatoms independently selected from nitrogen, oxygen and sulfur. Examples include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, benzothiadiazolyl, indazolyl, benzotriazolyl, benzothiazolyl, benzoxazolyl, quinolyl, isoquinolyl, phthalazinyl, quinoxalinyl, quinazolinyl, cinnolinyl and naphthyridinyl.

Heterocyclyl, as used herein, includes heteroaryl, and in addition may be a saturated or partially unsaturated cyclic hydrocarbon containing from 3 to 10 ring-atoms up to 4 of which are heteroatoms selected from nitrogen, oxygen and sulfur, and may be optionally substituted by one or more groups independently selected from halogen, nitro, cyano, alkyl, alkoxy. Examples of non-aromatic heterocyclyl groups are oxiranyl, azetidinyl, tetrahydrofuranyl, thiolanyl, pyrrolidinyl, pyrrolinyl, imidazolidinyl, imidazolinyl, sulfolanyl, dioxolanyl, dihydropyranyl, tetrahydropyranyl, piperidinyl, pyrazolinyl, pyrazolidinyl, dioxanyl, morpholinyl, dithianyl, thiomorpholinyl, piperazinyl, azepinyl, oxazepinyl, thiazepinyl, thiazolinyl and diazapanyl.

Preferred values of $A^1$ $A^2$, $A^3$, $A^4$, X, $R^1$, $R^2$, $R^5$, $G^1$, $G^2$, $Q^1$, $Q^2$, $Y^1$, $Y^2$, $Y^3$, and $Y^4$ are, in any combination, as set out below.

Preferably, $A^1$ is C-X.

Preferably, $A^2$ is C-X.

Preferably, $A^3$ is C-X.

Preferably, $A^4$ is C-X.

Preferably, X is hydrogen, halogen, cyano, methyl, trifluoromethyl or methoxy. More preferably, X is hydrogen, fluoro, chloro, cyano, trifluoromethyl or methoxy. Most preferably, X is hydrogen, fluoro, cyano or methoxy.

More preferably, $A^1$, $A^2$, $A^3$ and $A^4$ are C-X and each X is independently selected from hydrogen, halogen, cyano, methyl, trifluoromethyl and methoxy. Most preferably, $A^1$, $A^2$, $A^3$ and $A^4$ are C-X and each X is independently selected from hydrogen, fluoro, cyano and methoxy.

More preferably, $A^1$ is C-$X^1$ and $A^2$ is C-$X^2$ and $A^3$ is C-$X^3$ and $A^4$ is C-$X^4$ and $X^1$ and $X^4$ are independently selected from hydrogen, halogen, cyano, methyl, trifluoromethyl and methoxy and and $X^2$ and $X^3$ are both H. Most preferably, $A^1$ is C-$X^1$ and $A^2$ is C-$X^2$ and $A^3$ is C-$X^3$ and $A^4$ is C-$X^4$ and $X^1$ and $X^4$ are independently selected from from hydrogen, fluoro, cyano and methox. and and $X^2$ and $X^3$ are both H even more preferably $X^1$ and $X^4$ are independently selected from from fluoro, cyano and methoxy. and and $X^2$ and $X^3$ are both H

Preferably, $G^1$ is oxygen.

Preferably, $G^2$ is oxygen.

More preferably, $G^1$ is oxygen and $G^2$ is oxygen.

Preferably, $Y^1$ is bromo, chloro, cyano, methyl, ethyl, methoxymethyl, trifluoromethyl, trifluoromethylthio, trifluoromethylsulfinyl or trifluoromethylsulfonyl. More preferably, $Y^1$ is halogen, cyano, methyl, ethyl, methoxymethyl or trifluoromethyl. Even more preferably, $Y^1$ is bromo, chloro or methyl. Most preferably, $Y^1$ is chloro or methyl.

Preferably, $Y^2$ is hydrogen, fluoro, chloro or methyl. Most preferably, $Y^2$ is hydrogen.

Preferably, $Y^3$ is hydrogen, fluoro, chloro or methyl. Most preferably, $Y^3$ is hydrogen.

Preferably, $Y^4$ is bromo, chloro, cyano, methyl, ethyl, methoxymethyl, trifluoromethyl, trifluoromethylthio, trifluoromethylsulfinyl or trifluoromethylsulfonyl. More preferably, $Y^4$ is halogen, cyano, methyl, ethyl, methoxymethyl or trifluoromethyl. Even more preferably, $Y^4$ is bromo, chloro or methyl. Most preferably, $Y^4$ is chloro or methyl.

Preferably, $R^1$ is hydrogen, methyl, ethyl or acetyl. More preferably, $R^1$ is hydrogen, methyl or ethyl. Most preferably, $R^1$ is hydrogen.

Preferably, $R^2$ is hydrogen, methyl, ethyl or acetyl. More preferably, $R^2$ is hydrogen, methyl or ethyl. Most preferably, $R^2$ is hydrogen.

Preferably, $R^5$ is trifluoromethyl, pentafluoroethyl or heptafluoroprop-2-yl. Most preferably, $R^5$ is trifluoromethyl.

Preferably, $Q^1$ is aryl or heteroaryl; each optionally substituted by one to five substituents independently selected from cyano, nitro, hydroxy, bromo, chloro, fluoro, methyl, trifluoromethyl, methoxy, trifluoromethoxy, methylthio, methylsulfinyl, methylsulfonyl and phenyl.

More preferably, $Q^1$ is phenyl, pyridyl, furanyl, thiophenyl, pyrazolyl or 1,2,3-thiadiazolyl; each optionally substituted by one to four substituents independently selected from cyano, nitro, hydroxy, bromo, chloro, fluoro, methyl, trifluoromethyl, methoxy, trifluoromethoxy, methylthio, methylsulfinyl, methylsulfonyl and phenyl.

Even more preferably, $Q^1$ is phenyl, pyridyl or furanyl; each optionally substituted by one to four substituents independently selected from cyano, nitro, hydroxy, bromo, chloro, fluoro, methyl, trifluoromethyl, methoxy, trifluoromethoxy, methylthio, methylsulfinyl, methylsulfonyl and phenyl.

Most preferably, $Q^1$ is phenyl or furan-2-yl; each optionally substituted by one, two or three substituents independently selected from cyano, nitro, chloro, fluoro, methyl, trifluoromethyl, methoxy and trifluoromethoxy.

Representative $Q^1$ substituents are selected from the substituents of Table 1.

**Table 1**

| $Q^1$ | |
|---|---|
| 5-bromofuran-2-yl | 2-methoxyphenyl |
| 2-bromophenyl | 2-methylphenyl |
| 5-bromopyrid-3-yl | 3-methylpyrid-2-yl |
| 2-chloro-4-fluorophenyl | 4-methyl-1,2,3-thiadiazol-5-yl |
| 3-chloro-2-fluorophenyl | 4-nitrophenyl |
| 5-chloro-2-fluorophenyl | phenyl |
| 3-chloro-2-methylphenyl | 1,2,3-thiadiazol-4-yl |
| 2-chloro-4-nitrophenyl | thiophen-2-yl |
| 2-chloro-5-nitrophenyl | 2-trifluoromethoxyphenyl |
| 2-chlorophenyl | 4-trifluoromethoxyphenyl |

(continued)

| $Q^1$ | |
|---|---|
| 3-chlorophenyl | 2-trifluoromethylphenyl |
| 2-chloropyrid-3-yl | 4-trifluoromethylphenyl |
| 2-chloropyrid-4-yl | 2-methyl-4-cyanophenyl |
| 6-chloropyrid-3-yl | 2,4,6-trifluorophenyl |
| 5-chlorothiophen-2-yl | 2,6-difluoro-phenyl |
| 3-chloro-5-trifluoromethylpyrid-2-yl | 2,6-difluoro-4-cyanophenyl |
| 4-cyano-2-fluorophenyl | 2,chloro-6-fluorophenyl |
| 4-cyanophenyl | 2-methyl-4-nitrophenyl |
| 2,5-dichlorophenyl | 3-methyl-4-nitrophenyl |
| 2,3-difluorophenyl | 2-chloro-4-cyanophenyl |
| 1,3-dimethyl-1*H*-pyrazol-5-yl | 2-fluoro-4-cyanophenyl |
| 2-fluorophenyl | 4-methylthiophenyl |
| 4-fluorophenyl | 1 -methyl-3-trifluoromethylpyrazol-4-yl |
| 2-fluoropyrid-3-yl | 4-pyridyl |
| 2-fluoro-3-trifluoromethylphenyl | 1,3-dimethylpyrazol-4-yl |
| 2-fluoro-5-trifluoromethylphenyl | 4-methylphenyl |
| 4-fluoro-3-trifluoromethylphenyl | 4-fluoro-2-methylphenyl |
| furan-2-yl | 2,4-difluorophenyl |

Preferably, $Q^2$ is aryl or heteroaryl; each optionally substituted by one to five substituents independently selected from cyano, nitro, hydroxy, bromo, chloro, fluoro, methyl, trifluoromethyl, methoxy and trifluoromethoxy.
More preferably, $Q^2$ is phenyl, pyridyl, furanyl, thiophenyl or thiazolyl; each optionally substituted by one to three substituents independently selected from chloro, fluoro, methyl, trifluoromethyl, methoxy and trifluoromethoxy.
Most preferably, $Q^2$ is phenyl or thiophenyl; each optionally substituted by one, two or three substituents independently selected from chloro, fluoro, methyl, trifluoromethyl, methoxy and trifluoromethoxy.
Representative $Q^2$ substituents are selected from the substituents of Table 2.

**Table 2**

| $Q^2$ | |
|---|---|
| 4-trifluoromethylphenyl | 3,5-dichlorophenyl |
| 4-chlorophenyl | 3,5-di-(trifluoromethyl)phenyl |
| phenyl | 4-fluorophenyl |
| thiazol-1-yl | 4-methoxyphenyl |
| thiophen-2-yl | 4-trifluoromethoxyphenyl |
| thiophen-3-yl | 3-fluorophenyl |
| pyrid-2-yl | 3-methylthiophen-2-yl |
| pyrid-3-yl | 4-methylthiophen-2-yl |
| pyrid-4-yl | |

In a preferred aspect of the invention, $A^1$, $A^2$, $A^3$ and $A^4$ are CH.
In another preferred aspect of the invention, $A^1$ is C-F and $A^2$, $A^3$ and $A^4$ are CH.
In a further preferred aspect of the invention, $A^2$ is C-F and $A^1$, $A^3$, and $A^4$ are CH.

In a further preferred aspect of the invention, $A^3$ is C-F and $A^1$, $A^2$, and $A^4$ are CH.
In a further preferred aspect of the invention, $A^4$ is C-F and $A^1$, $A^2$, and $A^3$ are CH.
In a further preferred aspect of the invention, $A^1$ is C-CN and $A^2$, $A^3$, and $A^4$ are CH.
In a further preferred aspect of the invention, $A^4$ is C-$OCH_3$ and $A^1$, $A^2$, and $A^3$ are CH.

In a first preferred embodiment of the invention $A^1$, $A^2$, $A^3$ and $A^4$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are methyl; $Q^1$ is selected from the substituents of Table 1; and $Q^2$ is 4-chlorophenyl.

In a second preferred embodiment of the invention $A^1$ is C-CN and $A^2$, $A^3$, and $A^4$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are methyl; $Q^1$ is selected from the substituents of Table 1; and $Q^2$ is 4-chlorophenyl.

In a third preferred embodiment of the invention $A^4$ is C-$OCH_3$ and $A^1$, $A^2$, and $A^3$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are methyl; $Q^1$ is selected from the substituents of Table 1; and $Q^2$ is 4-chlorophenyl.

In a fourth preferred embodiment of the invention $A^4$ is C-F and $A^1$, $A^2$, and $A^3$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are methyl; $Q^1$ is selected from the substituents of Table 1; and $Q^2$ is 4-chlorophenyl.

In a fifth preferred embodiment of the invention $A^1$, $A^2$, $A^3$ and $A^4$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are chloro; $Q^1$ is selected from the substituents of Table 1; and $Q^2$ is 4-chlorophenyl.

In a sixth preferred embodiment of the invention $A^1$ is C-CN and $A^2$, $A^3$, and $A^4$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are chloro; $Q^1$ is selected from the substituents of Table 1; and $Q^2$ is 4-chlorophenyl.

In a seventh preferred embodiment of the invention $A^4$ is C-$OCH_3$ and $A^1$, $A^2$, and $A^3$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are chloro; $Q^1$ is selected from the substituents of Table 1; and $Q^2$ is 4-chlorophenyl.

In an eighth preferred embodiment of the invention $A^4$ is C-F and $A^1$, $A^2$, and $A^3$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are chloro; $Q^1$ is selected from the substituents of Table 1; and $Q^2$ is 4-chlorophenyl.

In a ninth preferred embodiment of the invention $A^1$, $A^2$, $A^3$ and $A^4$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are methyl; $Q^1$ is 2,4,6-trifluorophenyl; and $Q^2$ is selected from the substituents of Table 2.

In a tenth preferred embodiment of the invention $A^1$ is C-CN and $A^2$, $A^3$, and $A^4$ are CH; Tri and $R^2$ are hydrogen; $R^5$ is trifluoromethyl; $G^1$ and $G^2$ are oxygen; Y and $Y^3$ are hydrogen; $Y^1$ and $Y^4$ are methyl; $Q^1$ is 2,4,6-trifluorophenyl; and $Q^2$ is selected from the substituents of Table 2.

In a eleventh preferred embodiment of the invention $A^4$ is C-$OCH_3$ and $A^1$, $A^2$, and $A^3$ are CH; $R^1$ and $R^2$ are hydrogen; R is trifluoromethyl; $G^1$ and G are oxygen; Y and $Y^3$ are hydrogen; $Y^1$ and $Y^4$ are methyl; $Q^1$ is 2,4,6-trifluorophenyl; and $Q^2$ is selected from the substituents of Table 2.

In a twelth preferred embodiment of the invention $A^4$ is C-F and $A^1$, $A^2$, and $A^3$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are methyl; $Q^1$ is 2,4,6-trifluorophenyl; and $Q^2$ is selected from the substituents of Table 2.

In a thirteenth preferred embodiment of the invention $A^1$, $A^2$, $A^3$ and $A^4$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are chloro; $Q^1$ is 2,4,6-trifluorophenyl; and $Q^2$ is selected from the substituents of Table 2.

In a fourteenth preferred embodiment of the invention $A^1$ is C-CN and $A^2$, $A^3$, and $A^4$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are chloro; $Q^1$ is 2,4,6-trifluorophenyl; and $Q^2$ is selected from the substituents of Table 2.

In a fifteenth preferred embodiment of the invention $A^4$ is C-$OCH_3$ and $A^1$, $A^2$, and $A^3$ are CH; $R^1$ and $R^2$ are hydrogen; R is trifluoromethyl; $G^1$ and G are oxygen; Y and $Y^3$ are hydrogen; $Y^1$ and $Y^4$ are chloro; $Q^1$ is 2,4,6-trifluorophenyl; and $Q^2$ is selected from the substituents of Table 2.

In an sixteenth preferred embodiment of the invention $A^4$ is C-F and $A^1$, $A^2$, and $A^3$ are CH; $R^1$ and $R^2$ are hydrogen; $R^5$ is trifluoromethyl; $G^1$ and $G^2$ are oxygen; $Y^2$ and $Y^3$ are hydrogen; $Y^1$ and $Y^4$ are chloro; $Q^1$ is 2,4,6-trifluorophenyl; and $Q^2$ is selected from the substituents of Table 2.

Most preferred compounds of formula (I) are those of the Examples.

In a further aspect of the invention are intermediates, which are compounds of formula (II')

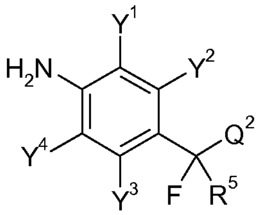

(II')

wherein $R^5$, $Q^2$, $Y^1$, $Y^2$, $Y^3$ and $Y^4$ are as defined in relation to formula (I); or a salt or *N*-oxide thereof. The preferences for $R^5$, $Q^2$, $Y^1$, $Y^2$, $Y^3$ and $Y^4$ are the same as the preferences set out for the corresponding substituents of the compounds of the formula (I).

In a further aspect of the invention are intermediates, which are compounds of formula (III)

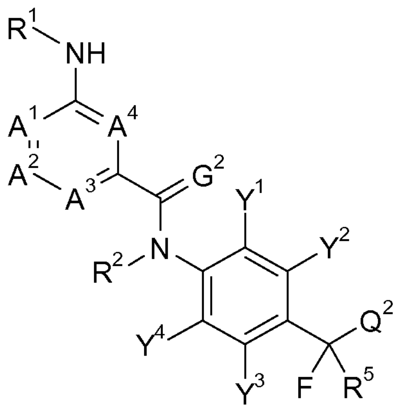

(III)

wherein $A^1$ $A^2$, $A^3$, $A^4$, $G^2$, $Y^1$, $Y^2$, $Y^3$, $Y^4$, $Q^2$, $R^1$, $R^2$ and $R^5$ are as defined in relation to formula (I); or a salt or *N*-oxide thereof. The preferences for $A^1$, $A^2$, $A^3$, $A^4$, $G^2$, $Y^1$, $Y^2$, $Y^3$, $Y^4$, $Q^2$, $R^1$, $R^2$ and $R^5$ are the same as the preferences set out for the corresponding substituents of the compounds of the formula (I).

In a further aspect of the invention are intermediates, which are compounds of formula (IV)

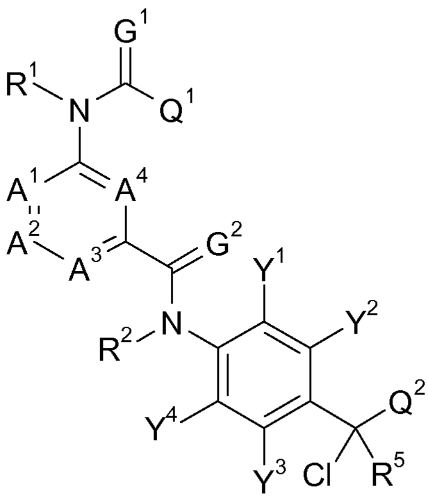

(IV)

wherein $A^1$ $A^2$, $A^3$, $A^4$, $G^1$, $G^2$, $Y^1$, $Y^2$, $Y^3$, $Y^4$, $Q^1$, $Q^2$, $R^1$, $R^2$ and $R^5$ are, are as defined in relation to formula (I); or a salt or *N*-oxide thereof provided that the compound or formula (IV) is not

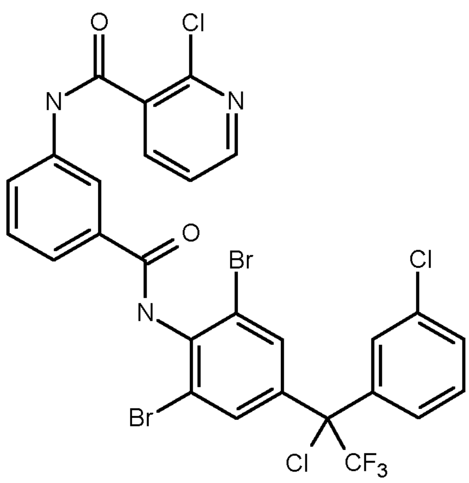

or

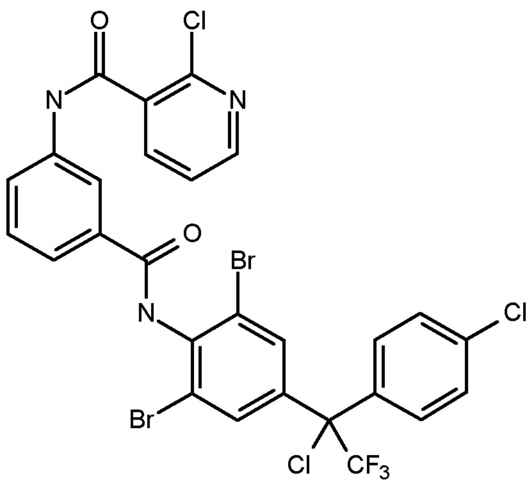

The preferences for $A^1$ $A^2$, $A^3$, $A^4$, $G^1$, $G^2$, $Y^1$, $Y^2$, $Y^3$, $Y^4$, $Q^1$, $Q^2$, $R^1$, $R^2$ and $R^5$ are the same as the preferences set out for the corresponding substituents of the compounds of the formula (I).

The compounds of the invention may be made by a variety of methods.

1) Compounds of formula (I), wherein $G^1$ and G are oxygen and $R^5$, $Q^2$, $Y^1$, $Y^2$, $Y^3$, $Y^4$, $R^2$, $A^1$, $A^2$, $A^3$, $A^4$, $R^1$ and $Q^1$ are as defined herein for compound of formula (I), may be made by the treatment of a compound of formula (III), wherein $G^2$ is oxygen and $R^5$, $Q^2$, $Y^1$, $Y^2$, $Y^3$, $Y^4$, $R^2$, $A^1$, $A^2$, $A^3$, $A^4$, $R^1$, $G^1$ and $Q^1$ are as defined herein for compound of formula (I), with a carboxylic acid of formula $Q^1$-COOH, an acid halide of formula $Q^1$-COHal, wherein Hal is Cl, F or Br or an ester of formula $Q^1$-COR, wherein R is $C_1$-$C_6$alkoxy and $Q^1$ are as defined herein for compound of formula (I).

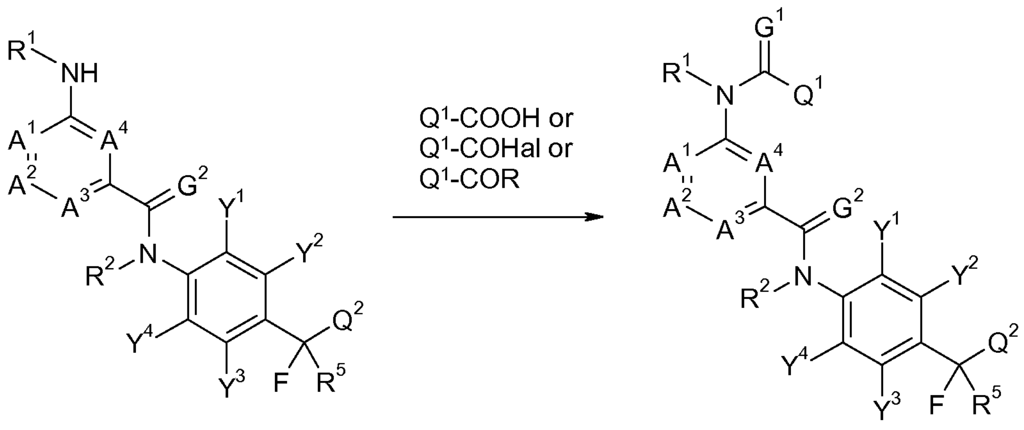


(III) (I)

provided that the compound of formula (I) is not

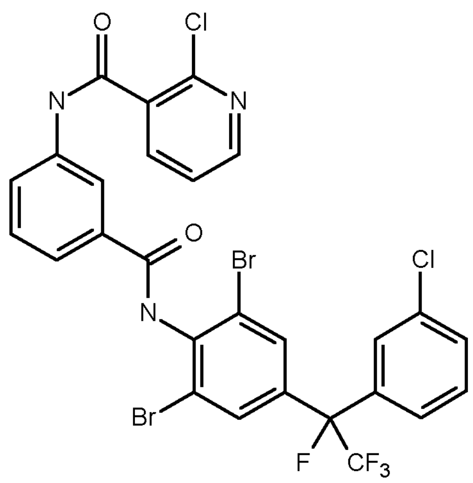

or

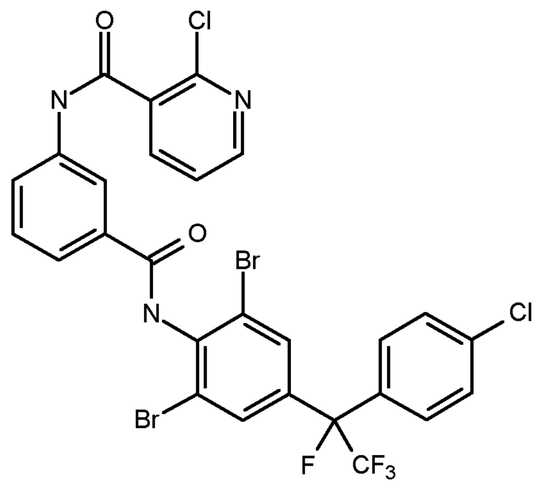

When a carboxylic acid is used such reactions are usually carried out in the presence of a coupling reagent, such as *N*,*N*-dicyclohexylcarbodiimide ("DCC"), 1-ethyl-3-[3-dimethylamino-propyl]carbodiimide hydrochloride ("EDC") or bis(2-oxo-3-oxazolidinyl)-phosphonic chloride ("BOP-Cl"), in the presence of a base, such as pyridine, triethylamine, 4-(dimethylamino)-pyridine or diisopropylethylamine, and optionally in the presence of a nucleophilic catalyst, such as hydroxybenzotriazole. When an acid halide is used, such reactions are usually carried out under basic conditions (for example in the presence of pyridine, triethylamine, 4-(dimethylamino)-pyridine or diisopropylethylamine), again optionally in the presence of a nucleophilic catalyst. When an ester is used it is sometimes possible to convert the ester directly to the amide by heating the ester and amine together in a thermal process.

2a) Compounds of formula (I), wherein $G^2$ is oxygen, may be made by treatment of an hydroxyl derivative of formula (V) as defined herein via an chlorine derivative of formula (IV) wherein $G^2$ is oxygen and $R^5$, $Q^2$, $Y^1$, $Y^2$, $Y^3$, $Y^4$, $R^2$, $A^1$, $A^2$, $A^3$, $A^4$, $R^1$, $G^1$ and $Q^1$ are as defined herein for compound of formula (I).

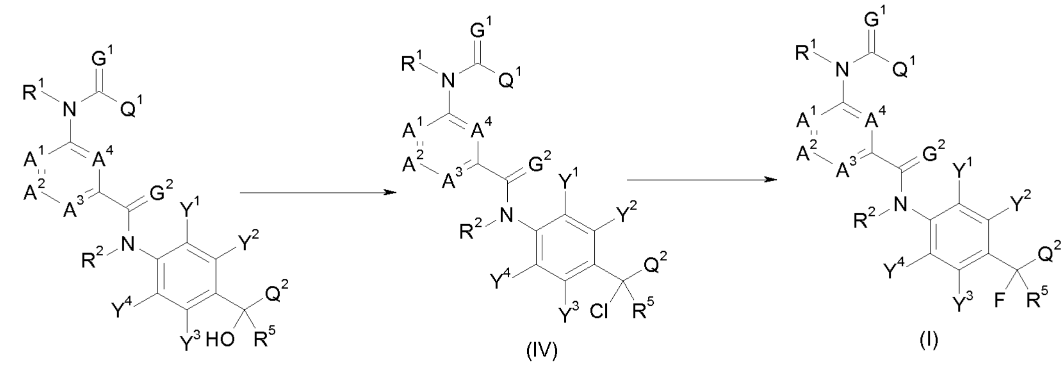

(V) (IV) (I)

provided that the compound of formula (I) is not

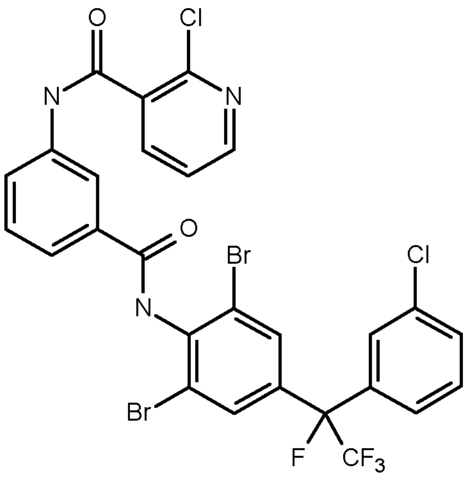

or

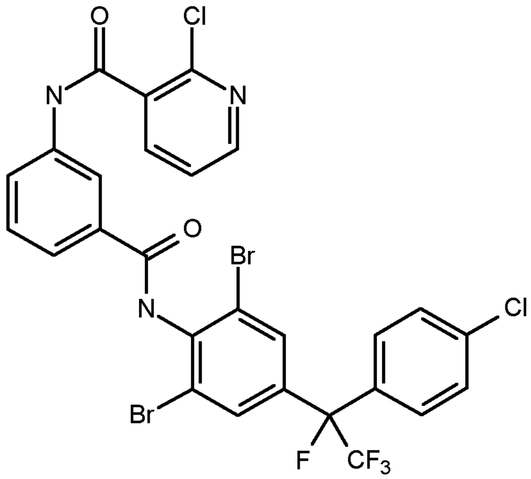

and provided that the compound or formula (IV) is not

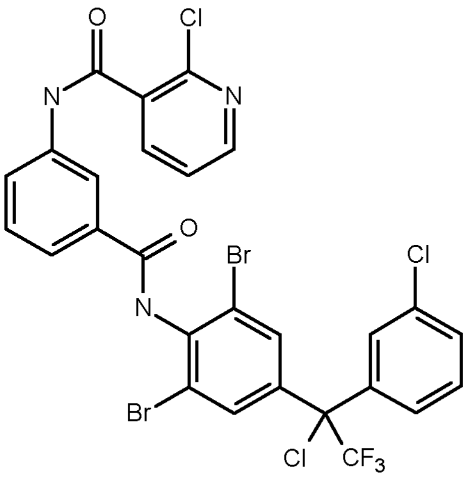

or

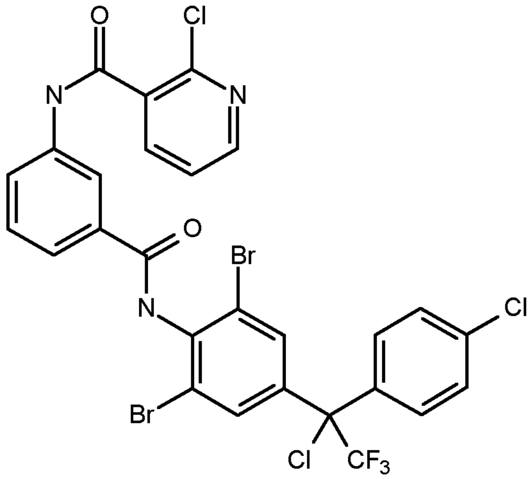

Compounds of formula (IV), wherein $G^2$ is oxygen, $R^1$ is hydrogen and $R^5$, $Q^2$, $Y^1$, $Y^2$, $Y^3$, $Y^4$, $R^2$, $A^1$, $A^2$, $A^3$, $A^4$, $G^1$ and $Q^1$ are as defined herein for compound of formula (I), may be made by treatment of a compound of formula (V) with phosphorous derivatives, for example in the presence of phosphorous trichloride. The reaction can preferably be carried out in a suitable solvent, preferably an aprotic solvent, for example a halogenated or non-halogenated aromatic (such as toluene or chlorobenzene).

Compounds of formula (I), wherein $G^1$ and $G^2$ are oxygen and $R^5$, $Q^2$, $Y^1$, $Y^2$, $Y^3$, $Y^4$, $R^2$, $A^1$, $A^2$, $A^3$, $A^4$, $R^1$ and $Q^1$ are as defined herein for compound of formula (I), may be made by the treatment of a compound of formula (IV), wherein $G^2$ is oxygen and $R^5$, $Q^2$, $Y^1$, $Y^2$, $Y^3$, $Y^4$, $R^2$, $A^1$, $A^2$, $A^3$, $A^4$, $R^1$, $G^1$ and $Q^1$ are as defined herein for compound of formula (I), with a source of fluorine, for example in the presence of cesium fluoride or potassium fluoride. The reaction can preferably be carried out in a suitable solvent, preferably an aprotic solvent, for example acetonitrile.

2b) Compounds of formula (I), wherein $G^2$ is oxygen and $R^5$, $Q^2$, $Y^1$, $Y^2$, $Y^3$, $Y^4$, $R^2$, $A^1$, $A^2$, $A^3$, $A^4$, $R^1$, $G^1$ and $Q^1$ are as defined herein for compound of formula (I), may be made by treatment of an hydroxyl derivative of formula (V) wherein wherein $G^2$ is oxygenand $R^5$, $Q^2$, $Y^1$, $Y^2$, $Y^3$, $Y^4$, $R^2$, $A^1$, $A^2$, $A^3$, $A^4$, $R^1$, $G^1$ and $Q^1$ are as defined herein for compound of formula (I) directely, with a source of fluorine for example in the presence of Perfluoromethanesulfonyl fluoride (see for example : JP2008174552) or in presence of Deoxy-Fluor in dichloromethane (see experimental section).

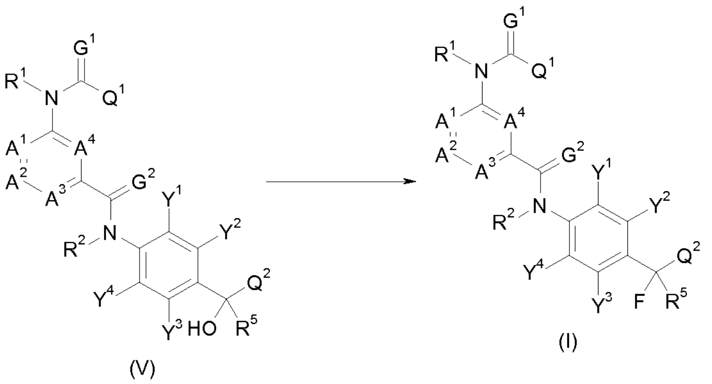

(V) (I)

provided that the compound of formula (I) is not

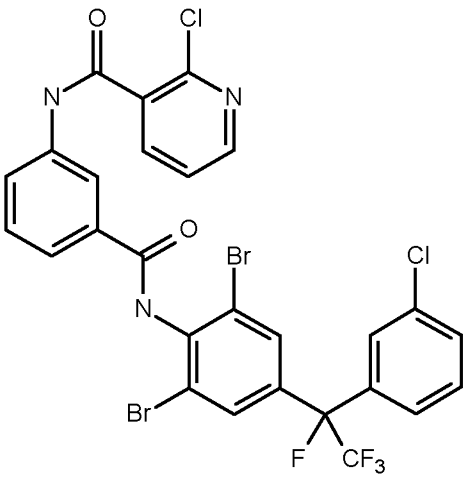

or

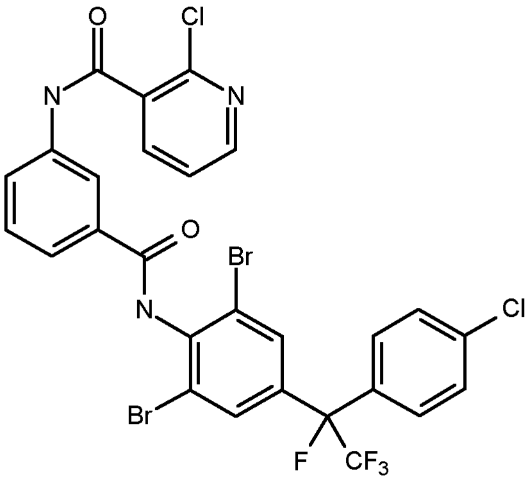

and provided that the compound of formula (V) is not

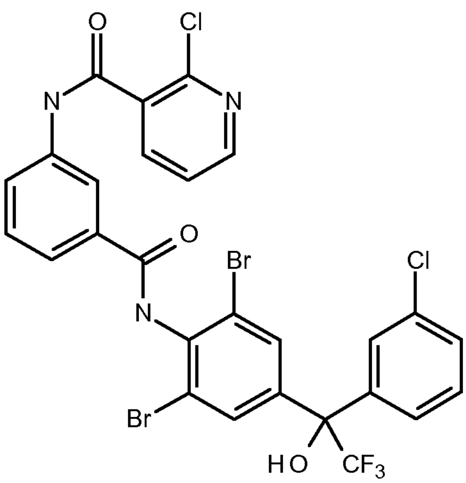

or

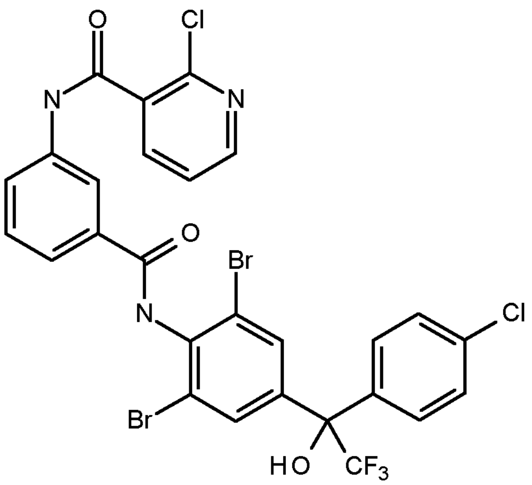

3a) Compounds of formula (V), wherein $G^2$ is oxygen and $R^5$, $Q^2$, $Y^1$, $Y^2$, $Y^3$, $Y^4$, $R^2$, $A^1$, $A^2$, $A^3$, $A^4$, $R^1$, $G^1$ and $Q^1$ are as defined herein for compound of formula (I), may be made by methods known to a person skilled in the art, as described, for example in WO2009049845. 3b) Compounds of formula (V), wherein $G^2$ is oxygen and $R^5$, $Q^2$, $Y^1$, $Y^2$, $Y^3$, $Y^4$, $R^2$, $A^1$, $A^2$, $A^3$, $A^4$, $R^1$, $G^1$ and $Q^1$ are as defined herein for compound of formula (I), may be made by treatment of a derivative of formula (VI) with a organometallic derived from Q2, such as organolithium or organomagnesium, in the presence of a diluent, such as tetrahydrofuran.

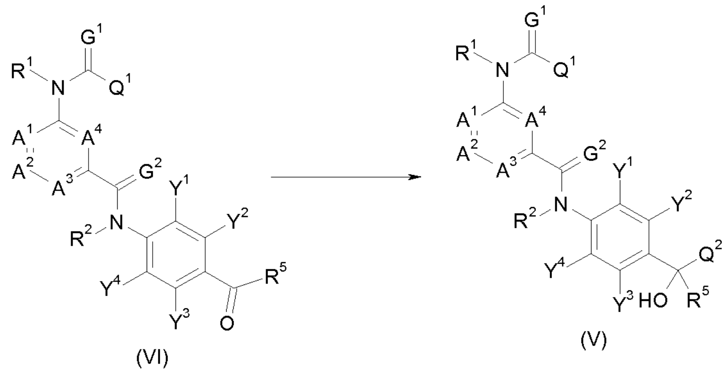

(VI) (V)

provided that the compound of formula (V) is not

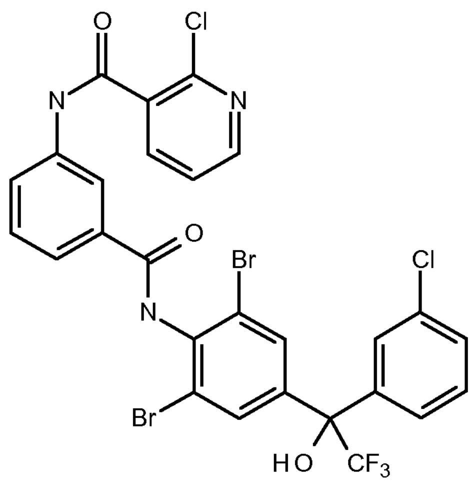

or

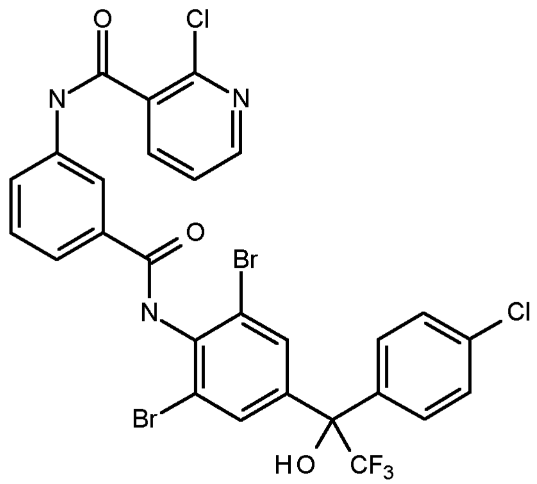

4) Compounds of formula (III), wherein $G^2$ is oxygen and $R^1$ is H and $R^5$, $Q^2$, $Y^1$, $Y^2$, $Y^3$, $Y^4$, $R^2$, $A^1$, $A^2$, $A^3$, $A^4$ are as defined herein for compound of formula (I), may be made by treatment of a derivative of formula (II) wherein $R^5$, $Q^2$, $Y^1$, $Y^2$, $Y^3$, $Y^4$, $R^2$ are as defined herein for compound of formula (I) with an acid chloride of formula (VII) wherein $G^2$, $A^1$, $A^2$, $A^3$ and $A^4$ are as defined in relation to formula (I).

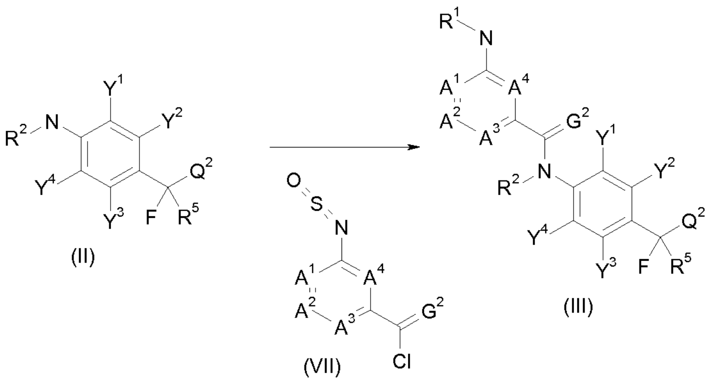

(II) (VII) (III)

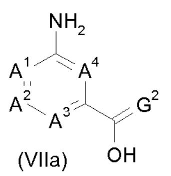

(VIIa)

Acid chloride of formula (VII), wherein $G^2$, $A^1$, $A^2$, $A^3$ and $A^4$ are as defined in relation to formula (I) may be made from an amino-carboxylic acid of formula (VIIa) by methods known to a person skilled in the art, such as treatment with thionyl chloride. This reaction is described, for example, in Journal fuer Praktische Chemie (Leipzig) (1937), 148, 161-9. The reaction can preferably be carried on in a suitable solvent, preferably an aprotic solvent, for example an ether (such as tetrahydrofuran or diethyl ether), a halogenated hydrocarbon (such as dichloromethane, chloroform, carbon tetrachloride, or 1,1,1-trichloroethane), a halogenated or non-halogenated aromatic (such as toluene or chlorobenzene), or a mixture thereof.

4a) Alternatively, Compounds of formula (III), wherein $G^2$ is oxygen and $R^1$ is H and $R^5$, $Q^2$, $Y^1$, $Y^2$, $Y^3$, $Y^4$, $R^2$, $A^1$, $A^2$, $A^3$, $A^4$ are as defined herein for compound of formula (I), may be made by treatment of an derivative of formula (II) wherein $R^5$, $Q^2$, $Y^1$, $Y^2$, $Y^3$, $Y^4$, $R^2$ are as defined herein for compound of formula (I) with a acid chloride (R = Cl) of formula (VII) wherein $G^2$, $A^1$, $A^2$, $A^3$ and $A^4$ are as defined in relation to formula (I) or carboxylic acid (R = OH) of formula (VIII) wherein $G^2$, $A^1$, $A^2$, $A^3$ and $A^4$ are as defined in relation to formula (I) to form an derivative of formula (IX) wherein $G^2$ is oxygen and $R^5$, $Q^2$, $Y^1$, $Y^2$, $Y^3$, $Y^4$, $R^2$, $A^1$, $A^2$, $A^3$, $A^4$ are as defined herein for compound of formula (I) . Compounds of formula (III) may be made by reduction of a derivative of formula (IX).

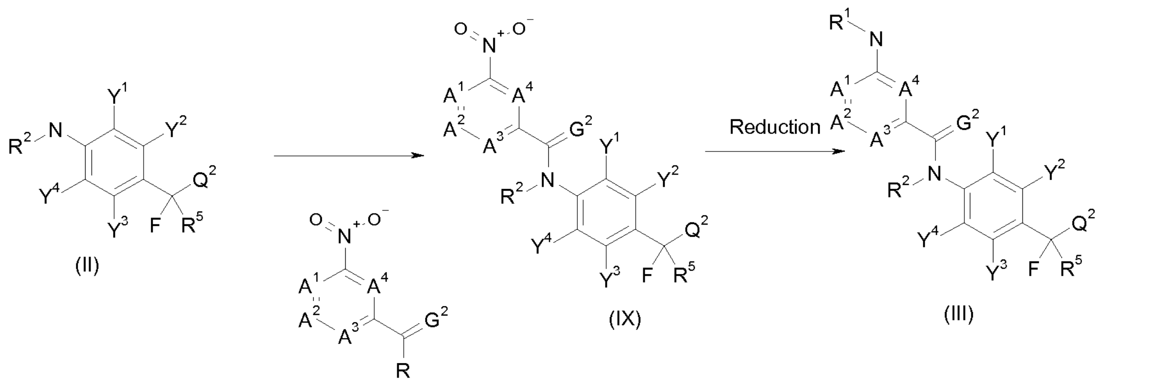

(II) (VIII or VII) (IX) (III)

Acid chlorides of formula (VII) wherein $G^2$, $A^1$, $A^2$, $A^3$ and $A^4$ are as defined in relation to formula (I) may be made from a carboxylic acid via by methods known to the person skilled in the art.

Compounds of formula (III), wherein $R^1$ is hydrogen, may be achieved by the reduction of nitro compounds of formula (IX). There are numerous methods for achieving such a transformation reported in the literature such as treatment with tin chloride under acidic conditions, or hydrogenation catalysed by a noble metal such as palladium on carbon.

5) Alternatively, compounds of formula (III), wherein $G^2$ is oxygen and $R^5$, $Q^2$, $Y^1$, $Y^2$, $Y^3$, $Y^4$, $R^2$, $A^1$, $A^2$, $A^3$, $A^4$ and $R^1$ are as defined herein for compound of formula (I), may be made from compounds of formula (X), wherein $G^2$ is oxygen, LG is a leaving group such as fluoro chloro or sulfonate and $R^5$, $Q^2$, $Y^1$, $Y^2$, $Y^3$, $Y^4$, $R^2$, $A^1$, $A^2$, $A^3$, $A^4$, by displacement of the leaving group with a compound of formula $R^1$-$NH_2$ wherein $R^1$ is as defined herein for compound of formula (I). Such reactions are usually performed under basic conditions or under metal catalysis like, such as palladium derivatives or copper salts via by methods known to the person skilled in the art.

6) Compounds of formula (X) wherein $G^2$ is oxygen, LG is a leaving group such as fluoro, chloro or sulfonate and $R^5$, $Q^2$, $Y^1$, $Y^2$, $Y^3$, $Y^4$, $R^2$, $A^1$, $A^2$, $A^3$, $A^4$ as defined herein for compound of formula (I) may be made from compounds of formula (XI), wherein R is Cl or OH, LG is a leaving group such as iodo, chloro, bromo, fluoro or sulfonate and $A^1$, $A^2$, $A^3$ and $A^4$ as defined herein for compound of formula (I), via amide bond formation under standard conditions as described in 1). Compounds of formula (XI) and formula (XII) are either known compounds or may be made by methods known to the person skilled in the art.

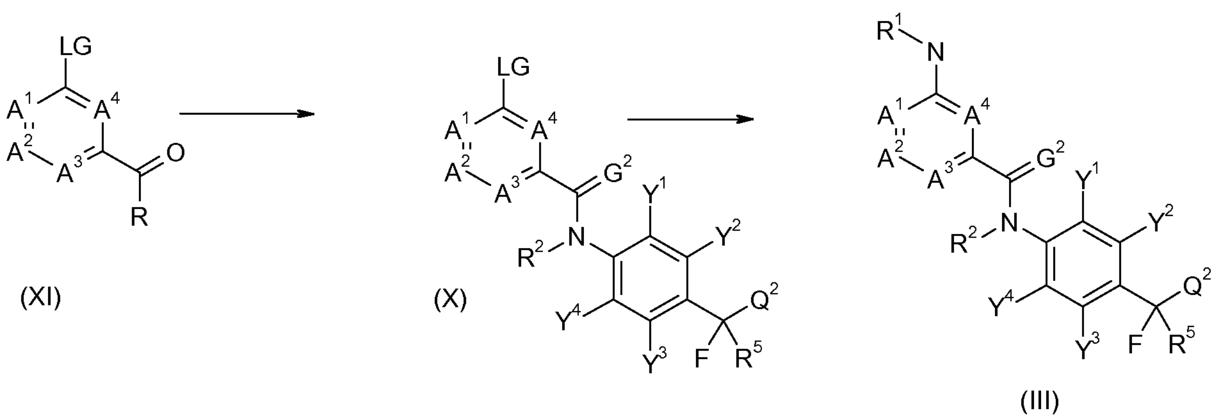
(XI) (X) (III)

7) Compounds of formula (I), wherein $G^1$ and $G^2$ are sulfur, may be made from a compound of formula (I), wherein

$G^1$ and $G^2$ are oxygen, by treatment with a thio-transfer reagent, such as Lawesson's reagent or phosphorus pentasulfide.

8) Compounds of formula (I), wherein $G^1$ is sulfur and $G^2$ is oxygen, may be made from a compound of formula (III), wherein $G^2$ is oxygen and $R^5$, $Q^2$, $Y^1$, $Y^2$, $Y^3$, $Y^4$, $R^2$, $A^1$, $A^2$, $A^3$, $A^4$ and $R^1$ are as defined herein for compound of formula (I), and coupling with a thio analogue such as a thiocarboxylic acid of formula $Q^1$-CSOH wherein $Q^1$ is as defined herein for compound of formula (I) or a thioacid halide of formula $Q^1$-CSHal, wherein Hal is Cl, F or Br and $Q^1$ is as defined herein for compound of formula (I).

9) Compounds of formula (I), wherein $G^1$ is oxygen and $G^2$ is sulfur, may be made from a compound of formula (III) wherein $R^5$, $Q^2$, $Y^1$, $Y^2$, $Y^3$, $Y^4$, $R^2$, $A^1$, $A^2$, $A^3$, $A^4$ and $R^1$ are as defined herein for compound of formula (I) which is treated with a thio-transfer reagent, such as Lawessen's reagent or phosphorus pentasulfide, prior to coupling with a carboxylic acid of formula $Q^1$-COOH wherein $Q^1$ is as defined herein for compound of formula (I), an acid halide of formula $Q^1$-COHal, wherein Hal is Cl, F or Br and $Q^1$ is as defined herein for compound of formula (I) or an ester of formula $Q^1$-COR, wherein R is $C_1$-$C_6$alkoxy and w$Q^1$ is as defined herein for compound of formula (I).

10) Compounds of formula (III) wherein X is cyano and $R^5$, $Q^2$, $Y^1$, $Y^2$, $Y^3$, $Y^4$, $R^2$, $G^2$, $A^1$, $A^2$, $A^3$, $A^4$ and $R^1$ are as defined herein for compound of formula (I), can be made from a compound of formula (III*) wherein LG is halogen, such as bromide or iodide and $R^5$, $Q^2$, $Y^1$, $Y^2$, $Y^3$, $Y^4$, $R^2$, $G^2$, $A^1$, $A^2$, $A^3$, $A^4$ and $R^1$ are as defined herein for compound of formula (I), by reaction with a cyanide salt, such as copper cyanide or zinc cyanide in presence of a palladium catalyst.

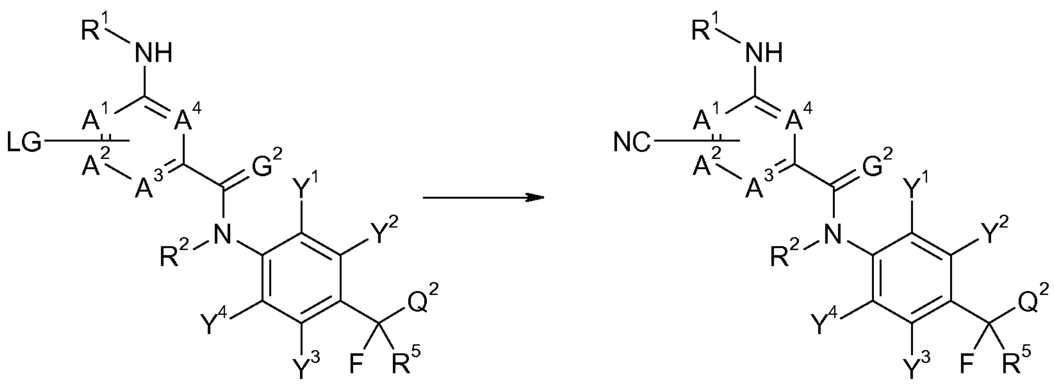

(III*) (III)

Similar reactions with copper cyanide have been described in, for example, J. Med. Chem. (2004), 47(8), 1969-1986, J. Med. Chem. (2002), 45(17), 3692-3702 and J. Med. Chem. (1989), 32(3), 575-83. Similar reactions with zinc cyanide in the presence of a palladium catalyst have been described in, for example, Bioorganic & Medicinal Chemistry Letters (2007), 17(7), 1908. The displacement of a halogen with cyanide can also be carried out on compounds of formula (I) and (IV).

10b) Alternatively, compounds of formula (III), wherein X is cyano or $C_1$-$C_4$alkoxy, $R^1$ hydrogen and $R^5$, $Q^2$, $Y^1$, $Y^2$, $Y^3$, $Y^4$, $R^2$, $G^2$, $A^1$, $A^2$, $A^3$ and $A^4$ are as defined herein for compound of formula (I), may be made by reaction of derivatives (IX*) wherein LG is a leaving group such as fluoro, chloro or sulfonate and $R^5$, $Q^2$, $Y^1$, $Y^2$, $Y^3$, $Y^4$, $R^2$, $G^2$, $A^1$, $A^2$, $A^3$ and $A^4$ are as defined herein for compound of formula (I), by displacement of the leaving group with a alcohol or a cyanide. The last step will be a reduction of the nitro group. There are numerous methods for achieving such a transformation reported in the literature such as treatment with tin chloride under acidic conditions, or hydrogenation catalysed by a noble metal such as palladium on carbon.

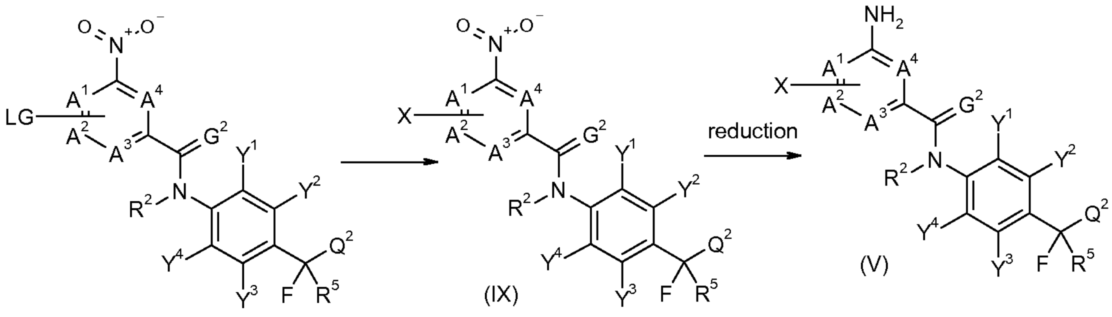


(IX*)

X = CN, OC1-C4 Alkyl

The compounds of formula (I) can be used to combat and control infestations of insect pests such as Lepidoptera, Diptera, Hemiptera, Thysanoptera, Orthoptera, Dictyoptera, Coleoptera, Siphonaptera, Hymenoptera and Isoptera and also other invertebrate pests, for example, acarine, nematode and mollusc pests. Insects, acarines, nematodes and molluscs are hereinafter collectively referred to as pests. The pests which may be combated and controlled by the use of the invention compounds include those pests associated with agriculture (which term includes the growing of crops for food and fiber products), horticulture and animal husbandry, companion animals, forestry and the storage of products of vegetable origin (such as fruit, grain and timber); those pests associated with the damage of man-made structures and the transmission of diseases of man and animals; and also nuisance pests (such as flies).

Examples of pest species which may be controlled by the compounds of formula (I) include: *Myzus persicae* (aphid), *Aphis gossypii* (aphid), *Aphis fabae* (aphid), *Lygus* spp. (capsids), *Dysdercus* spp. (capsids), *Nilaparvata lugens* (planthopper), *Nephotettixc incticeps* (leafhopper), *Nezara* spp. (stinkbugs), *Euschistus* spp. (stinkbugs), *Leptocorisa* spp. (stinkbugs), *Frankliniella occidentalis* (thrip), Thrips spp. (thrips), *Leptinotarsa decemlineata* (Colorado potato beetle), *Anthonomus grandis* (boll weevil), *Aonidiella* spp. (scale insects), *Trialeurodes* spp. (white flies), *Bemisia tabaci* (white fly), *Ostrinia nubilalis* (European corn borer), *Spodoptera littoralis* (cotton leafworm), *Heliothis virescens* (tobacco budworm), *Helicoverpa armigera* (cotton bollworm), *Helicoverpa zea* (cotton bollworm), *Sylepta derogata* (cotton leaf roller), *Pieris brassicae* (white butterfly), *Plutella xylostella* (diamond back moth), *Agrotis* spp. (cutworms), *Chilo suppressalis* (rice stem borer), *Locusta migratoria* (locust), *Chortiocetes terminifera* (locust), *Diabrotica* spp. (rootworms), *Panonychus ulmi* (European red mite), *Panonychus citri* (citrus red mite), *Tetranychus urticae* (two-spotted spider mite), *Tetranychus cinnabarinus* (carmine spider mite), *Phyllocoptruta oleivora* (citrus rust mite), *Polyphagotarsonemus latus* (broad mite), *Brevipalpus* spp. (flat mites), *Boophilus microplus* (cattle tick), *Dermacentor variabilis* (American dog tick), *Ctenocephalides felis* (cat flea), *Liriomyza* spp. (leafminer), *Musca domestica* (housefly), *Aedes aegypti* (mosquito), *Anopheles* spp. (mosquitoes), *Culex* spp. (mosquitoes), *Lucillia* spp. (blowflies), *Blattella germanica* (cockroach), *Periplaneta americana* (cockroach), *Blatta orientalis* (cockroach), termites of the Mastotermitidae (for example *Mastotermes* spp.), the Kalotermitidae (for example *Neotermes* spp.), the Rhinotermitidae (for example *Coptotermes formosanus*, *Reticulitermes flavipes, R. speratu, R. virginicus, R. hesperus*, and *R. santonensis*) and the Termitidae (for example *Globitermes sulfureus*), *Solenopsis geminata* (fire ant), *Monomorium pharaonis* (pharaoh's ant), *Damalinia* spp. and *Linognathus* spp. (biting and sucking lice), *Meloidogyne* spp. (root knot nematodes), *Globodera* spp. and *Heterodera* spp. (cyst nematodes), *Pratylenchus* spp. (lesion nematodes), *Rhodopholus* spp. (banana burrowing nematodes), *Tylenchulus* spp.(citrus nematodes), *Haemonchus contortus* (barber pole worm), *Caenorhabditis elegans* (vinegar eelworm), *Trichostrongylus* spp. (gastro intestinal nematodes) and *Deroceras reticulatum* (slug).

The invention therefore provides a method of combating and controlling insects, acarines, nematodes or molluscs which comprises applying an insecticidally, acaricidally, nematicidally or molluscicidally effective amount of a compound of formula (I), or a composition containing a compound of formula (I), to a pest, a locus of pest, preferably a plant, or to a plant susceptible to attack by a pest. The compounds of formula (I) are preferably used against insects, acarines or nematodes.

The term "plant" as used herein includes seedlings, bushes and trees.

Crops are to be understood as also including those crops which have been rendered tolerant to herbicides or classes of herbicides (e.g. ALS-, GS-, EPSPS-, PPO- and HPPD-inhibitors) by conventional methods of breeding or by genetic engineering. An example of a crop that has been rendered tolerant to imidazolinones, e.g. imazamox, by conventional methods of breeding is Clearfield® summer rape (canola). Examples of crops that have been rendered tolerant to herbicides by genetic engineering methods include e.g. glyphosate- and glufosinate-resistant maize varieties commercially available under the trade names RoundupReady® and LibertyLink®.

Crops are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK®

(Syngenta Seeds). Examples of transgenic plants comprising one or more genes that code for an insecticidal resistance and express one or more toxins are KnockOut® (maize), Yield Gard® (maize), NuCOTIN33B® (cotton), Bollgard® (cotton), NewLeaf® (potatoes), NatureGard® and Protexcta®.

Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood as being those which are obtained by conventional methods of breeding or genetic engineering and contain so-called output traits (e.g. improved storage stability, higher nutritional value and improved flavor).

In order to apply a compound of formula (I) as an insecticide, acaricide, nematicide or molluscicide to a pest, a locus of pest, or to a plant susceptible to attack by a pest, a compound of formula (I) is usually formulated into a composition which includes, in addition to the compound of formula (I), a suitable inert diluent or carrier and, optionally, a surface active agent (SFA). SFAs are chemicals which are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (I). The composition is generally used for the control of pests such that a compound of formula (I) is applied at a rate of from 0.1g to 10kg per hectare, preferably from 1g to 6kg per hectare, more preferably from 1g to 1kg per hectare.

When used in a seed dressing, a compound of formula (I) is used at a rate of 0.0001g to 10g (for example 0.001g or 0.05g), preferably 0.005g to 10g more preferably 0.005g to 4g, per kilogram of seed.

In another aspect the present invention provides an insecticidal, acaricidal, nematicidal or molluscicidal composition comprising an insecticidally, acaricidally, nematicidally or molluscicidally effective amount of a compound of formula (I) and a suitable carrier or diluent therefor. The composition is preferably an insecticidal, acaricidal, nematicidal or molluscicidal composition.

The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (I).

Dustable powders (DP) may be prepared by mixing a compound of formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulfur, lime, flours, talc and other organic and inorganic solid carriers) and mechanically grinding the mixture to a fine powder.

Soluble powders (SP) may be prepared by mixing a compound of formula (I) with one or more water-soluble inorganic salts (such as sodium bicarbonate, sodium carbonate or magnesium sulfate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (I) and one or more powdered solid diluents or carriers, or from pre-formed blank granules by absorbing a compound of formula (I) (or a solution thereof, in a suitable agent) in a porous granular material (such as pumice, attapulgite clays, fuller's earth, kieselguhr, diatomaceous earths or ground corn cobs) or by adsorbing a compound of formula (I) (or a solution thereof, in a suitable agent) on to a hard core material (such as sands, silicates, mineral carbonates, sulfates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (I) in water or an organic solvent, such as a ketone, alcohol or glycol ether. These solutions may contain a surface active agent (for example to improve water dilution or prevent crystallization in a spray tank).

Emulsifiable concentrates (EC) or oil-in-water emulsions (EW) may be prepared by dissolving a compound of formula (I) in an organic solvent (optionally containing one or more wetting agents, one or more emulsifying agents or

a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as $C_8$-$C_{10}$ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (I) either as a liquid (if it is not a liquid at room temperature, it may be melted at a reasonable temperature, typically below 70°C) or in solution (by dissolving it in an appropriate solvent) and then emulsifiying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of formula (I) is present initially in either the water or the solvent/SFA blend. Suitable solvents for use in MEs include those hereinbefore described for use in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula (I). SCs may be prepared by ball or bead milling the solid compound of formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (I) may be dry milled and added to water, containing agents hereinbefore described, to produce the desired end product.

Aerosol formulations comprise a compound of formula (I) and a suitable propellant (for example *n*-butane). A compound of formula (I) may also be dissolved or dispersed in a suitable medium (for example water or a water miscible liquid, such as *n*-propanol) to provide compositions for use in non-pressurized, hand-actuated spray pumps.

A compound of formula (I) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerization stage such that an aqueous dispersion of oil droplets is obtained, in which each oil droplet is encapsulated by a polymeric shell and contains a compound of formula (I) and, optionally, a carrier or diluent therefor. The polymeric shell may be produced by either an interfacial polycondensation reaction or by a coacervation procedure. The compositions may provide for controlled release of the compound of formula (I) and they may be used for seed treatment. A compound of formula (I) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (I)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (I)).

A compound of formula (I) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier).

Wetting agents, dispersing agents and emulsifying agents may be surface SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulfuric acid (for example sodium lauryl sulfate), salts of sulfonated aromatic compounds (for example sodium dodecylbenzenesulfonate, calcium dodecylbenzenesulfonate, butylnaphthalene sulfonate and mixtures of sodium di*iso*propyl- and tri-*iso*propylnaphthalene sulfonates), ether sulfates, alcohol ether sulfates (for example sodium laureth-3-sulfate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulfosuccinamates, paraffin or olefine sulfonates, taurates and lignosulfonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula (I) may be applied by any of the known means of applying pesticidal compounds. For example, it may be applied, formulated or unformulated, to the pests or to a locus of the pests (such as a habitat of the pests, or a growing plant liable to infestation by the pests) or to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapor or applied through distribution or incorporation of a composition (such as a granular composition or a composition packed in a water-soluble bag) in soil or an aqueous environment.

A compound of formula (I) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs, SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (I) (for example 0.0001 to 10%, by weight) depending upon the purpose for which they are to be used.

A compound of formula (I) may be used in mixtures with fertilizers (for example nitrogen-, potassium- or phosphorus-containing fertilizers). Suitable formulation types include granules of fertilizer. The mixtures preferably contain up to 25% by weight of the compound of formula (I).

The invention therefore also provides a fertilizer composition comprising a fertilizer and a compound of formula (I).

The compound of formula I (herein after abbreviated by the term "TX" thus means a compound encompassed by the compounds of formula I, or preferably the term "TX" refers to a compound selected from the Table A (compound A1 to compound A3 8)) may be the sole active ingredient of the composition or it may be admixed with one or more additional active ingredients such as a pesticide (insect, acarine, mollusc and nematode pesticide), fungicide, synergist, herbicide, safener or plant growth regulator where appropriate. The activity of the compositions according to the invention may thereby be broadened considerably and may have surprising advantages which can also be described, in a wider sense, as synergistic activity. An additional active ingredient may: provide a composition having a broader spectrum of activity or increased persistence at a locus; provide a composition demonstrating better plant/crop tolerance by reducing phytotoxicity; provide a composition controlling insects in their different development stages; synergise the activity or complement the activity (for example by increasing the speed of effect or overcoming repellency) of the TX; or help to overcome or prevent the development of resistance to individual components. The particular additional active ingredient will depend upon the intended utility of the composition. Examples of suitable pesticides include the following:

a) Pyrethroids, such as permethrin, cypermethrin, fenvalerate, esfenvalerate, deltamethrin, cyhalothrin (in particular lambda-cyhalothrin), bifenthrin, fenpropathrin, cyfluthrin, tefluthrin, fish safe pyrethroids (for example ethofenprox), natural pyrethrin, tetramethrin, s-bioallethrin, fenfluthrin, prallethrin or 5-benzyl-3-furylmethyl-(E)-(1R,3S)-2,2-dimethyl- 3-(2-oxothiolan-3-ylidenemethyl)cyclopr opane carboxylate;

b) Organophosphates, such as, profenofos, sulprofos, acephate, methyl parathion, azinphos-methyl, demeton-s-methyl, heptenophos, thiometon, fenamiphos, monocrotophos, profenofos, triazophos, methamidophos, dimethoate, phosphamidon, malathion, chlorpyrifos, phosalone, terbufos, fensulfothion, fonofos, phorate, phoxim, pirimiphos-methyl, pirimiphos-ethyl, fenitrothion, fosthiazate or diazinon;

c) Carbamates (including aryl carbamates), such as pirimicarb, triazamate, cloethocarb, carbofuran, furathiocarb, ethiofencarb, aldicarb, thiofurox, carbosulfan, bendiocarb, fenobucarb, propoxur, methomyl or oxamyl;

d) Benzoyl ureas, such as diflubenzuron, triflumuron, hexaflumuron, flufenoxuron or chlorfluazuron;

e)Organic tin compounds, such as cyhexatin, fenbutatin oxide or azocyclotin;

f) Pyrazoles, such as tebufenpyrad and fenpyroximate;
g) Macrolides, such as avermectins or milbemycins, for example abamectin, emamectin benzoate, ivermectin, milbemycin, or spinosad, spinetoram or azadirachtin;
h) Hormones or pheromones;
i) Organochlorine compounds such as endosulfan, benzene hexachloride, DDT, chlordane or dieldrin;
j) Amidines, such as chlordimeform or amitraz;
k) Fumigant agents, such as chloropicrin, dichloropropane, methyl bromide or metam;
l) Neonicotinoid compounds such as imidacloprid, thiacloprid, acetamiprid, clothianidin, nitenpyram, dinotefuran or thiamethoxam;
m) Diacylhydrazines, such as tebufenozide, chromafenozide or methoxyfenozide;
n) Diphenyl ethers, such as diofenolan or pyriproxifen;
o) Indoxacarb;
p) Chlorfenapyr;
q) Pymetrozine or pyrifluquinazon;
r) Spirotetramat, spirodiclofen or spiromesifen;
s) Flubendiamide, chloranthraliniprole, or cyanthraniliprole;
t) Cyenopyrafen or cyflumetofen; or
u) Sulfoxaflor.

In addition to the major chemical classes of pesticide listed above, other pesticides having particular targets may be employed in the composition, if appropriate for the intended utility of the composition. For instance, selective insecticides for particular crops, for example stemborer specific insecticides (such as cartap) or hopper specific insecticides (such as buprofezin) for use in rice may be employed. Alternatively insecticides or acaricides specific for particular insect species/stages may also be included in the compositions (for example acaricidal ovo-larvicides, such as clofentezine, flubenzimine, hexythiazox or tetradifon; acaricidal motilicides, such as dicofol or propargite; acaricides, such as bromopropylate or chlorobenzilate; or growth regulators, such as hydramethylnon, cyromazine, methoprene, chlorfluazuron or diflubenzuron).

The following mixtures of the compounds of formula I with active ingredients are preferred, wherein, preferably, the term "TX" refers to a compound covered by the compounds of formula I or preferably the term "TX" refers to a compound selected from the Table A (compound A1 to compound A38):

an adjuvant selected from the group of substances consisting of an oil of vegetable or animal origin, a mineral oil, alkyl esters of such oils or mixtures of such oils, and petroleum oils (alternative name) (628) + TX,
an acaricide selected from the group of substances consisting of 1,1-bis(4-chlorophenyl)-2-ethoxyethanol (IUPAC name) (910) + TX, 2,4-dichlorophenyl benzenesulfonate (IUPAC/Chemical Abstracts name) (1059) + TX, 2-fluoro-*N*-methyl-*N*-1-naphthylacetamide (IUPAC name) (1295) + TX, 4-chlorophenyl phenyl sulfone (IUPAC name) (981) + TX, abamectin (1) + TX, acequinocyl (3) + TX, acetoprole [CCN] + TX, acrinathrin (9) + TX, aldicarb (16) + TX, aldoxycarb (863) + TX, alpha-cypermethrin (202) + TX, amidithion (870) + TX, amidoflumet [CCN] + TX, amidothioate (872) + TX, amiton (875) + TX, amiton hydrogen oxalate (875) + TX, amitraz (24) + TX, aramite (881) + TX, arsenous oxide (882) + TX, AVI 382 (compound code) + TX, AZ 60541 (compound code) + TX, azinphos-ethyl (44) + TX, azinphos-methyl (45) + TX, azobenzene (IUPAC name) (888) + TX, azocyclotin (46) + TX, azothoate (889) + TX, benomyl (62) + TX, benoxafos (alternative name) [CCN] + TX, benzoximate (71) + TX, benzyl benzoate (IUPAC name) [CCN] + TX, bifenazate (74) + TX, bifenthrin (76) + TX, binapacryl (907) + TX, brofenvalerate (alternative name) + TX, bromocyclen (918) + TX, bromophos (920) + TX, bromophos-ethyl (921) + TX, bromopropylate (94) + TX, buprofezin (99) + TX, butocarboxim (103) + TX, butoxycarboxim (104) + TX, butylpyridaben (alternative name) + TX, calcium polysulfide (IUPAC name) (111) + TX, camphechlor (941) + TX, carbanolate (943) + TX, carbaryl (115) + TX, carbofuran (118) + TX, carbophenothion (947) + TX, CGA 50'439 (development code) (125) + TX, chinomethionat (126) + TX, chlorbenside (959) + TX, chlordimeform (964) + TX, chlordimeform hydrochloride (964) + TX, chlorfenapyr (130) + TX, chlorfenethol (968) + TX, chlorfenson (970) + TX, chlorfensulphide (971) + TX, chlorfenvinphos (131) + TX, chlorobenzilate (975) + TX, chloromebuform (977) + TX, chloromethiuron (978) + TX, chloropropylate (983) + TX, chlorpyrifos (145) + TX, chlorpyrifos-methyl (146) + TX, chlorthiophos (994) + TX, cinerin I (696) + TX, cinerin II (696) + TX, cinerins (696) + TX, clofentezine (158) + TX, closantel (alternative name) [CCN] + TX, coumaphos (174) + TX, crotamiton (alternative name) [CCN] + TX, crotoxyphos (1010) + TX, cufraneb (1013) + TX, cyanthoate (1020) + TX, cyenopyrafen [CCN] + TX, cyflumetofen (CAS Reg. No.: 400882-07-7) + TX, cyhalothrin (196) + TX, cyhexatin (199) + TX, cypermethrin (201) + TX, DCPM (1032) + TX, DDT (219) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dialifos (1042) + TX, diazinon (227) + TX,

dichlofluanid (230) + TX, dichlorvos (236) + TX, dicliphos (alternative name) + TX, dicofol (242) + TX, dicrotophos (243) + TX, dienochlor (1071) + TX, diflovidazin [CCN] + TX, dimefox (1081) + TX, dimethoate (262) + TX, dinactin (alternative name) (653) + TX, dinex (1089) + TX, dinex-diclexine (1089) + TX, dinobuton (269) + TX, dinocap (270) + TX, dinocap-4 [CCN] + TX, dinocap-6 [CCN] + TX, dinocton (1090) + TX, dinopenton (1092) + TX, dinosulfon (1097) + TX, dinoterbon (1098) + TX, dioxathion (1102) + TX, diphenyl sulfone (IUPAC name) (1103) + TX, disulfiram (alternative name) [CCN] + TX, disulfoton (278) + TX, DNOC (282) + TX, dofenapyn (1113) + TX, doramectin (alternative name) [CCN] + TX, endosulfan (294) + TX, endothion (1121) + TX, EPN (297) + TX, eprinomectin (alternative name) [CCN] + TX, ethion (309) + TX, ethoate-methyl (1134) + TX, etoxazole (320) + TX, etrimfos (1142) + TX, fenazaflor (1147) + TX, fenazaquin (328) + TX, fenbutatin oxide (330) + TX, fenothiocarb (337) + TX, fenpropathrin (342) + TX, fenpyrad (alternative name) + TX, fenpyroximate (345) + TX, fenson (1157) + TX, fentrifanil (1161) + TX, fenvalerate (349) + TX, fipronil (354) + TX, fluacrypyrim (360) + TX, fluazuron (1166) + TX, flubenzimine (1167) + TX, flucycloxuron (366) + TX, flucythrinate (367) + TX, fluenetil (1169) + TX, flufenoxuron (370) + TX, flumethrin (372) + TX, fluorbenside (1174) + TX, fluvalinate (1184) + TX, FMC 1137 (development code) (1185) + TX, formetanate (405) + TX, formetanate hydrochloride (405) + TX, formothion (1192) + TX, formparanate (1193) + TX, gamma-HCH (430) + TX, glyodin (1205) + TX, halfenprox (424) + TX, heptenophos (432) + TX, hexadecyl cyclopropanecarboxylate (IUPAC/Chemical Abstracts name) (1216) + TX, hexythiazox (441) + TX, IKA 2002 (CAS Reg. No.: 211923-74-9) + TX, iodomethane (IUPAC name) (542) + TX, isocarbophos (alternative name) (473) + TX, isopropyl *O*-(methoxyaminothiophosphoryl)salicylate (IUPAC name) (473) + TX, ivermectin (alternative name) [CCN] + TX, jasmolin I (696) + TX, jasmolin II (696) + TX, jodfenphos (1248) + TX, lindane (430) + TX, lufenuron (490) + TX, malathion (492) + TX, malonoben (1254) + TX, mecarbam (502) + TX, mephosfolan (1261) + TX, mesulfen (alternative name) [CCN] + TX, methacrifos (1266) + TX, methamidophos (527) + TX, methidathion (529) + TX, methiocarb (530) + TX, methomyl (531) + TX, methyl bromide (537) + TX, metolcarb (550) + TX, mevinphos (556) + TX, mexacarbate (1290) + TX, milbemectin (557) + TX, milbemycin oxime (alternative name) [CCN] + TX, mipafox (1293) + TX, monocrotophos (561) + TX, morphothion (1300) + TX, moxidectin (alternative name) [CCN] + TX, naled (567) + TX, NC-184 (compound code) + TX, NC-512 (compound code) + TX, nifluridide (1309) + TX, nikkomycins (alternative name) [CCN] + TX, nitrilacarb (1313) + TX, nitrilacarb 1:1 zinc chloride complex (1313) + TX, NNI-0101 (compound code) + TX, NNI-0250 (compound code) + TX, omethoate (594) + TX, oxamyl (602) + TX, oxydeprofos (1324) + TX, oxydisulfoton (1325) + TX, pp'-DDT (219) + TX, parathion (615) + TX, permethrin (626) + TX, petroleum oils (alternative name) (628) + TX, phenkapton (1330) + TX, phenthoate (631) + TX, phorate (636) + TX, phosalone (637) + TX, phosfolan (1338) + TX, phosmet (638) + TX, phosphamidon (639) + TX, phoxim (642) + TX, pirimiphos-methyl (652) + TX, polychloroterpenes (traditional name) (1347) + TX, polynactins (alternative name) (653) + TX, proclonol (1350) + TX, profenofos (662) + TX, promacyl (1354) + TX, propargite (671) + TX, propetamphos (673) + TX, propoxur (678) + TX, prothidathion (1360) + TX, prothoate (1362) + TX, pyrethrin I (696) + TX, pyrethrin II (696) + TX, pyrethrins (696) + TX, pyridaben (699) + TX, pyridaphenthion (701) + TX, pyrimidifen (706) + TX, pyrimitate (1370) + TX, quinalphos (711) + TX, quintiofos (1381) + TX, R-1492 (development code) (1382) + TX, RA-17 (development code) (1383) + TX, rotenone (722) + TX, schradan (1389) + TX, sebufos (alternative name) + TX, selamectin (alternative name) [CCN] + TX, SI-0009 (compound code) + TX, sophamide (1402) + TX, spirodiclofen (738) + TX, spiromesifen (739) + TX, SSI-121 (development code) (1404) + TX, sulfiram (alternative name) [CCN] + TX, sulfluramid (750) + TX, sulfotep (753) + TX, sulfur (754) + TX, SZI-121 (development code) (757) + TX, tau-fluvalinate (398) + TX, tebufenpyrad (763) + TX, TEPP (1417) + TX, terbam (alternative name) + TX, tetrachlorvinphos (777) + TX, tetradifon (786) + TX, tetranactin (alternative name) (653) + TX, tetrasul (1425) + TX, thiafenox (alternative name) + TX, thiocarboxime (1431) + TX, thiofanox (800) + TX, thiometon (801) + TX, thioquinox (1436) + TX, thuringiensin (alternative name) [CCN] + TX, triamiphos (1441) + TX, triarathene (1443) + TX, triazophos (820) + TX, triazuron (alternative name) + TX, trichlorfon (824) + TX, trifenofos (1455) + TX, trinactin (alternative name) (653) + TX, vamidothion (847) + TX, vaniliprole [CCN] and YI-5302 (compound code) + TX,

an algicide selected from the group of substances consisting of bethoxazin [CCN] + TX, copper dioctanoate (IUPAC name) (170) + TX, copper sulfate (172) + TX, cybutryne [CCN] + TX, dichlone (1052) + TX, dichlorophen (232) + TX, endothal (295) + TX, fentin (347) + TX, hydrated lime [CCN] + TX, nabam (566) + TX, quinoclamine (714) + TX, quinonamid (1379) + TX, simazine (730) + TX, triphenyltin acetate (IUPAC name) (347) and triphenyltin hydroxide (IUPAC name) (347) + TX,

an anthelmintic selected from the group of substances consisting of abamectin (1) + TX, crufomate (1011) + TX, doramectin (alternative name) [CCN] + TX, emamectin (291) + TX, emamectin benzoate (291) + TX, eprinomectin (alternative name) [CCN] + TX, ivermectin (alternative name) [CCN] + TX, milbemycin oxime (alternative name) [CCN] + TX, moxidectin (alternative name) [CCN] + TX, piperazine [CCN] + TX, selamectin (alternative name) [CCN] + TX, spinosad (737) and thiophanate (1435) + TX,

an avicide selected from the group of substances consisting of chloralose (127) + TX, endrin (1122) + TX, fenthion (346) + TX, pyridin-4-amine (IUPAC name) (23) and strychnine (745) + TX,

a bactericide selected from the group of substances consisting of 1-hydroxy-1*H*-pyridine-2-thione (IUPAC name)

(1222) + TX, 4-(quinoxalin-2-ylamino)benzenesulfonamide (IUPAC name) (748) + TX, 8-hydroxyquinoline sulfate (446) + TX, bronopol (97) + TX, copper dioctanoate (IUPAC name) (170) + TX, copper hydroxide (IUPAC name) (169) + TX, cresol [CCN] + TX, dichlorophen (232) + TX, dipyrithione (1105) + TX, dodicin (1112) + TX, fenaminosulf (1144) + TX, formaldehyde (404) + TX, hydrargaphen (alternative name) [CCN] + TX, kasugamycin (483) + TX, kasugamycin hydrochloride hydrate (483) + TX, nickel bis(dimethyldithiocarbamate) (IUPAC name) (1308) + TX, nitrapyrin (580) + TX, octhilinone (590) + TX, oxolinic acid (606) + TX, oxytetracycline (611) + TX, potassium hydroxyquinoline sulfate (446) + TX, probenazole (658) + TX, streptomycin (744) + TX, streptomycin sesquisulfate (744) + TX, tecloftalam (766) + TX, and thiomersal (alternative name) [CCN] + TX,

a biological agent selected from the group of substances consisting of *Adoxophyes orana* GV (alternative name) (12) + TX, *Agrobacterium radiobacter* (alternative name) (13) + TX, *Amblyseius* spp. (alternative name) (19) + TX, *Anagrapha falcifera* NPV (alternative name) (28) + TX, *Anagrus atomus* (alternative name) (29) + TX, *Aphelinus abdominalis* (alternative name) (33) + TX, *Aphidius colemani* (alternative name) (34) + TX, *Aphidoletes aphidimyza* (alternative name) (35) + TX, *Autographa californica* NPV (alternative name) (38) + TX, *Bacillus firmus* (alternative name) (48) + TX, *Bacillus sphaericus* Neide (scientific name) (49) + TX, *Bacillus thuringiensis* Berliner (scientific name) (51) + TX, *Bacillus thuringiensis* subsp. *aizawai* (scientific name) (51) + TX, *Bacillus thuringiensis* subsp. *israelensis* (scientific name) (51) + TX, *Bacillus thuringiensis subsp. japonensis* (scientific name) (51) + TX, *Bacillus thuringiensis* subsp. *kurstaki* (scientific name) (51) + TX, *Bacillus thuringiensis* subsp. *tenebrionis* (scientific name) (51) + TX, *Beauveria bassiana* (alternative name) (53) + TX, *Beauveria brongniartii* (alternative name) (54) + TX, *Chrysoperla carnea* (alternative name) (151) + TX, *Cryptolaemus montrouzieri* (alternative name) (178) + TX, *Cydia pomonella* GV (alternative name) (191) + TX, *Dacnusa sibirica* (alternative name) (212) + TX, *Diglyphus isaea* (alternative name) (254) + TX, *Encarsia formosa* (scientific name) (293) + TX, *Eretmocerus eremicus* (alternative name) (300) + TX, *Helicoverpa zea* NPV (alternative name) (431) + TX, *Heterorhabditis bacteriophora* and *H. megidis* (alternative name) (433) + TX, *Hippodamia convergens* (alternative name) (442) + TX, *Leptomastix dactylopii* (alternative name) (488) + TX, *Macrolophus caliginosus* (alternative name) (491) + TX, *Mamestra brassicae* NPV (alternative name) (494) + TX, *Metaphycus helvolus* (alternative name) (522) + TX, *Metarhizium anisopliae* var. *acridum* (scientific name) (523) + TX, *Metarhizium anisopliae* var. *anisopliae* (scientific name) (523) + TX, *Neodiprion sertifer* NPV and *N. lecontei* NPV (alternative name) (575) + TX, *Orius* spp. (alternative name) (596) + TX, *Paecilomyces fumosoroseus* (alternative name) (613) + TX, *Phytoseiulus persimilis* (alternative name) (644) + TX, *Spodoptera exigua* multicapsid nuclear polyhedrosis virus (scientific name) (741) + TX, *Steinernema bibionis* (alternative name) (742) + TX, *Steinernema carpocapsae* (alternative name) (742) + TX, *Steinernema feltiae* (alternative name) (742) + TX, *Steinernema glaseri* (alternative name) (742) + TX, *Steinernema riobrave* (alternative name) (742) + TX, *Steinernema riobravis* (alternative name) (742) + TX, *Steinernema scapterisci* (alternative name) (742) + TX, *Steinernema* spp. (alternative name) (742) + TX, *Trichogramma* spp. (alternative name) (826) + TX, *Typhlodromus occidentalis* (alternative name) (844) and *Verticillium lecanii* (alternative name) (848) + TX,

a soil sterilant selected from the group of substances consisting of iodomethane (IUPAC name) (542) and methyl bromide (537) + TX,

a chemosterilant selected from the group of substances consisting of apholate [CCN] + TX, bisazir (alternative name) [CCN] + TX, busulfan (alternative name) [CCN] + TX, diflubenzuron (250) + TX, dimatif (alternative name) [CCN] + TX, hemel [CCN] + TX, hempa [CCN] + TX, metepa [CCN] + TX, methiotepa [CCN] + TX, methyl apholate [CCN] + TX, morzid [CCN] + TX, penfluron (alternative name) [CCN] + TX, tepa [CCN] + TX, thiohempa (alternative name) [CCN] + TX, thiotepa (alternative name) [CCN] + TX, tretamine (alternative name) [CCN] and uredepa (alternative name) [CCN] + TX,

an insect pheromone selected from the group of substances consisting of (E)-dec-5-en-1-yl acetate with (*E*)-dec-5-en-1-ol (IUPAC name) (222) + TX, (*E*)-tridec-4-en-1-yl acetate (IUPAC name) (829) + TX, (*E*)-6-methylhept-2-en-4-ol (IUPAC name) (541) + TX, (*E,Z*)-tetradeca-4,10-dien-1-yl acetate (IUPAC name) (779) + TX, (*Z*)-dodec-7-en-1-yl acetate (IUPAC name) (285) + TX, (Z)-hexadec-11-enal (IUPAC name) (436) + TX, (Z)-hexadec-11-en-1-yl acetate (IUPAC name) (437) + TX, (Z)-hexadec-13-en-11-yn-1-yl acetate (IUPAC name) (438) + TX, (*Z*)-icos-13-en-10-one (IUPAC name) (448) + TX, (Z)-tetradec-7-en-1-al (IUPAC name) (782) + TX, (*Z*)-tetradec-9-en-1-ol (IUPAC name) (783) + TX, (*Z*)-tetradec-9-en-1-yl acetate (IUPAC name) (784) + TX, (7*E,*9*Z*)-dodeca-7,9-dien-1-yl acetate (IUPAC name) (283) + TX, (9*Z,*11*E*)-tetradeca-9,11-dien-1-yl acetate (IUPAC name) (780) + TX, (9*Z*,12*E*)-tetradeca-9,12-dien-1-yl acetate (IUPAC name) (781) + TX, 14-methyloctadec-1-ene (IUPAC name) (545) + TX, 4-methylnonan-5-ol with 4-methylnonan-5-one (IUPAC name) (544) + TX, alpha-multistriatin (alternative name) [CCN] + TX, brevicomin (alternative name) [CCN] + TX, codlelure (alternative name) [CCN] + TX, codlemone (alternative name) (167) + TX, cuelure (alternative name) (179) + TX, disparlure (277) + TX, dodec-8-en-1-yl acetate (IUPAC name) (286) + TX, dodec-9-en-1-yl acetate (IUPAC name) (287) + TX, dodeca-8 + TX, 10-dien-1-yl acetate (IUPAC name) (284) + TX, dominicalure (alternative name) [CCN] + TX, ethyl 4-methyloctanoate (IUPAC name) (317) + TX, eugenol (alternative name) [CCN] + TX, frontalin (alternative name) [CCN] + TX, gossyplure (alternative name) (420) + TX, grandlure (421) + TX, grandlure I (alternative name) (421) + TX, grandlure II (alternative name)

(421) + TX, grandlure III (alternative name) (421) + TX, grandlure IV (alternative name) (421) + TX, hexalure [CCN] + TX, ipsdienol (alternative name) [CCN] + TX, ipsenol (alternative name) [CCN] + TX, japonilure (alternative name) (481) + TX, lineatin (alternative name) [CCN] + TX, litlure (alternative name) [CCN] + TX, looplure (alternative name) [CCN] + TX, medlure [CCN] + TX, megatomoic acid (alternative name) [CCN] + TX, methyl eugenol (alternative name) (540) + TX, muscalure (563) + TX, octadeca-2,13-dien-1-yl acetate (IUPAC name) (588) + TX, octadeca-3,13-dien-1-yl acetate (IUPAC name) (589) + TX, orfralure (alternative name) [CCN] + TX, oryctalure (alternative name) (317) + TX, ostramone (alternative name) [CCN] + TX, siglure [CCN] + TX, sordidin (alternative name) (736) + TX, sulcatol (alternative name) [CCN] + TX, tetradec-11-en-1-yl acetate (IUPAC name) (785) + TX, trimedlure (839) + TX, trimedlure A (alternative name) (839) + TX, trimedlure $B_1$ (alternative name) (839) + TX, trimedlure $B_2$ (alternative name) (839) + TX, trimedlure C (alternative name) (839) and trunc-call (alternative name) [CCN] + TX,

an insect repellent selected from the group of substances consisting of 2-(octylthio)-ethanol (IUPAC name) (591) + TX, butopyronoxyl (933) + TX, butoxy(polypropylene glycol) (936) + TX, dibutyl adipate (IUPAC name) (1046) + TX, dibutyl phthalate (1047) + TX, dibutyl succinate (IUPAC name) (1048) + TX, diethyltoluamide [CCN] + TX, dimethyl carbate [CCN] + TX, dimethyl phthalate [CCN] + TX, ethyl hexanediol (1137) + TX, hexamide [CCN] + TX, methoquin-butyl (1276) + TX, methylneodecanamide [CCN] + TX, oxamate [CCN] and picaridin [CCN] + TX,

an insecticide selected from the group of substances consisting of 1-dichloro-1-nitroethane (IUPAC/Chemical Abstracts name) (1058) + TX, 1,1-dichloro-2,2-bis(4-ethylphenyl)ethane (IUPAC name) (1056), + TX, 1,2-dichloropropane (IUPAC/Chemical Abstracts name) (1062) + TX, 1,2-dichloropropane with 1,3-dichloropropene (IUPAC name) (1063) + TX, 1-bromo-2-chloroethane (IUPAC/Chemical Abstracts name) (916) + TX, 2,2,2-trichloro-1-(3,4-dichlorophenyl)ethyl acetate (IUPAC name) (1451) + TX, 2,2-dichlorovinyl 2-ethylsulfinylethyl methyl phosphate (IUPAC name) (1066) + TX, 2-(1,3-dithiolan-2-yl)phenyl dimethylcarbamate (IUPAC/ Chemical Abstracts name) (1109) + TX, 2-(2-butoxyethoxy)ethyl thiocyanate (IUPAC/Chemical Abstracts name) (935) + TX, 2-(4,5-dimethyl-1,3-dioxolan-2-yl)phenyl methylcarbamate (IUPAC/ Chemical Abstracts name) (1084) + TX, 2-(4-chloro-3,5-xylyloxy)ethanol (IUPAC name) (986) + TX, 2-chlorovinyl diethyl phosphate (IUPAC name) (984) + TX, 2-imidazolidone (IUPAC name) (1225) + TX, 2-isovalerylindan-1,3-dione (IUPAC name) (1246) + TX, 2-methyl(prop-2-ynyl)aminophenyl methylcarbamate (IUPAC name) (1284) + TX, 2-thiocyanatoethyl laurate (IUPAC name) (1433) + TX, 3-bromo-1-chloroprop-1-ene (IUPAC name) (917) + TX, 3-methyl-1-phenylpyrazol-5-yl dimethylcarbamate (IUPAC name) (1283) + TX, 4-methyl(prop-2-ynyl)amino-3,5-xylyl methylcarbamate (IUPAC name) (1285) + TX, 5,5-dimethyl-3-oxocyclohex-1-enyl dimethylcarbamate (IUPAC name) (1085) + TX, abamectin (1) + TX, acephate (2) + TX, acetamiprid (4) + TX, acethion (alternative name) [CCN] + TX, acetoprole [CCN] + TX, acrinathrin (9) + TX, acrylonitrile (IUPAC name) (861) + TX, alanycarb (15) + TX, aldicarb (16) + TX, aldoxycarb (863) + TX, aldrin (864) + TX, allethrin (17) + TX, allosamidin (alternative name) [CCN] + TX, allyxycarb (866) + TX, alpha-cypermethrin (202) + TX, alpha-ecdysone (alternative name) [CCN] + TX, alpha-endosulfan [CCN] + TX, aluminium phosphide (640) + TX, amidithion (870) + TX, amidothioate (872) + TX, aminocarb (873) + TX, amiton (875) + TX, amiton hydrogen oxalate (875) + TX, amitraz (24) + TX, anabasine (877) + TX, athidathion (883) + TX, AVI 382 (compound code) + TX, AZ 60541 (compound code) + TX, azadirachtin (alternative name) (41) + TX, azamethiphos (42) + TX, azinphos-ethyl (44) + TX, azinphos-methyl (45) + TX, azothoate (889) + TX, *Bacillus thuringiensis* delta endotoxins (alternative name) (52) + TX, barium hexafluorosilicate (alternative name) [CCN] + TX, barium polysulfide (IUPAC/Chemical Abstracts name) (892) + TX, barthrin [CCN] + TX, Bayer 22/190 (development code) (893) + TX, Bayer 22408 (development code) (894) + TX, bendiocarb (58) + TX, benfuracarb (60) + TX, bensultap (66) + TX, beta-cyfluthrin (194) + TX, beta-cypermethrin (203) + TX, bifenthrin (76) + TX, bioallethrin (78) + TX, bioallethrin *S*-cyclopentenyl isomer (alternative name) (79) + TX, bioethanomethrin [CCN] + TX, biopermethrin (908) + TX, bioresmethrin (80) + TX, bis(2-chloroethyl) ether (IUPAC name) (909) + TX, bistrifluron (83) + TX, borax (86) + TX, brofenvalerate (alternative name) + TX, bromfenvinfos (914) + TX, bromocyclen (918) + TX, bromo-DDT (alternative name) [CCN] + TX, bromophos (920) + TX, bromophos-ethyl (921) + TX, bufencarb (924) + TX, buprofezin (99) + TX, butacarb (926) + TX, butathiofos (927) + TX, butocarboxim (103) + TX, butonate (932) + TX, butoxycarboxim (104) + TX, butylpyridaben (alternative name) + TX, cadusafos (109) + TX, calcium arsenate [CCN] + TX, calcium cyanide (444) + TX, calcium polysulfide (IUPAC name) (111) + TX, camphechlor (941) + TX, carbanolate (943) + TX, carbaryl (115) + TX, carbofuran (118) + TX, carbon disulfide (IUPAC/Chemical Abstracts name) (945) + TX, carbon tetrachloride (IUPAC name) (946) + TX, carbophenothion (947) + TX, carbosulfan (119) + TX, cartap (123) + TX, cartap hydrochloride (123) + TX, cevadine (alternative name) (725) + TX, chlorantraniliprole [CCN] + TX, chlorbicyclen (960) + TX, chlordane (128) + TX, chlordecone (963) + TX, chlordimeform (964) + TX, chlordimeform hydrochloride (964) + TX, chlorethoxyfos (129) + TX, chlorfenapyr (130) + TX, chlorfenvinphos (131) + TX, chlorfluazuron (132) + TX, chlormephos (136) + TX, chloroform [CCN] + TX, chloropicrin (141) + TX, chlorphoxim (989) + TX, chlorprazophos (990) + TX, chlorpyrifos (145) + TX, chlorpyrifos-methyl (146) + TX, chlorthiophos (994) + TX, chromafenozide (150) + TX, cinerin I (696) + TX, cinerin II (696) + TX, cinerins (696) + TX, cis-resmethrin (alternative name) + TX, cismethrin (80) + TX, clocythrin (alternative name) + TX, cloethocarb (999) + TX, closantel (alternative name) [CCN] + TX, clothianidin (165) + TX, copper acetoarsenite [CCN] + TX, copper arsenate [CCN] + TX, copper oleate [CCN] + TX,

coumaphos (174) + TX, coumithoate (1006) + TX, crotamiton (alternative name) [CCN] + TX, crotoxyphos (1010) + TX, crufomate (1011) + TX, cryolite (alternative name) (177) + TX, CS 708 (development code) (1012) + TX, cyanofenphos (1019) + TX, cyanophos (184) + TX, cyanthoate (1020) + TX, cyantraniliprole [CCN] + TX, cyclethrin [CCN] + TX, cycloprothrin (188) + TX, cyfluthrin (193) + TX, cyhalothrin (196) + TX, cypermethrin (201) + TX, cyphenothrin (206) + TX, cyromazine (209) + TX, cythioate (alternative name) [CCN] + TX, *d*-limonene (alternative name) [CCN] + TX, *d*-tetramethrin (alternative name) (788) + TX, DAEP (1031) + TX, dazomet (216) + TX, DDT (219) + TX, decarbofuran (1034) + TX, deltamethrin (223) + TX, demephion (1037) + TX, demephion-O (1037) + TX, demephion-S (1037) + TX, demeton (1038) + TX, demeton-methyl (224) + TX, demeton-O (1038) + TX, demeton-O-methyl (224) + TX, demeton-S (1038) + TX, demeton-S-methyl (224) + TX, demeton-S-methylsulphon (1039) + TX, diafenthiuron (226) + TX, dialifos (1042) + TX, diamidafos (1044) + TX, diazinon (227) + TX, dicapthon (1050) + TX, dichlofenthion (1051) + TX, dichlorvos (236) + TX, dicliphos (alternative name) + TX, dicresyl (alternative name) [CCN] + TX, dicrotophos (243) + TX, dicyclanil (244) + TX, dieldrin (1070) + TX, diethyl 5-methylpyrazol-3-yl phosphate (IUPAC name) (1076) + TX, diflubenzuron (250) + TX, dilor (alternative name) [CCN] + TX, dimefluthrin [CCN] + TX, dimefox (1081) + TX, dimetan (1085) + TX, dimethoate (262) + TX, dimethrin (1083) + TX, dimethylvinphos (265) + TX, dimetilan (1086) + TX, dinex (1089) + TX, dinex-diclexine (1089) + TX, dinoprop (1093) + TX, dinosam (1094) + TX, dinoseb (1095) + TX, dinotefuran (271) + TX, diofenolan (1099) + TX, dioxabenzofos (1100) + TX, dioxacarb (1101) + TX, dioxathion (1102) + TX, disulfoton (278) + TX, dithicrofos (1108) + TX, DNOC (282) + TX, doramectin (alternative name) [CCN] + TX, DSP (1115) + TX, ecdysterone (alternative name) [CCN] + TX, EI 1642 (development code) (1118) + TX, emamectin (291) + TX, emamectin benzoate (291) + TX, EMPC (1120) + TX, empenthrin (292) + TX, endosulfan (294) + TX, endothion (1121) + TX, endrin (1122) + TX, EPBP (1123) + TX, EPN (297) + TX, epofenonane (1124) + TX, eprinomectin (alternative name) [CCN] + TX, esfenvalerate (302) + TX, etaphos (alternative name) [CCN] + TX, ethiofencarb (308) + TX, ethion (309) + TX, ethiprole (310) + TX, ethoate-methyl (1134) + TX, ethoprophos (312) + TX, ethyl formate (IUPAC name) [CCN] + TX, ethyl-DDD (alternative name) (1056) + TX, ethylene dibromide (316) + TX, ethylene dichloride (chemical name) (1136) + TX, ethylene oxide [CCN] + TX, etofenprox (319) + TX, etrimfos (1142) + TX, EXD (1143) + TX, famphur (323) + TX, fenamiphos (326) + TX, fenazaflor (1147) + TX, fenchlorphos (1148) + TX, fenethacarb (1149) + TX, fenfluthrin (1150) + TX, fenitrothion (335) + TX, fenobucarb (336) + TX, fenoxacrim (1153) + TX, fenoxycarb (340) + TX, fenpirithrin (1155) + TX, fenpropathrin (342) + TX, fenpyrad (alternative name) + TX, fensulfothion (1158) + TX, fenthion (346) + TX, fenthion-ethyl [CCN] + TX, fenvalerate (349) + TX, fipronil (354) + TX, flonicamid (358) + TX, flubendiamide (CAS. Reg. No.: 272451-65-7) + TX, flucofuron (1168) + TX, flucycloxuron (366) + TX, flucythrinate (367) + TX, fluenetil (1169) + TX, flufenerim [CCN] + TX, flufenoxuron (370) + TX, flufenprox (1171) + TX, flumethrin (372) + TX, fluvalinate (1184) + TX, FMC 1137 (development code) (1185) + TX, fonofos (1191) + TX, formetanate (405) + TX, formetanate hydrochloride (405) + TX, formothion (1192) + TX, formparanate (1193) + TX, fosmethilan (1194) + TX, fospirate (1195) + TX, fosthiazate (408) + TX, fosthietan (1196) + TX, furathiocarb (412) + TX, furethrin (1200) + TX, gamma-cyhalothrin (197) + TX, gamma-HCH (430) + TX, guazatine (422) + TX, guazatine acetates (422) + TX, GY-81 (development code) (423) + TX, halfenprox (424) + TX, halofenozide (425) + TX, HCH (430) + TX, HEOD (1070) + TX, heptachlor (1211) + TX, heptenophos (432) + TX, heterophos [CCN] + TX, hexaflumuron (439) + TX, HHDN (864) + TX, hydramethylnon (443) + TX, hydrogen cyanide (444) + TX, hydroprene (445) + TX, hyquincarb (1223) + TX, imidacloprid (458) + TX, imiprothrin (460) + TX, indoxacarb (465) + TX, iodomethane (IUPAC name) (542) + TX, IPSP (1229) + TX, isazofos (1231) + TX, isobenzan (1232) + TX, isocarbophos (alternative name) (473) + TX, isodrin (1235) + TX, isofenphos (1236) + TX, isolane (1237) + TX, isoprocarb (472) + TX, isopropyl *O*-(methoxyaminothiophosphoryl)salicylate (IUPAC name) (473) + TX, isoprothiolane (474) + TX, isothioate (1244) + TX, isoxathion (480) + TX, ivermectin (alternative name) [CCN] + TX, jasmolin I (696) + TX, jasmolin II (696) + TX, jodfenphos (1248) + TX, juvenile hormone I (alternative name) [CCN] + TX, juvenile hormone II (alternative name) [CCN] + TX, juvenile hormone III (alternative name) [CCN] + TX, kelevan (1249) + TX, kinoprene (484) + TX, lambda-cyhalothrin (198) + TX, lead arsenate [CCN] + TX, lepimectin (CCN) + TX, leptophos (1250) + TX, lindane (430) + TX, lirimfos (1251) + TX, lufenuron (490) + TX, lythidathion (1253) + TX, *m*-cumenyl methylcarbamate (IUPAC name) (1014) + TX, magnesium phosphide (IUPAC name) (640) + TX, malathion (492) + TX, malonoben (1254) + TX, mazidox (1255) + TX, mecarbam (502) + TX, mecarphon (1258) + TX, menazon (1260) + TX, mephosfolan (1261) + TX, mercurous chloride (513) + TX, mesulfenfos (1263) + TX, metaflumizone (CCN) + TX, metam (519) + TX, metam-potassium (alternative name) (519) + TX, metam-sodium (519) + TX, methacrifos (1266) + TX, methamidophos (527) + TX, methanesulfonyl fluoride (IUPAC/Chemical Abstracts name) (1268) + TX, methidathion (529) + TX, methiocarb (530) + TX, methocrotophos (1273) + TX, methomyl (531) + TX, methoprene (532) + TX, methoquin-butyl (1276) + TX, methothrin (alternative name) (533) + TX, methoxychlor (534) + TX, methoxyfenozide (535) + TX, methyl bromide (537) + TX, methyl isothiocyanate (543) + TX, methylchloroform (alternative name) [CCN] + TX, methylene chloride [CCN] + TX, metofluthrin [CCN] + TX, metolcarb (550) + TX, metoxadiazone (1288) + TX, mevinphos (556) + TX, mexacarbate (1290) + TX, milbemectin (557) + TX, milbemycin oxime (alternative name) [CCN] + TX, mipafox (1293) + TX, mirex (1294) + TX, monocrotophos (561) + TX, morphothion (1300) + TX,

moxidectin (alternative name) [CCN] + TX, naftalofos (alternative name) [CCN] + TX, naled (567) + TX, naphthalene (IUPAC/Chemical Abstracts name) (1303) + TX, NC-170 (development code) (1306) + TX, NC-184 (compound code) + TX, nicotine (578) + TX, nicotine sulfate (578) + TX, nifluridide (1309) + TX, nitenpyram (579) + TX, nithiazine (1311) + TX, nitrilacarb (1313) + TX, nitrilacarb 1:1 zinc chloride complex (1313) + TX, NNI-0101 (compound code) + TX, NNI-0250 (compound code) + TX, nornicotine (traditional name) (1319) + TX, novaluron (585) + TX, noviflumuron (586) + TX, *O*-5-dichloro-4-iodophenyl *O*-ethyl ethylphosphonothioate (IUPAC name) (1057) + TX, *O*,*O*-diethyl *O*-4-methyl-2-oxo-2*H*-chromen-7-yl phosphorothioate (IUPAC name) (1074) + TX, *O*,*O*-diethyl *O*-6-methyl-2-propylpyrimidin-4-yl phosphorothioate (IUPAC name) (1075) + TX, *O,O,O',O'*-tetrapropyl dithiopyrophosphate (IUPAC name) (1424) + TX, oleic acid (IUPAC name) (593) + TX, omethoate (594) + TX, oxamyl (602) + TX, oxydemeton-methyl (609) + TX, oxydeprofos (1324) + TX, oxydisulfoton (1325) + TX, pp'-DDT (219) + TX, para-dichlorobenzene [CCN] + TX, parathion (615) + TX, parathion-methyl (616) + TX, penfluron (alternative name) [CCN] + TX, pentachlorophenol (623) + TX, pentachlorophenyl laurate (IUPAC name) (623) + TX, permethrin (626) + TX, petroleum oils (alternative name) (628) + TX, PH 60-38 (development code) (1328) + TX, phenkapton (1330) + TX, phenothrin (630) + TX, phenthoate (631) + TX, phorate (636) + TX, phosalone (637) + TX, phosfolan (1338) + TX, phosmet (638) + TX, phosnichlor (1339) + TX, phosphamidon (639) + TX, phosphine (IUPAC name) (640) + TX, phoxim (642) + TX, phoxim-methyl (1340) + TX, pirimetaphos (1344) + TX, pirimicarb (651) + TX, pirimiphos-ethyl (1345) + TX, pirimiphos-methyl (652) + TX, polychlorodicyclopentadiene isomers (IUPAC name) (1346) + TX, polychloroterpenes (traditional name) (1347) + TX, potassium arsenite [CCN] + TX, potassium thiocyanate [CCN] + TX, prallethrin (655) + TX, precocene I (alternative name) [CCN] + TX, precocene II (alternative name) [CCN] + TX, precocene III (alternative name) [CCN] + TX, primidophos (1349) + TX, profenofos (662) + TX, profluthrin [CCN] + TX, promacyl (1354) + TX, promecarb (1355) + TX, propaphos (1356) + TX, propetamphos (673) + TX, propoxur (678) + TX, prothidathion (1360) + TX, prothiofos (686) + TX, prothoate (1362) + TX, protrifenbute [CCN] + TX, pymetrozine (688) + TX, pyraclofos (689) + TX, pyrafluprole [CCN] + TX, pyrazophos (693) + TX, pyresmethrin (1367) + TX, pyrethrin I (696) + TX, pyrethrin II (696) + TX, pyrethrins (696) + TX, pyridaben (699) + TX, pyridalyl (700) + TX, pyridaphenthion (701) + TX, pyrifluquinazon [CCN] + TX, pyrimidifen (706) + TX, pyrimitate (1370) + TX, pyriprole [CCN] + TX, pyriproxyfen (708) + TX, quassia (alternative name) [CCN] + TX, quinalphos (711) + TX, quinalphosmethyl (1376) + TX, quinothion (1380) + TX, quintiofos (1381) + TX, R-1492 (development code) (1382) + TX, rafoxanide (alternative name) [CCN] + TX, resmethrin (719) + TX, rotenone (722) + TX, RU 15525 (development code) (723) + TX, RU 25475 (development code) (1386) + TX, ryania (alternative name) (1387) + TX, ryanodine (traditional name) (1387) + TX, sabadilla (alternative name) (725) + TX, schradan (1389) + TX, sebufos (alternative name) + TX, selamectin (alternative name) [CCN] + TX, SI-0009 (compound code) + TX, SI-0205 (compound code) + TX, SI-0404 (compound code) + TX, SI-0405 (compound code) + TX, silafluofen (728) + TX, SN 72129 (development code) (1397) + TX, sodium arsenite [CCN] + TX, sodium cyanide (444) + TX, sodium fluoride (IUPAC/Chemical Abstracts name) (1399) + TX, sodium hexafluorosilicate (1400) + TX, sodium pentachlorophenoxide (623) + TX, sodium selenate (IUPAC name) (1401) + TX, sodium thiocyanate [CCN] + TX, sophamide (1402) + TX, spinetoram [CCN] + TX, spinosad (737) + TX, spiromesifen (739) + TX, spirotetramat [CCN] + TX, sulcofuron (746) + TX, sulcofuron-sodium (746) + TX, sulfluramid (750) + TX, sulfotep (753) + TX, sulfoxaflor [CCN] + TX, sulfuryl fluoride (756) + TX, sulprofos (1408) + TX, tar oils (alternative name) (758) + TX, tau-fluvalinate (398) + TX, tazimcarb (1412) + TX, TDE (1414) + TX, tebufenozide (762) + TX, tebufenpyrad (763) + TX, tebupirimfos (764) + TX, teflubenzuron (768) + TX, tefluthrin (769) + TX, temephos (770) + TX, TEPP (1417) + TX, terallethrin (1418) + TX, terbam (alternative name) + TX, terbufos (773) + TX, tetrachloroethane [CCN] + TX, tetrachlorvinphos (777) + TX, tetramethrin (787) + TX, tetramethylfluthrin (CAS. Reg. No.: 84937-88-2) + TX, thetacypermethrin (204) + TX, thiacloprid (791) + TX, thiafenox (alternative name) + TX, thiamethoxam (792) + TX, thicrofos (1428) + TX, thiocarboxime (1431) + TX, thiocyclam (798) + TX, thiocyclam hydrogen oxalate (798) + TX, thiodicarb (799) + TX, thiofanox (800) + TX, thiometon (801) + TX, thionazin (1434) + TX, thiosultap (803) + TX, thiosultap-sodium (803) + TX, thuringiensin (alternative name) [CCN] + TX, tolfenpyrad (809) + TX, tralomethrin (812) + TX, transfluthrin (813) + TX, transpermethrin (1440) + TX, triamiphos (1441) + TX, triazamate (818) + TX, triazophos (820) + TX, triazuron (alternative name) + TX, trichlorfon (824) + TX, trichlormetaphos-3 (alternative name) [CCN] + TX, trichloronat (1452) + TX, trifenofos (1455) + TX, triflumuron (835) + TX, trimethacarb (840) + TX, triprene (1459) + TX, vamidothion (847) + TX, vaniliprole [CCN] + TX, veratridine (alternative name) (725) + TX, veratrine (alternative name) (725) + TX, XMC (853) + TX, xylylcarb (854) + TX, YI-5302 (compound code) + TX, zeta-cypermethrin (205) + TX, zetamethrin (alternative name) + TX, zinc phosphide (640) + TX, zolaprofos (1469) and ZXI 8901 (development code) (858) + TX, a molluscicide selected from the group of substances consisting of bis(tributyltin) oxide (IUPAC name) (913) + TX, bromoacetamide [CCN] + TX, calcium arsenate [CCN] + TX, cloethocarb (999) + TX, copper acetoarsenite [CCN] + TX, copper sulfate (172) + TX, fentin (347) + TX, ferric phosphate (IUPAC name) (352) + TX, metaldehyde (518) + TX, methiocarb (530) + TX, niclosamide (576) + TX, niclosamide-olamine (576) + TX, pentachlorophenol (623) + TX, sodium pentachlorophenoxide (623) + TX, tazimcarb (1412) + TX, thiodicarb (799) + TX, tralopyril [CCN] + TX, tributyltin oxide (913) + TX, trifenmorph (1454) + TX, trimethacarb (840) + TX, triphenyltin acetate (IUPAC name)

(347) and triphenyltin hydroxide (IUPAC name) (347) + TX,

a nematicide selected from the group of substances consisting of AKD-3088 (compound code) + TX, 1,2-dibromo-3-chloropropane (IUPAC/Chemical Abstracts name) (1045) + TX, 1,2-dichloropropane (IUPAC/ Chemical Abstracts name) (1062) + TX, 1,2-dichloropropane with 1,3-dichloropropene (IUPAC name) (1063) + TX, 1,3-dichloropropene (233) + TX, 3,4-dichlorotetrahydrothiophene 1,1-dioxide (IUPAC/Chemical Abstracts name) (1065) + TX, 3-(4-chlorophenyl)-5-methylrhodanine (IUPAC name) (980) + TX, 5-methyl-6-thioxo-1,3,5-thiadiazinan-3-ylacetic acid (IUPAC name) (1286) + TX, 6-isopentenylaminopurine (alternative name) (210) + TX, abamectin (1) + TX, acetoprole [CCN] + TX, alanycarb (15) + TX, aldicarb (16) + TX, aldoxycarb (863) + TX, AZ 60541 (compound code) + TX, benclothiaz [CCN] + TX, benomyl (62) + TX, butylpyridaben (alternative name) + TX, cadusafos (109) + TX, carbofuran (118) + TX, carbon disulfide (945) + TX, carbosulfan (119) + TX, chloropicrin (141) + TX, chlorpyrifos (145) + TX, cloethocarb (999) + TX, cytokinins (alternative name) (210) + TX, dazomet (216) + TX, DBCP (1045) + TX, DCIP (218) + TX, diamidafos (1044) + TX, dichlofenthion (1051) + TX, dicliphos (alternative name) + TX, dimethoate (262) + TX, doramectin (alternative name) [CCN] + TX, emamectin (291) + TX, emamectin benzoate (291) + TX, eprinomectin (alternative name) [CCN] + TX, ethoprophos (312) + TX, ethylene dibromide (316) + TX, fenamiphos (326) + TX, fenpyrad (alternative name) + TX, fensulfothion (1158) + TX, fluensulfone (CAS. Reg. No.: 318290-98-1) + TX, fosthiazate (408) + TX, fosthietan (1196) + TX, furfural (alternative name) [CCN] + TX, GY-81 (development code) (423) + TX, heterophos [CCN] + TX, imicyafos [CCN] + TX, iodomethane (IUPAC name) (542) + TX, isamidofos (1230) + TX, isazofos (1231) + TX, ivermectin (alternative name) [CCN] + TX, kinetin (alternative name) (210) + TX, mecarphon (1258) + TX, metam (519) + TX, metam-potassium (alternative name) (519) + TX, metam-sodium (519) + TX, methyl bromide (537) + TX, methyl isothiocyanate (543) + TX, milbemycin oxime (alternative name) [CCN] + TX, moxidectin (alternative name) [CCN] + TX, *Myrothecium verrucaria* composition (alternative name) (565) + TX, NC-184 (compound code) + TX, oxamyl (602) + TX, phorate (636) + TX, phosphamidon (639) + TX, phosphocarb [CCN] + TX, sebufos (alternative name) + TX, selamectin (alternative name) [CCN] + TX, spinosad (737) + TX, terbam (alternative name) + TX, terbufos (773) + TX, tetrachlorothiophene (IUPAC/ Chemical Abstracts name) (1422) + TX, thiafenox (alternative name) + TX, thionazin (1434) + TX, triazophos (820) + TX, triazuron (alternative name) + TX, xylenols [CCN] + TX, YI-5302 (compound code) and zeatin (alternative name) (210) + TX,

a nitrification inhibitor selected from the group of substances consisting of potassium ethylxanthate [CCN] and nitrapyrin (580) + TX,

a plant activator selected from the group of substances consisting of acibenzolar (6) + TX, acibenzolar-*S*-methyl (6) + TX, probenazole (658) and *Reynoutria sachalinensis* extract (alternative name) (720) + TX,

a rodenticide selected from the group of substances consisting of 2-isovalerylindan-1,3-dione (IUPAC name) (1246) + TX, 4-(quinoxalin-2-ylamino)benzenesulfonamide (IUPAC name) (748) + TX, alpha-chlorohydrin [CCN] + TX, aluminium phosphide (640) + TX, antu (880) + TX, arsenous oxide (882) + TX, barium carbonate (891) + TX, bisthiosemi (912) + TX, brodifacoum (89) + TX, bromadiolone (91) + TX, bromethalin (92) + TX, calcium cyanide (444) + TX, chloralose (127) + TX, chlorophacinone (140) + TX, cholecalciferol (alternative name) (850) + TX, coumachlor (1004) + TX, coumafuryl (1005) + TX, coumatetralyl (175) + TX, crimidine (1009) + TX, difenacoum (246) + TX, difethialone (249) + TX, diphacinone (273) + TX, ergocalciferol (301) + TX, flocoumafen (357) + TX, fluoroacetamide (379) + TX, flupropadine (1183) + TX, flupropadine hydrochloride (1183) + TX, gamma-HCH (430) + TX, HCH (430) + TX, hydrogen cyanide (444) + TX, iodomethane (IUPAC name) (542) + TX, lindane (430) + TX, magnesium phosphide (IUPAC name) (640) + TX, methyl bromide (537) + TX, norbormide (1318) + TX, phosacetim (1336) + TX, phosphine (IUPAC name) (640) + TX, phosphorus [CCN] + TX, pindone (1341) + TX, potassium arsenite [CCN] + TX, pyrinuron (1371) + TX, scilliroside (1390) + TX, sodium arsenite [CCN] + TX, sodium cyanide (444) + TX, sodium fluoroacetate (735) + TX, strychnine (745) + TX, thallium sulfate [CCN] + TX, warfarin (851) and zinc phosphide (640) + TX,

a synergist selected from the group of substances consisting of 2-(2-butoxyethoxy)-ethyl piperonylate (IUPAC name) (934) + TX, 5-(1,3-benzodioxol-5-yl)-3-hexylcyclohex-2-enone (IUPAC name) (903) + TX, farnesol with nerolidol (alternative name) (324) + TX, MB-599 (development code) (498) + TX, MGK 264 (development code) (296) + TX, piperonyl butoxide (649) + TX, piprotal (1343) + TX, propyl isomer (1358) + TX, S421 (development code) (724) + TX, sesamex (1393) + TX, sesasmolin (1394) and sulfoxide (1406) + TX,

an animal repellent selected from the group of substances consisting of anthraquinone (32) + TX, chloralose (127) + TX, copper naphthenate [CCN] + TX, copper oxychloride (171) + TX, diazinon (227) + TX, dicyclopentadiene (chemical name) (1069) + TX, guazatine (422) + TX, guazatine acetates (422) + TX, methiocarb (530) + TX, pyridin-4-amine (IUPAC name) (23) + TX, thiram (804) + TX, trimethacarb (840) + TX, zinc naphthenate [CCN] and ziram (856) + TX,

a virucide selected from the group of substances consisting of imanin (alternative name) [CCN] and ribavirin (alternative name) [CCN] + TX,

a wound protectant selected from the group of substances consisting of mercuric oxide (512) + TX, octhilinone (590) and thiophanate-methyl (802) + TX,

an insecticide selected from the group consisting of the compound of

the formula A-1

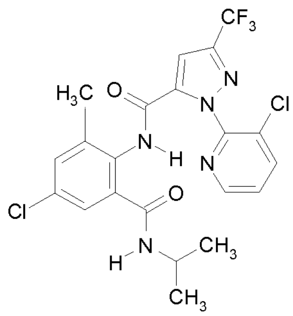

(A-1) + TX,

the formula A-2

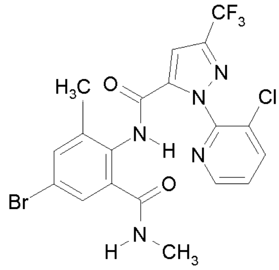

(A-2) + TX

the formula A-3

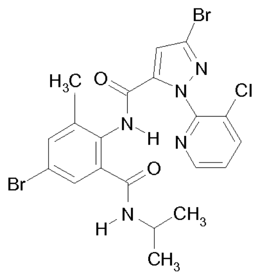

(A-3) + TX,

the formula A-4

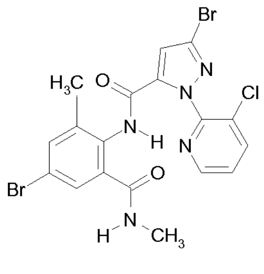

(A-4) + TX,

the formula A-5

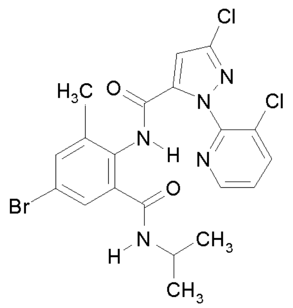

(A-5) + TX,

the formula A-6

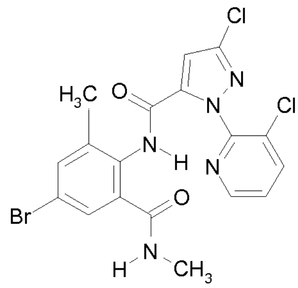

(A-6) + TX,

the formula A-7

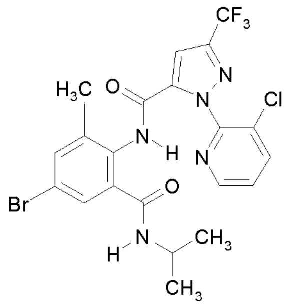

(A-7) + TX,

the formula A-8

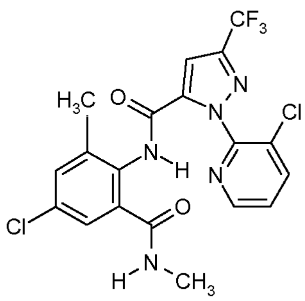

(A-8) + TX,

the formula A-9

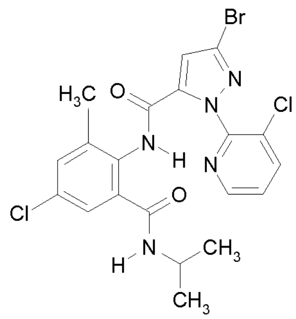

(A-9) + TX,

the formula A-10

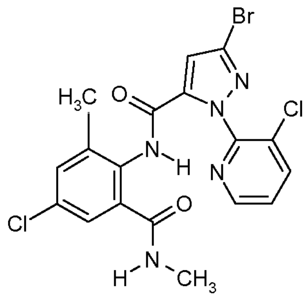

(A-10) + TX,

the formula A-11

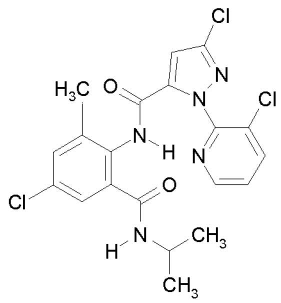

(A-11) + TX,

the formula A-12

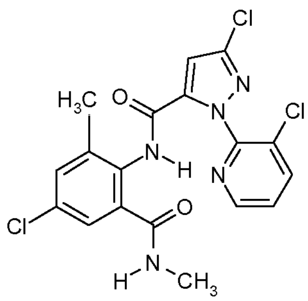

(A-12) + TX,

the formula A-13

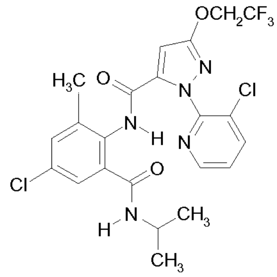

(A-13) + TX,

the formula A-14

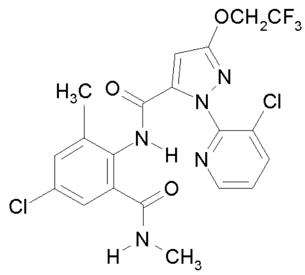

(A-14) + TX,

the formula A-15

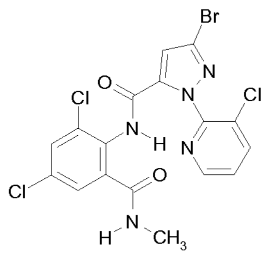

(A-15) + TX

the formula A-16

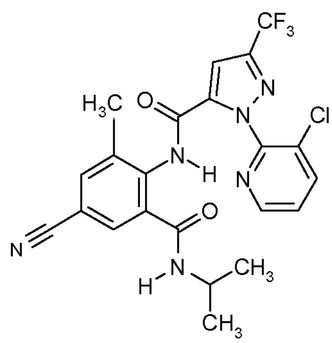

(A-16) + TX,

the formula A-17

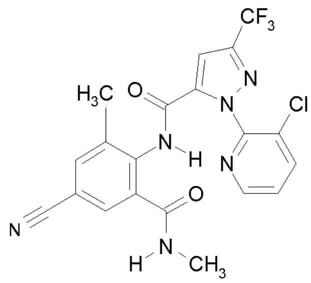 (A-17) + TX,

the formula A-18

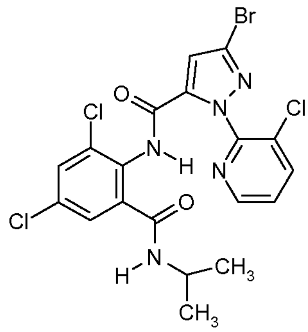 (A-18) + TX,

the formula A-19

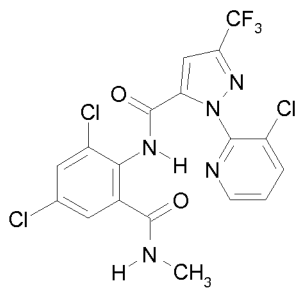 (A-19) + TX,

the formula A-20

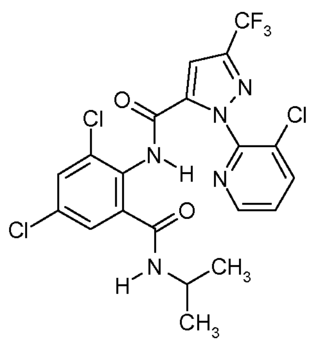 (A-20) + TX,

the formula A-21

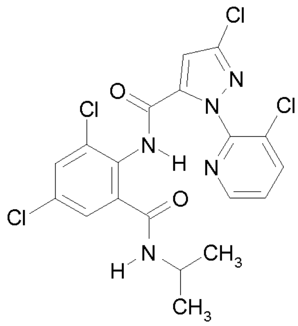 (A-21) + TX

the formula A-22

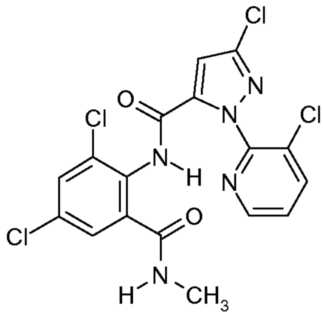 (A-22) + TX,

the formula A-23

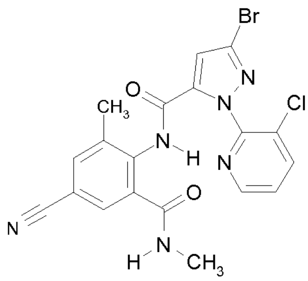 (A-23) + TX,

the formula A-24

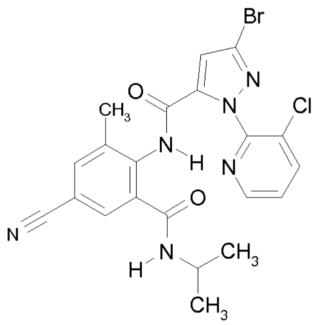 (A-24) + TX,

the formula A-25

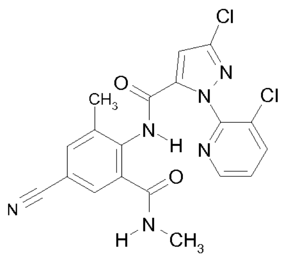

(A-25) + TX,

the formula A-26

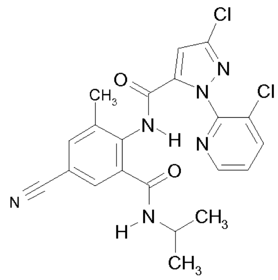

(A-26) + TX,

and the formula A-27

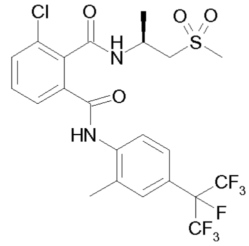

(A-27) + TX.

The references in brackets behind the active ingredients, e.g. [3878-19-1] refer to the Chemical Abstracts Registry number. The compounds of the formula A-1 to A-26 are described in WO 03/015518 or in WO 04/067528. The compound of the formula A-27 is described in WO 06/022225 and in WO 07/112844. The above described mixing partners are known. Where the active ingredients are included in "The Pesticide Manual" [The Pesticide Manual - A World Compendium; Thirteenth Edition; Editor: C. D. S. TomLin; The British Crop Protection Council], they are described therein under the entry number given in round brackets hereinabove for the particular compound; for example, the compound "abamectin" is described under entry number (1). Where "[CCN]" is added hereinabove to the particular compound, the compound in question is included in the "Compendium of Pesticide Common Names", which is accessible on the internet [A. Wood; Compendium of Pesticide Common Names, Copyright © 1995-2004]; for example, the compound "acetoprole" is described under the internet address http://www.alanwood.net/pesticides/acetoprole.htmL.

Most of the active ingredients described above are referred to hereinabove by a so-called "common name", the relevant "ISO common name" or another "common name" being used in individual cases. If the designation is not a "common name", the nature of the designation used instead is given in round brackets for the particular compound; in that case, the IUPAC name, the IUPAC/Chemical Abstracts name, a "chemical name", a "traditional name", a "compound name" or a "develoment code" is used or, if neither one of those designations nor a "common name" is used, an "alternative name" is employed. "CAS Reg. No" means the Chemical Abstracts Registry Number.

The compounds of formula I according to the invention can also be used in combination with one or more fungicides. In particular, in the following mixtures of the compounds of formula I with fungicides, the term TX preferably refers to a compound selected from one of the Table A (compound A1 to compound A38):

TX + (*E*)-*N*-methyl-2-[2-(2,5-dimethylphenoxymethyl)phenyl]-2-methoxy-iminoacetamide (SSF-129), TX + 4-bromo-2-cyano-*N*,*N*-dimethyl-6-trifluoromethylbenzimidazole-1-sulphonamide, TX + $\alpha$-[*N*-(3-chloro-2,6-xylyl)-2-methoxy-acetamido]-$\gamma$-butyrolactone, TX + 4-chloro-2-cyano-*N*,*N*-dimethyl-5-*p*-tolylimidazole-1-sulfonamide (IKF-916, cyamidazosulfamid), TX + 3-5-dichloro-*N*-(3-chloro-1-ethyl-1-methyl-2-oxopropyl)-4-methylbenzamide (RH-7281, zoxamide), TX + *N*-allyl-4,5,-dimethyl-2-trimethylsilylthiophene-3-carboxamide (MON65500), TX + *N*-(1-cyano-1,2-dimethylpropyl)-2-(2,4-dichlorophenoxy)propionamide (AC382042), TX + *N*-(2-methoxy-5-pyridyl)-cyclopropane carboxamide, TX + acibenzolar, TX + alanycarb, TX + aldimorph, TX + amisulbrom, TX + anilazine, TX + azaconazole, TX + azoxystrobin, TX + benalaxyl, TX + benalaxyl-M, TX + benomyl, TX + benthiavalicarb, TX + biloxazol, TX + bitertanol, TX + bixafen, TX + blasticidin S, TX + boscalid, TX + bromuconazole, TX + bupirimate, TX + captafol, TX + captan, TX + carbendazim, TX + carbendazim chlorhydrate, TX + carboxin, TX + carpropamid, carvone, TX + CGA41396, TX + CGA41397, TX + chinomethionate, TX + chlazafenone, TX + chlorothalonil, TX + chlorozolinate, TX + clozylacon, TX + copper containing compounds such as copper oxychloride, copper oxyquinolate, copper sulphate, copper tallate and Bordeaux mixture, TX + cyazofamid, TX + cyflufenamid, TX + cymoxanil, TX + cyproconazole, TX + cyprodinil, TX + debacarb, TX + di-2-pyridyl disulphide 1,1'-dioxide, TX + dichlofluanid, TX + diclomezine, TX + dicloran, TX + diethofencarb, TX + difenoconazole, TX + difenzoquat, TX + diflumetorim, TX + *O*,*O*-di-*iso*-propyl-*S*-benzyl thiophosphate, TX + dimefluazole, TX + dimetconazole, TX + dimethomorph, TX + dimethirimol, TX + dimoxystrobin, TX + diniconazole, TX + dinocap, TX + dithianon, TX + dodecyl dimethyl ammonium chloride, TX + dodemorph, TX + dodine, TX + doguadine, TX + edifenphos, TX + epoxiconazole, TX + ethirimol, TX + ethyl(*Z*)-*N*-benzyl-*N*([methyl(methyl-thioethylideneaminooxycarbonyl)amino]thio)--β-alaninate, TX + etridiazole, TX + famoxadone, TX + fenamidone (RPA407213), TX + fenarimol, TX + fenbuconazole, TX + fenfuram, TX + fenhexamid (KBR2738), TX + fenoxanil, TX + fenpiclonil, TX + fenpropidin, TX + fenpropimorph, TX + fenpyrazamine/ipfenpyrazolone, TX + fentin acetate, TX + fentin hydroxide, TX + ferbam, TX + ferimzone, TX + fluazinam, TX + fludioxonil, TX + flumetover, TX + flumorph, TX + fluopicolide, TX + fluopyram, TX + fluoxastrobin, TX + fluoroimide, TX + fluquinconazole, TX + flusilazole, TX + flutianil, TX + flutolanil, TX + flutriafol, TX + fluxapyroxad, TX + folpet, TX + fuberidazole, TX + furalaxyl, TX + furametpyr, TX + guazatine, TX + hexaconazole, TX + hydroxyisoxazole, TX + hymexazole, TX + imazalil, TX + imibenconazole, TX + iminoctadine, TX + iminoctadine triacetate, TX + ipconazole, TX + iprobenfos, TX + iprodione, TX + iprovalicarb (SZX0722), TX + isopropanyl butyl carbamate, TX + isoprothiolane, TX + isopyrazam, TX + isotianil, TX + kasugamycin, TX + kresoxim-methyl, TX + LY186054, TX + LY211795, TX + LY248908, TX + mancozeb, TX + mandipropamid, TX + maneb, TX + mefenoxam, TX + mepanipyrim, TX + mepronil, TX + meptyldinocap, TX + metalaxyl, TX + metconazole, TX + metiram, TX + metiram-zinc, TX + metominostrobin, TX + metrafenone, TX + myclobutanil, TX + neoasozin, TX + nickel dimethyldithiocarbamate, TX + nicobifen, TX + nitrothal-*iso*propyl, TX + nuarimol, TX + ofurace, TX + organomercury compounds, TX + orysastrobin, TX + oxadixyl, TX + oxasulfuron, TX + oxolinic acid, TX + oxpoconazole, TX + oxycarboxin, TX + pefurazoate, TX + penconazole, TX + pencycuron, TX + penthiopyrad, TX + phenazin oxide, TX + phosetyl-Al, TX + phosphorus acids, TX + phthalide, TX + picoxystrobin (ZA1963), TX + polyoxin D, TX + polyram, TX + probenazole, TX + prochloraz, TX + procymidone, TX + propamocarb, TX + propiconazole, TX + propineb, TX + propionic acid, TX + proquinazid, TX + prothioconazole, TX + pyraclostrobin, TX + pyrazophos, TX + pyribencarb, TX + pyrifenox, TX + pyrimethanil, TX + pyroquilon, TX + pyroxyfur, TX + pyrrolnitrin, TX + quaternary ammonium compounds, TX + quinomethionate, TX + quinoxyfen, TX + quintozene, TX + sedaxane, TX + sipconazole (F-155), TX + sodium pentachlorophenate, TX + spiroxamine, TX + streptomycin, TX + sulphur, TX + tebuconazole, TX + tecloftalam, TX + tecnazene, TX + tetraconazole, TX + thiabendazole, TX + thifluzamid, TX + 2-(thiocyanomethylthio)benzothiazole, TX + thiophanate-methyl, TX + thiram, TX + tiadinil, TX + timibenconazole, TX + tolclofos-methyl, TX + tolylfluanid, TX + triadimefon, TX + triadimenol, TX + triazbutil, TX + triazoxide, TX + tricyclazole, TX + tridemorph, TX + trifloxystrobin, TX + triforine, TX + triflumizole, TX + triticonazole, TX + validamycin A, TX + valiphenal, TX + vapam, TX + vinclozolin, TX + zineb and TX + ziram.

The compounds of formula I may be mixed with soil, peat or other rooting media for the protection of plants against seed-borne, soil-borne or foliar fungal diseases.

The compounds of formula I according to the invention can also be used in combination with one or more other synergists. In particular, the following mixtures of the TX, where this term preferably refers to a compound selected from one of the Table A (compound A1 to compound A38), are important:

TX + piperonyl butoxide, TX + sesamex, TX + safroxan and TX + dodecyl imidazole.

The compounds of formula I according to the invention can also be used in combination with one or more other herbicides. In particular, the following mixtures of the TX, where this term preferably refers to a compound selected from one of the Table A (compound A1 to compound A38), are important:

TX + acetochlor, TX + acifluorfen, TX + acifluorfen-sodium, TX + aclonifen, TX + acrolein, TX + alachlor, TX + alloxydim, TX + allyl alcohol, TX + ametryn, TX + amicarbazone, TX + amidosulfuron, TX + aminocyclopyrachlor, TX + aminopyralid, TX + amitrole, TX + ammonium sulfamate, TX + anilofos, TX + asulam, TX + atraton, TX +

atrazine, TX + azimsulfuron, TX + BCPC, TX + beflubutamid, TX + benazolin, TX +bencarbazone, TX + benfluralin, TX + benfuresate, TX + bensulfuron, TX + bensulfuron-methyl, TX + bensulide, TX + bentazone, TX + benzfendizone, TX + benzobicyclon, TX + benzofenap, COMPOUND OF THE FORMULA I + bicyclopyrone, TX + bifenox, TX + bilanafos, TX + bispyribac, TX + bispyribac-sodium, TX + borax, TX + bromacil, TX + bromobutide, TX + bromoxynil, TX + butachlor, TX + butafenacil, TX + butamifos, TX + butralin, TX + butroxydim, TX + butylate, TX + cacodylic acid, TX + calcium chlorate, TX + cafenstrole, TX + carbetamide, TX + carfentrazone, TX + carfentrazone-ethyl, TX + CDEA, TX + CEPC, TX + chlorflurenol, TX + chlorflurenol-methyl, TX + chloridazon, TX + chlorimuron, TX + chlorimuron-ethyl, TX + chloroacetic acid, TX + chlorotoluron, TX + chlorpropham, TX + chlorsulfuron, TX + chlorthal, TX + chlorthal-dimethyl, TX + cinidonethyl, TX + cinmethylin, TX + cinosulfuron, TX + cisanilide, TX + clethodim, TX + clodinafop, TX + clodinafop-propargyl, TX + clomazone, TX + clomeprop, TX + clopyralid, TX + cloransulam, TX + cloransulam-methyl, TX + CMA, TX + 4-CPB, TX + CPMF, TX + 4-CPP, TX + CPPC, TX + cresol, TX + cumyluron, TX + cyanamide, TX + cyanazine, TX + cycloate, TX + cyclosulfamuron, TX + cycloxydim, TX + cyhalofop, TX + cyhalofop-butyl, TX + 2,4-D, TX + 3,4-DA, TX + daimuron, TX + dalapon, TX + dazomet, TX + 2,4-DB, TX + 3,4-DB, TX + 2,4-DEB, TX + desmedipham, TX + dicamba, TX + dichlobenil, TX + ortho-dichlorobenzene, TX + para-dichlorobenzene, TX + dichlorprop, TX + dichlorprop-P, TX + diclofop, TX + diclofop-methyl, TX + diclosulam, TX + difenzoquat, TX + difenzoquat metilsulfate, TX + diflufenican, TX + diflufenzopyr, TX + dimefuron, TX + dimepiperate, TX + dimethachlor, TX + dimethametryn, TX + dimethenamid, TX + dimethenamid-P, TX + dimethipin, TX + dimethylarsinic acid, TX + dinitramine, TX + dinoterb, TX + diphenamid, TX + diquat, TX + diquat dibromide, TX + dithiopyr, TX + diuron, TX + DNOC, TX + 3,4-DP, TX + DSMA, TX + EBEP, TX + endothal, TX + EPTC, TX + esprocarb, TX + ethalfluralin, TX + ethametsulfuron, TX + ethametsulfuron-methyl, TX + ethofumesate, TX + ethoxyfen, TX + ethoxysulfuron, TX + etobenzanid, TX + fenoxaprop-P, TX + fenoxaprop-P-ethyl, TX + fentrazamide, TX + ferrous sulfate, TX + flamprop-M, TX + flazasulfuron, TX + florasulam, TX + fluazifop, TX + fluazifop-butyl, TX + fluazifop-P, TX + fluazifop-P-butyl, TX + flucarbazone, TX + flucarbazone-sodium, TX + flucetosulfuron, TX + fluchloralin, TX + flufenacet, TX + flufenpyr, TX + flufenpyrethyl, TX + flumetsulam, TX + flumiclorac, TX + flumiclorac-pentyl, TX + flumioxazin, TX + fluometuron, TX + fluoroglycofen, TX + fluoroglycofen-ethyl, TX + flupropanate, TX + flupyrsulfuron, TX + flupyrsulfuron-methyl-sodium, TX + flurenol, TX + fluridone, TX + flurochloridone, TX + fluroxypyr, TX + flurtamone, TX + fluthiacet, TX + fluthiacet-methyl, TX + fomesafen, TX + foramsulfuron, TX + fosamine, TX + glufosinate, TX + glufosinate-ammonium, TX + glufosinate-P, TX + glyphosate, TX + glyphosate-trimesium, TX + halosulfuron, TX + halosulfuron-methyl, TX + haloxyfop, TX + haloxyfop-P, TX + HC-252, TX + hexazinone, TX + imazamethabenz, TX + imazamethabenz-methyl, TX + imazamox, TX + imazapic, TX + imazapyr, TX + imazaquin, TX + imazethapyr, TX + imazosulfuron, TX + indanofan, TX + indaziflam, TX + iodomethane, TX + iodosulfuron, TX + iodosulfuron-methyl-sodium, TX + ioxynil, TX + ipfencarbazone, TX + isoproturon, TX + isouron, TX + isoxaben, TX + isoxachlortole, TX + isoxaflutole, TX + karbutilate, TX + lactofen, TX + lenacil, TX + linuron, TX + MAA, TX + MAMA, TX + MCPA, TX + MCPA-thioethyl, TX + MCPB, TX + mecoprop, TX + mecoprop-P, TX + mefenacet, TX + mefluidide, TX + mesosulfuron, TX + mesosulfuron-methyl, TX + mesotrione, TX + metam, TX + metamifop, TX + metamitron, TX + metazachlor, TX + methabenzthiazuron, TX + methylarsonic acid, TX + methyldymron, TX + methyl isothiocyanate, TX + metobenzuron, TX + metolachlor, TX + S-metolachlor, TX + metosulam, TX + metoxuron, TX + metribuzin, TX + metsulfuron, TX + metsulfuron-methyl, TX + MK-616, TX + molinate, TX + monolinuron, TX + MSMA, TX + naproanilide, TX + napropamide, TX + naptalam, TX + neburon, TX + nicosulfuron, TX + nonanoic acid, TX + norflurazon, TX + oleic acid (fatty acids), TX + orbencarb, TX + orthosulfamuron, TX + oryzalin, TX + oxadiargyl, TX + oxadiazon, TX + oxasulfuron, TX + oxaziclomefone, TX + oxyfluorfen, TX + paraquat, TX + paraquat dichloride, TX + pebulate, TX + pendimethalin, TX + penoxsulam, TX + pentachlorophenol, TX + pentanochlor, TX + pentoxazone, TX + pethoxamid, TX + petrolium oils, TX + phenmedipham, TX + phenmedipham-ethyl, TX + picloram, TX + picolinafen, TX + pinoxaden, TX + piperophos, TX + potassium arsenite, TX + potassium azide, TX + pretilachlor, TX + primisulfuron, TX + primisulfuron-methyl, TX + prodiamine, TX + profluazol, TX + profoxydim, TX + prometon, TX + prometryn, TX + propachlor, TX + propanil, TX + propaquizafop, TX + propazine, TX + propham, TX + propisochlor, TX + propoxycarbazone, TX + propoxycarbazone-sodium, TX + propyrisulfuron, TX + propyzamide, TX + prosulfocarb, TX + prosulfuron, TX + pyraclonil, TX + pyraflufen, TX + pyraflufen-ethyl, TX + pyrasulfutole, TX + pyrazolynate, TX + pyrazosulfuron, TX + pyrazosulfuron-ethyl, TX + pyrazoxyfen, TX + pyribenzoxim, TX + pyributicarb, TX + pyridafol, TX + pyridate, TX + pyriftalid, TX + pyriminobac, TX + pyriminobac-methyl, TX + pyrimisulfan, TX + pyrithiobac, TX + pyrithiobac-sodium, TX + pyroxsulam, TX + pyroxasulfone, TX + quinclorac, TX + quinmerac, TX + quinoclamine, TX + quizalofop, TX + quizalofop-P, TX + rimsulfuron, TX +saflufenacil, TX + sethoxydim, TX + siduron, TX + simazine, TX + simetryn, TX + SMA, TX + sodium arsenite, TX + sodium azide, TX + sodium chlorate, TX + sulcotrione, TX + sulfentrazone, TX + sulfometuron, TX + sulfometuron-methyl, TX + sulfosate, TX + sulfosulfuron, TX + sulfuric acid, TX + tar oils, TX + 2,3,6-TBA, TX + TCA, TX + TCA-sodium, TX + tebuthiuron, TX + tefuryltrione, TX + tembotrione, TX + tepraloxydim, TX + terbacil, TX + terbumeton, TX + terbuthylazine, TX + terbutryn, TX + thenylchlor, TX + thiazopyr, TX + thiencarbazone, TX + thifensulfuron, TX + thifensulfuron-methyl, TX + thiobencarb, TX + tiocarbazil, TX + topramezone, TX + tralkoxydim, TX + tri-allate,

TX + triasulfuron, TX + triaziflam, TX + tribenuron, TX + tribenuron-methyl, TX + tricamba, TX + triclopyr, TX + trietazine, TX + trifloxysulfuron, TX + trifloxysulfuron-sodium, TX + trifluralin, TX + triflusulfuron, TX + triflusulfuron-methyl, TX + trihydroxytriazine, TX + tritosulfuron, TX + [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetic acid ethyl ester (CAS RN 353292-31-6), TX + 4-[(4,5-dihydro-3-methoxy-4-methyl-5-oxo)-1H-1,2,4-triazol-1-ylcarbonylsulfamoyl]-5-methylthiophene-3-carboxylic acid (BAY636), TX + BAY747 (CAS RN 335104-84-2), TX + topramezone (CAS RN 210631-68-8), TX + 4-hydroxy-3-[[2-[(2-methoxyethoxy)methyl]-6-(trifluoro-methyl)-3-pyridinyl]carbonyl]-bicyclo[3.2.1]oct-3-en-2-one (CAS RN 352010-68-5), and TX + 4-hydroxy-3-[[2-(3-methoxypropyl)-6-(difluoromethyl)-3-pyridinyl]carbonyl]-bicyclo[3.2.1]oct-3-en-2-one.

The compounds of formula (I) according to the invention can also be used in combination with safeners. Preferably, in these mixtures, the compound of the formula (I) is one of those compounds listed in Table A (compound A1 to compound A38) above. The following mixtures with safeners, especially, come into consideration:

compound of formula (I) + cloquintocet-mexyl, compound of formula (I) + cloquintocet acid and salts thereof, compound of formula (I) + fenchlorazole-ethyl, compound of formula (I) + fenchlorazole acid and salts thereof, compound of formula (I) + mefenpyr-diethyl, compound of formula (I) + mefenpyr diacid, compound of formula (I) + isoxadifen-ethyl, compound of formula (I) + isoxadifen acid, compound of formula (I) + furilazole, compound of formula (I) + furilazole R isomer, compound of formula (I) + benoxacor, compound of formula (I) + dichlormid, compound of formula (I) + AD-67, compound of formula (I) + oxabetrinil, compound of formula (I) + cyometrinil, compound of formula (I) + cyometrinil Z-isomer, compound of formula (I) + fenclorim, compound of formula (I) + cyprosulfamide, compound of formula (I) + naphthalic anhydride, compound of formula (I) + flurazole, compound of formula (I) + N-(2-methoxybenzoyl)-4-[(methylaminocarbonyl)amino]benzenesulfonamide, compound of formula (I) + CL 304,415, compound of formula (I) + dicyclonon, compound of formula (I) + fluxofenim, compound of formula (I) + DKA-24, compound of formula (I) + R-29148 and compound of formula (I) + PPG-1292. A safening effect can also be observed for the mixtures compound of the formula (I) + dymron, compound of the formula (I) + MCPA, compound of the formula (I) + mecoprop and compound of the formula (I) + mecoprop-P.

The mixing partners of the TX may also be in the form of esters or salts, as mentioned e.g. in The Pesticide Manual, 12th Edition (BCPC), 2000.
In the above different lists of active ingredients to be mixed with a TX, the compound of the formula I is preferably a compound of Table A (compound A1 to compound A38); and more preferably, a compound selected from A2, A3, A6 to A12, A14 to A17, A19, A21, A23 to A27, A30, A37; or more preferably, a compound selected from A1, A2 & A3, A7 to A21, A23 to A28, A30, A36, A37; or more preferably, a compound selected from A1, A2 , A3, A7 to A18, A21, A23 to A27, A29, A30, A36, A37; or more preferably, a compound selected from
A1, A2, A3, A6 to A21, A23 to A27, A30, A32, A33, A34, A36, A37; or more preferably, a compound selected from A2, A6 to A14, A16, A18, A20, A23 to A27, A32, A33, A30, A34, A36, A37; or more preferably, a compound selected from A3; or more preferably, a compound selected from: A3, A6 to A8, A10 to A12, A14, A16, A20, A23, A24, A28, A27, A32, A36.

In the above-mentioned mixtures of compounds of formula I, in particular a compound selected from said Table A (compound A1 to compound A38), with other insecticides, fungicides, herbicides, safeners, adjuvants and the like, the mixing ratios can vary over a large range and are, preferably

100:1 to 1:6000, especially 50:1 to 1:50, more especially 20:1 to 1:20, even more especially 10:1 to 1:10. Those mixing ratios are understood to include, on the one hand, ratios by weight and also, on other hand, molar ratios.

The mixtures can advantageously be used in the above-mentioned formulations (in which case "active ingredient" relates to the respective mixture of TX with the mixing partner).

Some mixtures may comprise active ingredients which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The mixtures comprising a TX selected from Table A (compound A1 to compound A38) and one or more active ingredients as described above can be applied, for example, in a single "ready-mix" form, in a combined spray mixture composed from separate formulations of the single active ingredient components, such as a "tank-mix", and in a combined

use of the single active ingredients when applied in a sequential manner, i.e. one after the other with a reasonably short period, such as a few hours or days. The order of applying the compounds of formula I selected from Table A (compound A1 to compound A38) and the active ingredients as described above is not essential for working the present invention.

Some mixtures may comprise active ingredients which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The following methods were used for HPLC-MS analysis:

**Method A:** ZQ Mass Spectrometer from Waters (Single quadrupole mass spectrometer) Instrument Parameter:

Ionization method: Electrospray
Polarity: positive ions
Capillary (kV) 3.00, Cone (V) 30.00, Extractor (V) 2.00, Source Temperature (°C) 100, Desolvation Temperature (°C) 250, Cone Gas Flow (L/Hr) 50, Desolvation Gas Flow (L/Hr) 400
Mass range: 150 to 1000 Da
HP 1100 HPLC from Agilent: solvent degasser, quaternary pump (ZCQ) / binary pump (ZDQ), heated column compartment and diode-array detector.
Column: Phenomenex Gemini C18, 3 μm particle size, 110 Angstroms, 30 x 3 mm,
Temp: 60 °C
DAD Wavelength range (nm): 200 to 500
Solvent Gradient:

$$A = \text{water} + 0.05\ \%\ HCOOH$$

$$B = \text{Acetonitrile/Methanol}\ (4{:}1,\ v{:}v) + 0.04\ \%\ HCOOH$$

| Time | A% | B% | Flow (ml/min) |
|---|---|---|---|
| 0.00 | 95.0 | 5.0 | 1.700 |
| 2.00 | 0.0 | 100.0 | 1.700 |
| 2.80 | 0.0 | 100.0 | 1.700 |
| 2.90 | 95.0 | 5.0 | 1.700 |
| 3.00 | 95.0 | 5.0 | 1.700 |

**Method B**: ZMD Mass Spectrometer from Waters (Single quadrupole mass spectrometer) Instrument Parameter:

Ionization method: Electrospray
Polarity: positive ions
Capillary (kV) 3.80, Cone (V) 30.00, Extractor (V) 3.00, Source Temperature (°C) 150, Desolvation Temperature (°C) 350, Cone Gas Flow (L/Hr) OFF, Desolvation Gas Flow (L/Hr) 600
Mass range: 150 to 1000 Da (100 to 1500 for Low Mass)
HP 1100 HPLC from Agilent: solvent degasser, binary pump, heated column compartment and diode-array detector.
Column: Phenomenex Gemini C18, 3 μm (micro meter) particle size, 110 Angstroms, 30 x 3 mm,
Temp: 60 °C
DAD Wavelength range (nm): 200 to 500
Solvent Gradient:

$$A = \text{water} + 0.05\ \%\ HCOOH$$

B= Acetonitrile/Methanol (4:1, v:v) + 0.04 % HCOOH

| Time | A% | B% | Flow(ml/min) |
|---|---|---|---|
| 0.00 | 95.0 | 5.0 | 1.700 |
| 2.00 | 0.0 | 100.0 | 1.700 |
| 2.80 | 0.0 | 100.0 | 1.700 |
| 2.90 | 95.0 | 5.0 | 1.700 |
| 3.00 | 95.0 | 5.0 | 1.700 |

**Method C:**

| | |
|---|---|
| MS | ZQ Mass Spectrometer from Waters (single quadrupole mass spectrometer), ionization method: electrospray, polarity: positive ionization, capillary (kV) 3.00, cone (V) 30.00, extractor (V) 3.00, source temperature (°C) 100, desolvation temperature (°C) 200, cone gas flow (L/Hr) 200, desolvation gas flow (L/Hr) 250, mass range: 150 to 800 Da. |
| LC | 1100er Series HPLC from Agilent: quaternary pump, heated column compartment and diode-array detector. Column: Waters Atlantis dc18; length: 20 mm; internal diameter: 3 mm; particle size: 3 μm, temperature (°C) 40, DAD wavelength range (nm): 200 to 500, solvent gradient: A = 0.1% of formic acid in water and B: 0.1% of formic acid in acetonitrile. |

| Time (min) | A% | B% | Flow (ml/min) |
|---|---|---|---|
| 0.0 | 90 | 10 | 1.7 |
| 5.5 | 0.0 | 100 | 1.7 |
| 5.8 | 0.0 | 100 | 1.7 |
| 5.9 | 90 | 10 | 1.7 |

Preparation of Examples

Example I: Preparation of 3-[(4-fluorobenzoyl)amino]-*N*-{2,6-dimethyl-4-[1-(4-trifluoromethylphenyl)-2,2,2-trifluoro-1-fluoroethyl]phenyl}benzamide

Step 1: Preparation of 3-[(4-fluorobenzoyl)amino]-*N*-{2,6-dimethyl-4-[1-(4-trifluoromethylphenyl)-2,2,2-trifluoro-1-chloroethyl]phenyl}benzamide

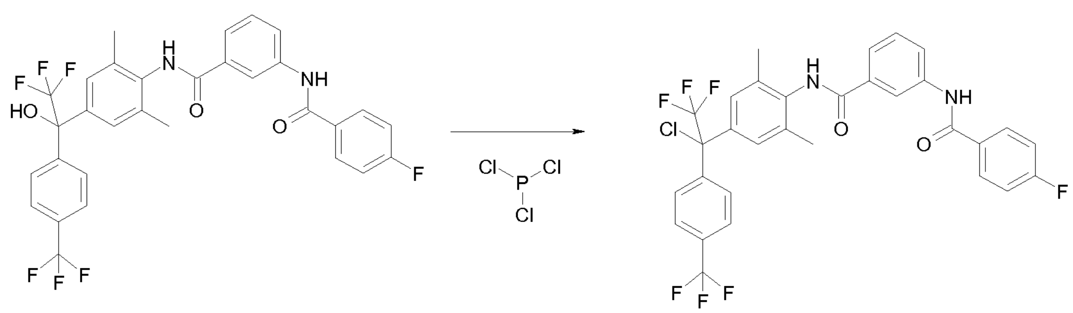

To a solution of 3-[(4-fluorobenzoyl)amino]-*N*-{2,6-dimethyl-4-[1-(4-trifluoromethylphenyl)-2,2,2-trifluoro-1-hydroxyethyl]phenyl}benzamide (prepared as described in WO 2009/049845) (0.065 mg, 0.11 mmol) in chlorobenzene (5

ml) was added phosphorous trichloride (0.005 ml, 0.05 mmol). The reaction mixture was stirred at reflux for 2 hours. The reaction mixture was concentrated under vacuo and the residue was purified by column chromatography on silica gel (eluent: ethyl acetate) to give the title compound.
$^1$H NMR (400 MHz, $CDCl_3$): 8.31 (s, 1H), 7.94 (m, 4H), 7.73 (d, 1H), 7.66 (m, 4H), 7.52 (m, 2H), 7.22 (m, 4H), 2.32 (s, 6H) ppm. LC-MS (Method A) RT 2.16 (623, $MH^+$)

Analogous procedures were used to prepare the following compounds: 3-[(2,3-difluorobenzoylamino]-*N*-{2,6-dimethyl-4-[1-(4-trifluoromethylphenyl)-2,2,2-trifluoro-1-chloroethyl]phenyl}benzamide. $^1$H NMR (400 MHz, $CDCl_3$): 8.49 (d, 1H), 8.32 (s, 1H), 7.93 (t, 1H), 7.87 (d, 1H), 7.76 (d, 1H), 7.67 (m, 4H), 7.55(m, 2H), 7.41 (q, 1H), 7.30 (m, 1H), 7.21 (s, 2H), 2.32 (s, 6H) ppm. LC-MS (Method A) RT 2.16 (641, $MH^+$)

Step 2: Preparation of 3-[(4-fluorobenzoyl)amino]-*N*-{2,6-dimethyl-4-[1-(4-trifluoromethyl-phenyl)-2,2,2-trifluoro-1-fluoroethyl]phenyl}benzamide (compound A1)

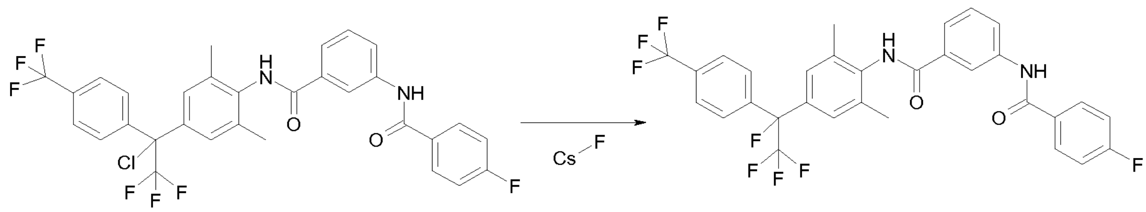


To a solution of 3-[(4-fluorobenzoyl)amino]-N-{2,6-dimethyl-4-[1-(4-trifluoromethyl-phenyl)-2,2,2-trifluoro-1-chloroethyl]phenyl}benzamide (prepared as described in step 1) (0.019 mg, 0.03 mmol) in acetonitrile (3 ml) was added cesium fluoride (0.025 g, 0.16 mmol). The reaction mixture was stirred at 100°C in a sealed vial for 5 hours. The reaction mixture was concentrated under vacuo and the residue was purified by column chromatography on silica gel (eluent: ethyl acetate / Cyclohexane 1 : 4) to give the title compound. $^1$H NMR (400 MHz, $CDCl_3$): 8.03 (s, 1H), 7.80 (s, 1H), 7.65 (m, 3H), 7.40 (m, 6H), 7.29 (t, 1H), 6.95 (m, 4H), 5.35 (s, 2H) ppm. LC-MS (Method B) RT 2.15 (607, $MH^+$, 629, $M+Na^+$)

Analogous procedures were used to prepare the following compounds: 3-[(2,3-difluorobenzoylamino]-*N*- {2,6-dimethyl-4-[1-(4-trifluoromethylphenyl)-2,2,2-trifluoro-1-fluoroethyl]phenyl}benzamide (compound A2). $^1$H NMR (400 MHz, $CDCl_3$): 8.50 (d, 1H), 8.30 (s, 1H), 7.85 (m, 2H), 7.66 (m, 6H), 7.50 (t, 1H), 7.39 (q, 1H), 7.21 (s, 3H), 2.3 (s, 6H) ppm. LC-MS (Method B) RT 2.16 (625.4, $MH^+$, 647, $M+Na^+$)

Example II: Preparation of 3-[(benzoyl)amino]-*N*-{2,6-dichloro-4-[1-(4-chlorophenyl)-2,2,2-trifluoro-1-fluoroethyl]-2-methoxyphenyl}benzamide

Step 1: Preparation of 1-(4-amino-3,5-dichlorophenyl)-2,2,2-trifluoroethanone

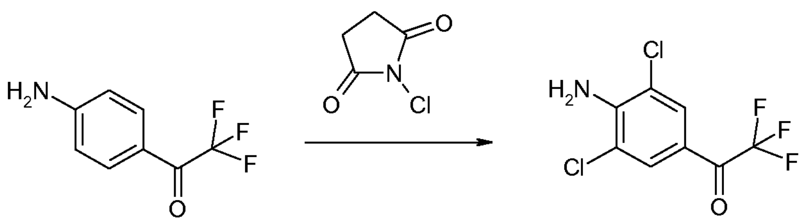

1-(4-Aminophenyl)-2,2,2-trifluoroethanone (7.0g, 37.0 mmol) was dissolved in acetonitrile (100 ml) and *N*-chlorosuccinimide ("NCS") (11.36 g, 85.12 mmol) was added. The reaction mixture was heated to reflux for 90 minutes. The reaction mixture was concentrated under vacuum, the residue suspended in diethyl ether and the solids removed via filtration. The filtrate was concentrated and the residue was purified by column chromatography on silica gel (eluent: cyclohexane / ethylacetate 3:1) to give 1-(4-amino-3,5-dichlorophenyl)-2,2,2-trifluoroethanone (8.30 g, 87% yield). $^1$H NMR (400 MHz, $CDCl_3$): 7.95 (s, 2H), 5.23 (bs, 2H) ppm.

Step 2: Preparation of 1-(4-amino-3,5-dichlorophenyl-1-(4-chlorophenyl)-2,2,2-trifluoro-ethanol

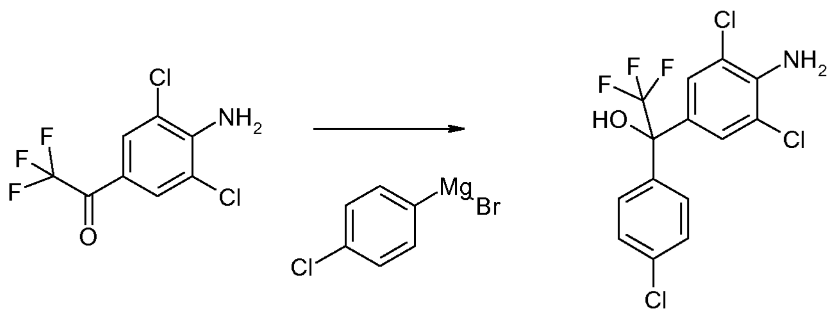

To a solution of 1-(4-amino-3,5-dichlorophenyl)-2,2,2-trifluoroethanone (Step1) (3.0 g, 11.35mmol) in tetrahydrofuran (35 ml) under a nitrogen atmosphere at 0°C was added a solution of 4-chlorophenylmagnesium bromide (1 M) (35 ml, 34.95 mmol). The reaction mixture was stirred at 0°C for 4 hours. The reaction mixture was neutralized by addition of saturated solution of ammonium chloride and filtered. The filtrate was poured into a biphasic mixture of water and ethyl acetate. The aqueous phase was extracted twice with ethyl acetate. The combined organic extracts were dried over sodium sulfate and concentrated. and the residue was purified by column chromatography on silica gel (eluent: cyclohexane / ethylacetate 4:1) to give 1-(4-amino-3,5-dichlorophenyl)-1-(4-chlorophenyl)-2,2,2-trifluoro-ethanol (4.00 g, 93% yield). LC-MS (Method A) RT 1.98 (411-415, $M+CH_3CN+H$),
$^1H$ NMR (400 MHz, $CDCl_3$): 7.42 (m, 2H), 7.35 (m, 2H), 7.27 (m, 2H), 4.52 (bs, 2H) ppm.

Step 3: Preparation of 3-nitro-*N*-{2,6-dichloro-4-[1-(4-chlorophenyl)-2,2,2-trifluoro-1-hydroxyethyl]-2-fluorophenyl}benzamide

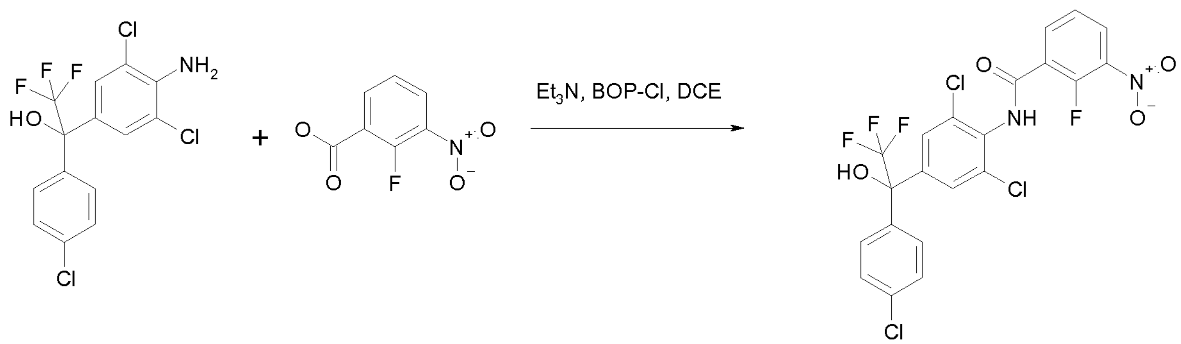


To a suspension of 1-(4-amino-3,5-dichlorophenyl)-1-(4-chlorophenyl)-2,2,2-trifluoroethanol (Step 2) (2.0 g, 5.40 mmol) in 1,2-dichloroethane (20 ml) was added triethylamine (1.63 g, 16.3 mmol), followed by 2-fluoro-3-nitrobenzoic acid (1.499 g, 8.10 mmol) and bis(2-oxo-3-oxazolidinyl)phosphonic chloride ("BOP-Cl") (2.749 g, 10.80 mmol). The reaction mixture was stirred overnight at reflux. The reaction was quenched by addition of aqueous sodium hydrogen carbonate and ethyl acetate. The phases were separated. The aqueous phase was extracted twice with ethyl acetate the organic phases were combinated. The organic phase was washed with aqueous sodium hydrogen carbonate (saturated), water and brine. The organic phase was dried over sodium sulfate and concentrated. The residue was used without extra purification.

Step 4: Preparation of 3-nitro-*N*-{2,6-dichloro-4-[1-(4-chlorophenyl)-2,2,2-trifluoro-1-hydroxyethyl]-2-methoxyphenyl}benzamide

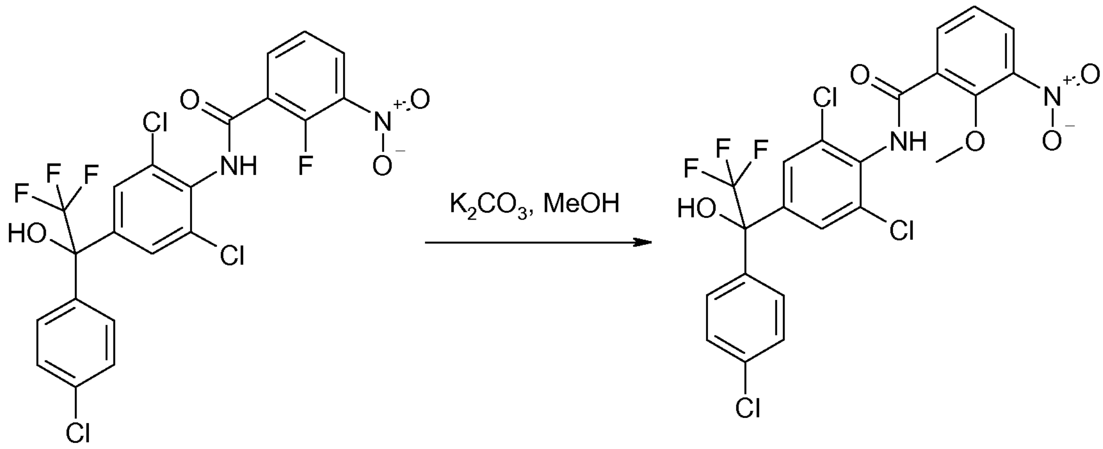


To a suspension of 3-nitro-*N*-{2,6-dichloro-4-[1-(4-chlorophenyl)-2,2,2-trifluoro-1-hydroxylethyl]-2-fluorophenyl}benzamide (Step 3) (3.307 g, 6.15 mmol) in methanol (100 ml) was added potassium carbonate (1.70 g, 12.30 mmol). The reaction mixture was stirred overnight at room temperature. The mixture was concentrated under vacuo and redissolved in a mixture of ethyl acetate and water. The organic and aqueous phases were separated. The organic phase was washed with water, dried over sodium sulfate and concentrated. The residue was purified by column chromatography on silica gel (Büchi sampler, SiOH 150*40, Gradient 99% to 50% cyclohexane in ethyl acetate over 40 min.) to give 3-nitro-N-{2,6-dichloro-4-[1-(4-chlorophenyl)-2,2,2-trifluoro-1-hydroxyethyl]-2-methoxyphenyl}-benzamide (2.10 g, 62% yield, 90% purity). The compound was used without extra purification. LC-MS (Method B, Positive) RT 2.04 (550-553, $MH^+$), $^1H$ NMR (400 MHz, $CDCl_3$): 9.12 (s, 1H), 8.43 (d, 1H), 8.08 (d, 1H), 7.57 (s, 2H), 7.45 (m, 5H), 4.18 (s, 3H), 3.05 (s,1H) ppm.

Step 5 Preparation of 3-amino-*N*-{2,6-dichloro-4-[1-(4-chlorophenyl)-2,2,2-trifluoro-1-hydroxyethyl]-2-methoxyphenyl}benzamide

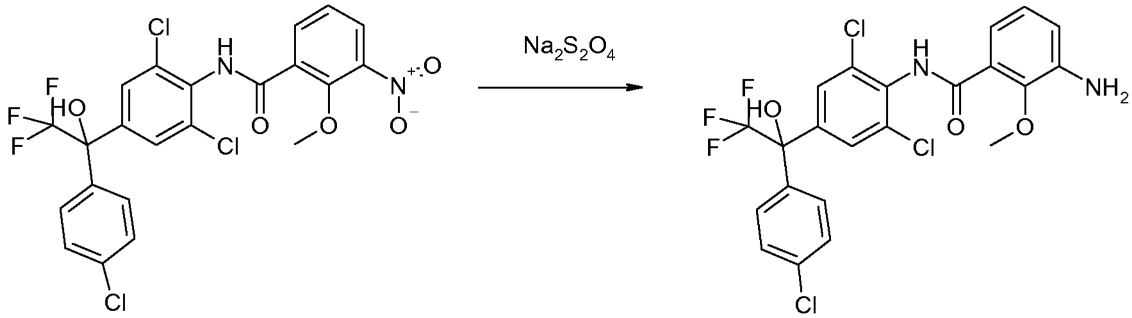


To a solution of 3-nitro-*N*-{2,6-dichloro-4-[1-(4-chlorophenyl)-2,2,2-trifluoro-1-hydroxyethyl]-2-methoxyphenyl}benzamide (Step 4) (2.089g, 3.8 mmol) in tetrahydrofuran (21 ml) was added aqueous sodium hydroxide (0.1 M) (7 ml), sodium hydrosulfite (3.227 g, 15.20 mmol) and tetrabutylammonium bromide ("TBAB") (0.123 g, 0.38 mmol). The reaction mixture was stirred at ambient temperature for 3 hours. Then, sodium hydrosulfite (10g) was added and the mixture was heated at reflux for 4 hours. The aqueous and organic phases were separated. The aqueous phase was extracted twice with ethyl acetate. The combined organic extracts were dried over sodium sulfate and concentrated. The residue was purified by column chromatography on silica gel (Büchi sampler, SiOH 150*40, Gradient 1% to 50% ethyl acetate in cyclohexane over 60 min.) to give 3-amino-N-{2,6-dichloro-4-[1-(4-chlorophenyl)-2,2,2-trifluoro-1-hydroxyethyl]-2-methoxyphenyl}benzamide (0.720 g, 36.5% yield). LC-MS (Method B, Negative) RT 1.94 (517-521, $M-H^+$), $^1H$ NMR (400 MHz, $CDCl_3$): 7.58 (m, 3H), 7.46 (d, 1H), 7.41 (d, 1H), 7.12 (t, 1H), 6.98 (dd, 1H), 3.99 (bs, 5H), 3.08 (s, 1H) ppm.

Step 7: Preparation of 3-[(benzoyl)amino]-2-methoxy-*N*-{2,6-dichloro-4-[1-(4-chlorophenyl)-2,2,2-trifluoro-1-hydroxyethyl]-2-methoxyphenyl}benzamide

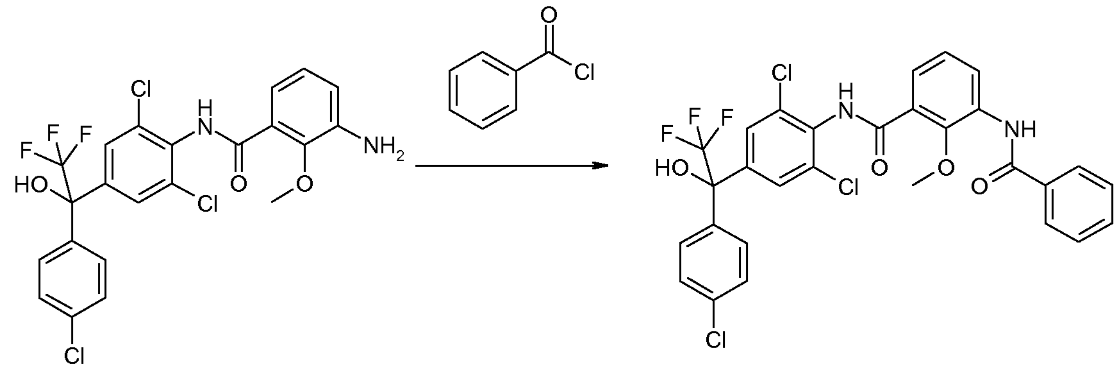

To a solution of 3-amino-N-{2,6-dichloro-4-[1-(4-chlorophenyl)-2,2,2-trifluoro-1-hydroxyethyl]-2-methoxyphenyl}benzamide (0.728 g, 1.40 mmol) (Step 6) in tetrahydrofuran (60 ml) were added successively pyridine (0.225 ml, 2.80 mmol) and benzoyl chloride (0.179 mL, 1.54 mmol). The reaction mixture was stirred for 7 hours at ambient temperature. A mixture of ethyl acetate and aqueous sodium hydrogen carbonate (saturated) were added and the phases were separated. The aqueous phase was extracted twice with ethyl acetate. The combined organic extracts were dried over sodium sulfate and concentrated. The residue was purified by column chromatography on silica gel (Büchi sampler, SiOH 150*40, Gradient 1% to 50% ethyl acetate in cyclohexane over 60 min.) to give 3-[benzoylamino]-2-methoxy -N-{2,6-dichloro-4-[1-(4-chlorophenyl)-2,2,2-trifluoro-1-hydroxyethyl]-2-methoxyphenyl}benzamide (0.70 g, 80% yield). LC-MS (Method B, Positive) RT 2.05 (623, $MH^+$), $^1H$ NMR (400 MHz, $CDCl_3$): 9.04 (s, 1H), 8.68 (d, 1H), 8.51 (s, 1H), 7.97 (d, 2H), 7.91 (d, 1H), 7.65-7.38 (m, 10H), 4.04 (s, 3H), 3.52 (s, 1H) ppm.

Step 8: Preparation of 3-[benzoylamino]-2-methoxy-*N*-{2,6-dichloro-4-[1-(4-chlorophenyl)-2,2,2-trifluoro-1-fluoroethyl]-2-methoxyphenyl}benzamide (compound A3)

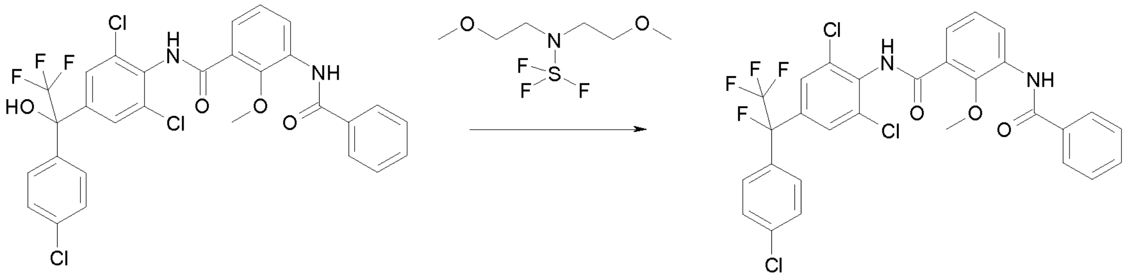

To a precooled solution (0°C) of 3-[benzoylamino]-2-methoxy-*N*-{2,6-dichloro-4-[1-(4-chlorophenyl)-2,2,2-trifluoro-1-hydroxyethyl]-2-methoxyphenyl}benzamide (0.10 g, 0.16 mmol) (Step 7) in anhydrous dichloromethane (7 ml) was added Deoxy-Fluor (0.078g, 0.075 μL, 0.18 mmol, solution 50% in THF). The reaction mixture was stirred for 1 hour at 0°C. The mixture reaction was quenched with water. The organic and aqeous phases were separated. The aqueous phase was extracted twice with dichloromethane. The combined organic extracts were dried over sodium sulfate and concentrated. The residue was purified by column chromatography on silica gel (Büchi sampler, SiOH 150*12, Gradient 1% to 50% ethyl acetate in cyclohexane over 90 min.) to give 3-[benzoylamino]-2-methoxy-N-{2,6-dichloro-4-[1-(4-chlorophenyl)-2,2,2-trifluoro-1-fluoroethyl]-2-methoxyphenyl}benzamide (0.058 g, 58% yield). Mp = 163-166°C, LC-MS (Method B, Positive) RT 2.20 (625, $MH^+$), $^1H$ NMR (400 MHz, $CDCl_3$): 9.10 (s, 1H), 8.72 (d, 1H), 8.48 (s, 1H), 7.95 (m, 3H), 7.67-7.55 (m, 5H), 7.48 (s, 2H), 7.40 (t, 1H), 4.07 (s, 3H) ppm.

Example III: Preparation of N-{3-[2,6-dichloro-4-(2,2,2-trifluoro-1-fluoro-1-thiophen-2-yl-ethyl)phenylcarbamoyl]-2-fluorophenyl}-2,4,6-trifluorobenzamide

Step 1: Preparation of N-[2,6-dichloro-4-(2,2,2-trifluoroacetyl)phenyl-2-fluoro-3-nitro-benzamide

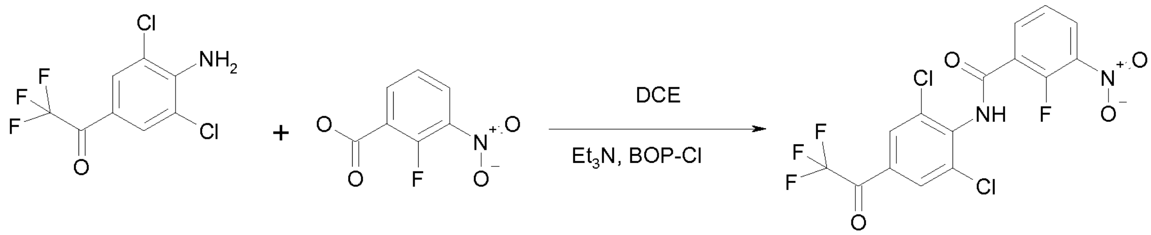


To a suspension of 1-(4-amino-3,5-dichlorophenyl)-2,2,2-trifluoroethanone (Example II, Step 1) (11.99 g, 46.5 mmol) in 1,2-dichloroethane (20 ml) was added triethylamine (19.44 mL, 139.5 mmol), followed by 2-fluoro-3-nitrobenzoic acid (10.33 g, 55.80 mmol) and bis(2-oxo-3-oxazolidinyl)phosphonic chloride ("BOP-Cl") (23.67 g, 93.0 mmol). The reaction mixture was stirred overnight at 110°C. The reaction was quenched by addition of aqueous sodium hydrogen carbonate and ethyl acetate. The phases were separated. The aqueous phase was extracted twice with ethyl acetate and the organic phases were combined. The organic phase was washed with aqueous sodium hydrogen carbonate (saturated), water and brine. The organic phase was dried over sodium sulfate and concentrated in presence of 45 mL of SiOH 60. The residue was purified, in 3 batches by column chromatography on silica gel (Büchi sampler, SiOH 150*12, Gradient 1% to 50% ethyl acetate in cyclohexane over 65 min.) to give N-[2,6-dichloro-4-(2,2,2-trifluoroacetyl)phenyl]-2-fluoro-3-nitrobenzamide (10.1g (around 90% purity), 51%). The compound was used without extra purification.

Step 2 :Preparation of N-[2,6-dichloro-4-(2,2,2-trifluoroacetyl)phenyl]-2-fluorobenzamide

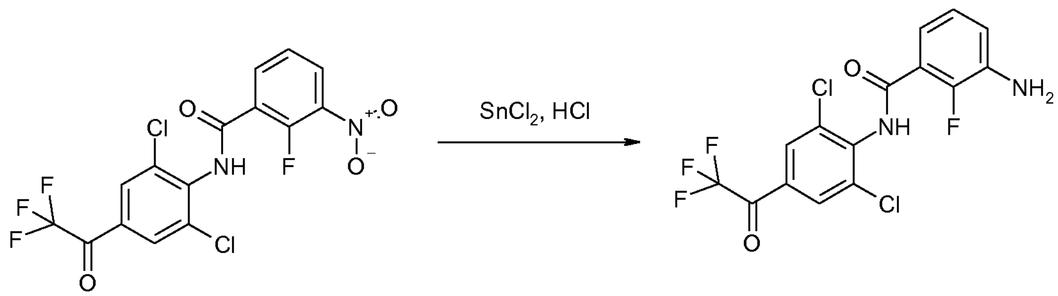


To a solution of N-[2,6-dichloro-4-(2,2,2-trifluoroacetyl)phenyl]-2-fluoro-3-nitrobenzamide (3.31 g, 7.80 mmol) (Step 1) in isopropanol (25 ml) was added tin chloride (7.0 g, 31.20 mmol). The mixture was cooled to 0°C and 2.56 ml of concentrated hydrochloric acid (37%) was added slowly. The mixture was stirred at 80°C for 2 hours. Then about a third of the total volume of isopropanol was evaporated. Water (100 ml) was added to the concentrated mixture followed by aqueous sodium hydroxide (4N) to adjust the pH to 8 to 9. The aqueous phase was extracted three times with ethyl acetate (200 ml). The combined organic extracts were dried over sodium sulfate and the solvent evaporated. The residue was purified by column chromatography on silica gel (eluent: ethyl acetate / cyclohexane 1 : 3) to give N-[2,6-dichloro-4-(2,2,2-trifluoroacetyl)phenyl]-2-fluorobenzamide (1.0 g, 32% yield). LC-MS (Method C, Negative) RT 1.45 (393-395, $M-H^+$). The compound was used without extra purification.

Step 3: Preparation of N-{3-[2,6-dichloro-4-(2,2,2-trifluoroacetyl)phenylcarbamoyl]-2-fluorophenyl}-2,4,6-trifluorobenzamide

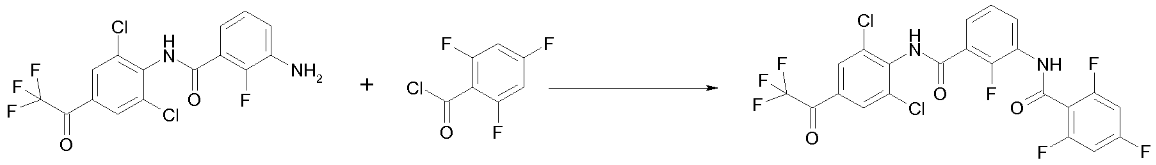

N-[2,6-dichloro-4-(2,2,2-trifluoroacetyl)phenyl]-2-fluorobenzamide (1.0 g, 2.55 mmol) (Step 2) was dissolved in a biphasic mixture of ethyl acetate (25 ml) and saturated solution of sodium hydrogen carbonate (25 ml). 2,4,6-Trifluorobenzoyl chloride (0.695 mg, 3.57 mmol) was added under vigorous stirring. The reaction mixture was stirred for 1 hour at ambient temperature. The phases were separated. The organic phase was dried over sodium sulfate and concentrated in presence of 15 mL of SiOH 60. The residue was purified, in 3 batches by column chromatography on silica gel (Büchi sampler, SiOH 150*40, Gradient 1% to 50% ethyl acetate in cyclohexane over 65 min.) to give N-{3-[2,6-dichloro-4-(2,2,2-trifluoroacetyl)phenylcarbamoyl]-2-fluorophenyl}-2,4,6-trifluorobenzamide (1.30g, 92.2%). LC-MS (Method A, Negative) RT 1.73(551,552, M-H$^+$), $^1$H NMR (400 MHz, $CDCl_3$): 8.68 (s, 1H), 8.24 (d, 1H), 8.12 (s, 2H), 8.0 (sb, 1H), 7.72 (t, 1H), 7.40 (t, 1H), 6.86 (t, 2H) ppm.

Step 4: Preparation of N-{3-[2,6-dichloro-4-(2,2,2-trifluoro-1-hydroxy-1-thiophen-2-yl-ethyl)phenylcarbamoyl]-2-fluorophenyl}-2,4,6-trifluorobenzamide

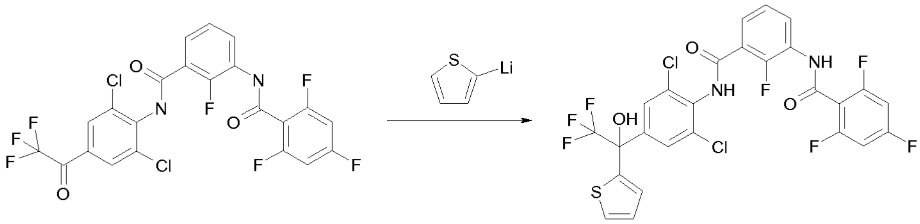

To a solution, at -20°C of N-{3-[2,6-dichloro-4-(2,2,2-trifluoroacetyl)-phenylcarbamoyl]-2-fluorophenyl}-2,4,6-trifluorobenzamide (0.354 g, 0.64 mmol) (Step 3) in tetrahydrofuran (10 ml) was added 2-thienyl lithium (1 M, 5.12 mL, 5.12 mmol). The mixture was stirred at -20 °C for 2 hours. The solution was quenched with a saturated solution of ammonium chloride. The aqueous phase was extracted three times with ethyl acetate (50 ml). The combined organic extracts were dried over sodium sulfate and filtered. The solution was concentrated in presence of 5 mL of SiOH 60. The residue was purified by column chromatography on silica gel (Büchi sampler, SiOH 150*12, Gradient 1% to 50% ethyl acetate in cyclohexane over 120 min.) to give N-{3-[2,6-dichloro-4-(2,2,2-trifluoro-1-hydroxy-1-thiophen-2-ylethyl)phenylcarbamoyl]-2-fluorophenyl} -2,4,6-trifluoro-enzamide (0.084g, 21%). LC-MS (Method A, Negative) RT 1.93 (635, 637, M-H$^+$),$^1$H NMR (400 MHz, $CDCl_3$): 8.68 (t, 1H), 8.03(d, 1H), 7.95(m, 2H), 7.70 (s, 2H), 7.47 (d, 1H), 7.40 (t, 1H), 7.28 (m, 1H), 7.09(t, 1H), 6.85 (t, 1H), 3.18 (s, 1H) ppm.

Analogous procedures were used to prepare the following compounds: N-{3-[2,6-Dichloro-4-(2,2,2-trifluoro-1-hydroxy-1-thiophen-3-yl-ethyl)-phenylcarbamoyl]-2-fluorophenyl}-2,4,6-trifluorobenzamide using 3-thienyl magnesium iodide. LC-MS (Method A, Negative) RT 1.91 (635, 636.7, M-H$^+$), $^1$H NMR (400 MHz, $CDCl_3$): 8.68 (t, 1H), 8.05-7.88(m, 3H), 7.61 (s, 2H), 7.54 (d, 1H), 7.40 (m, 2H), 7.08(d, 1H), 6.85 (t, 2H), 3.02 (s, 1H) ppm.

Step 5: Preparation of N-{3-[2,6-dichloro-4-(2,2,2-trifluoro-1-fluoro-1-thiophen-2-ylethyl-phenylcarbamoyl]-2-fluorophenyl}-2,4,6-trifluorobenzamide(compound A4)

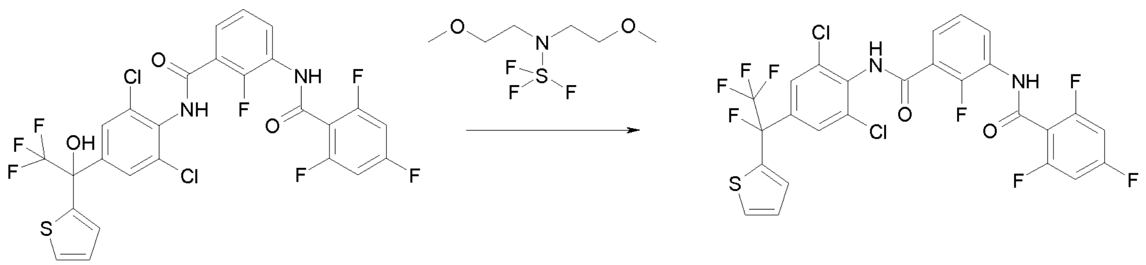

To a precooled solution (0°C) of N-{3-[2,6-dichloro-4-(2,2,2-trifluoro-1-hydroxy-1-thiophen-2-ylethyl)phenylcarbamoyl]-2-fluorophenyl}-2,4,6-trifluorobenzamide (0.07 g, 0.11 mmol) (Step 4) in anhydrous dichloromethane (7 ml) was added Deoxy-Fluor (0.058g, 56 μL, 0.13 mmol, solution 50% in THF). The reaction mixture was stirred for 1 hour at 0°C. The mixture reaction was quenched with water. The organic and aqeous phases were separated. The aqueous phase was extracted twice with dichloromethane. The combined organic extracts were dried over sodium sulphate, filtered and concentrated, after addition of 5 mL of SiOH 60. The residue was purified by column chromatography on silica gel (Büchi sampler, SiOH 150*12, Gradient 1% to 50% ethyl acetate in cyclohexane over 120 min.) to give N- {3-[2,6-dichloro-4-(2,2,2-trifluoro-1-fluoro-1-thiophen-2-ylethyl)phenylcarbamoyl]-2-fluorophenyl}-2,4,6-trifluorobenzamide (0.035 g, 50% yield). Mp = 190-193°C, LC-MS (Method B, Negative) RT 2.09 (639, 639, 640, M-H$^+$), $^1$H NMR (400 MHz, $CDCl_3$): 8.68 (t, 1H), 8.05 (d, 1H), 7.93 (m, 2H), 7.63 (s, 2H), 7.53 (d, 1H), 7.40(t, 1H), 7.30 (sb, 1H), 7.12 (m, 1H), 6.83 (t, 2H) ppm.

Analogous procedures were used to prepare the following compounds: N-{3-[2,6-Dichloro-4-(2,2,2-trifluoro-1-fluoro-1-thiophen-3-ylethyl)-phenylcarbamoyl]-2-fluorophenyl}-2,4,6-trifluorobenzamide (compound A5). Mp = 197-199°C, LC-MS (Method B, Positive) RT 2.08 (637, 639, M-H$^+$), $^1$H NMR (400 MHz, $CDCl_3$): 8.65 (t, 1H), 8.05 (d, 1H), 7.99-7.92 (m, 2H), 7.57 (s, 3H), 7.42 (m, 1H), 7.38(t, 1H), 7.12 (m, 1H), 6.84 (t, 2H) ppm.

Analogous procedures were used to prepare the compounds A29 to A38 of Table A by changing the organometallic in step 4.

Example IV: Preparation of N-{3-[2,6-dichloro-4-(2,2,2-trifluoro-1-fluoro-1-(4-chlorophenyl)ethyl)phenylcarbamoyl]-4-cyanophenyl}-2-chloro-4-fluorobenzamide

Step 1: Preparation of 2,6-dichloro-4-[1-(4-chlorophenyl)-1,2,2,2-tetrafluoroethyl]-phenylamine

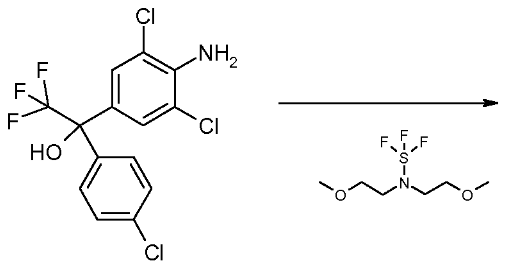

To a precooled solution (0°C) of 1-(4-amino-3,5-dichlorophenyl)-1-(4-chloro-phenyl)-2,2,2-trifluoroethanol (Example II, Step 2, 1.93 g, 5.20 mmol) in anhydrous tetrahydrofuran (100 ml) was added Deoxy-Fluor (1.381g, 1.327 mL, 6.24 mmol, solution 50% in THF). The reaction mixture was stirred for 1 hour at 0°C, then, as the reaction was not finished, 1.33 mL of Deoxy-Fluor was added. The reaction mixture was stirred for 1 hour at 0°C. The mixture reaction was quenched with water. The organic and aqeous phases were separated. The aqueous phase was extracted twice with dichloromethane. The combined organic extracts were dried over sodium sulfate, filtered and concentrated, after addition of 5 mL of SiOH 60. The residue was purified by column chromatography on silica gel (ethyl acetate / cyclohexane 1:5) to give 2,6-dichloro-4-[1-(4-chlorophenyl)-1,2,2,2-tetrafluoro-ethyl]phenylamine (1.830 g, 95% yield). $^1$H NMR (400 MHz, $CDCl_3$): 7.45 (m, 4H), 7.25 (m, 2H), 4.68 (sb, 2H) ppm.

Step 2: Preparation of 4-Cyano-N-{2,6-dichloro-4-[1-(4-chloro-phenyl)-1,2,2,2-tetrafluoro-ethyl]-phenyl} -3-nitro-benzamide

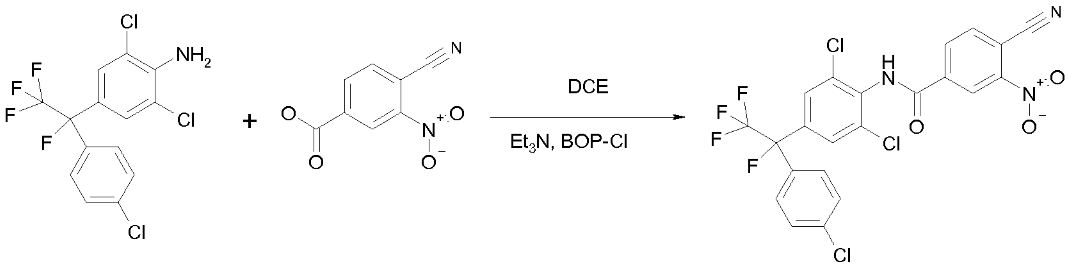


To a suspension of 2,6-dichloro-4-[1-(4-chlorophenyl)-1,2,2,2-tetrafluoroethyl]-phenylamine (Example IV, Step 1) (1.863 g, 5.0 mmol) in 1,2-dichloroethane (50 ml) was added triethylamine (3.49 mL, 25 mmol), followed by 4-cyano-3-nitrobenzoic acid (Prepared as described in WO 2008/074427) (1.729 g, 9.0 mmol) and bis(2-oxo-3-oxazolidinyl)-phosphonic chloride ("BOP-Cl") (3.82 g, 15.0 mmol). The reaction mixture was stirred overnight at 110°C. The reaction was quenched by addition of aqueous sodium hydrogen carbonate and ethyl acetate. The phases were separated. The aqueous phase was extracted twice with ethyl acetate then, the organic phase were combinated. The organic phase was washed with aqueous sodium hydrogen carbonate (saturated), water and brine. The organic phase was dried over sodium sulfate and concentrated. The residue was purified by column chromatography on silica gel (ethyl acetate / cyclohexane 1:5 to 1:4) to give 4-cyano-N-{2,6-dichloro-4-[1-(4-chlorophenyl)-1,2,2,2-tetrafluoroethyl]phenyl}-3-nitrobenzamide (1.70 g, 62 % yield). LC-MS (Method A, Negative) RT 2.14 (544, 546, 548 M-$H^+$).

Step 3: Preparation of 3-amino-4-cyano-N-{2,6-dichloro-4-[1-(4-chlorophenyl)-1,2,2,2-tetrafluoroethyl]phenyl}benzamide

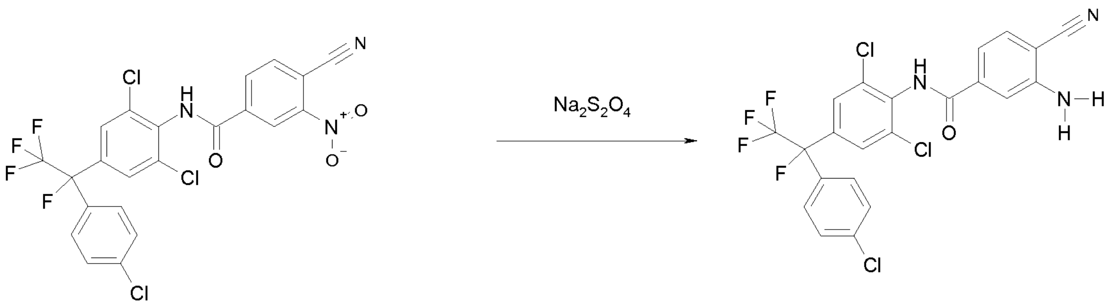


The reduction of the nitro group was realized as described in Example II, Step 5 to give 3-amino-4-cyano-N-{2,6-dichloro-4-[1-(4-chlorophenyl)-1,2,2,2-tetrafluoroethyl]phenyl}-benzamide (71% yield). LC-MS (Method A, Negative) RT 2.05 (514, 516, M-$H^+$).

Step 4: Preparation of N-{3-[2,6-dichloro-4-(2,2,2-trifluoro-1-fluoro-1-(4-chlorophenyl)-ethyl)phenylcarbamoyl]-4-cyanophenyl}-2-chloro-4-fluorobenzamide (compound A6)

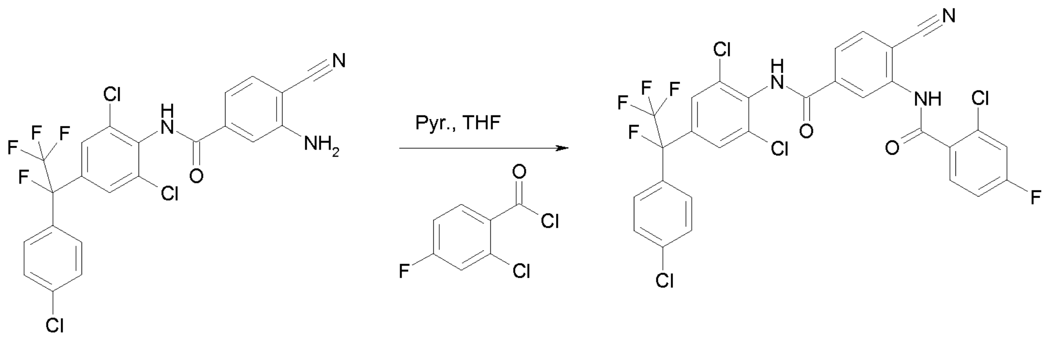

The N-acylation of the amino group was realized as described in Example II, Step 7 to give the (65% yield). LC-MS (Method A, Negative) RT 2.16 (670, 672, 673.6, M-H$^+$).

Analogous procedures were used to prepare the following compounds:

N-{3-[2,6-Dichloro-4-(2,2,2-trifluoro-1-fluoro-1-(4-chlorophenyl)ethyl)-phenylcarbamoyl]-4-cyanophenyl}-2-methyl-4-cyanobenzamide(compound A7).

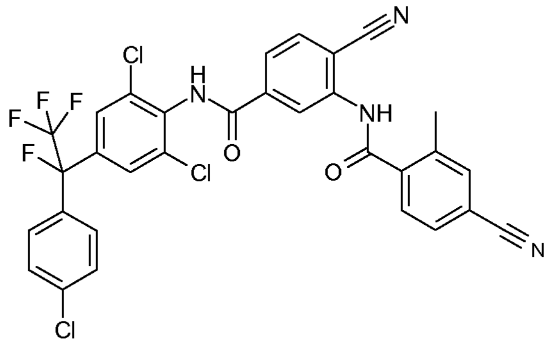

Mp = 138-140°C, LC-MS (Method B, Negative) RT 2.14 (658, M-H$^+$).

Analogous procedures were used to prepare the following compounds:

N-{3-[2,6-Dichloro-4-(2,2,2-trifluoro-1-fluoro-1-(4-chlorophenyl)ethyl)phenylcarbamoyl]-4-cyanophenyl}-2,4,6-trifluorobenzamide(compound A8).

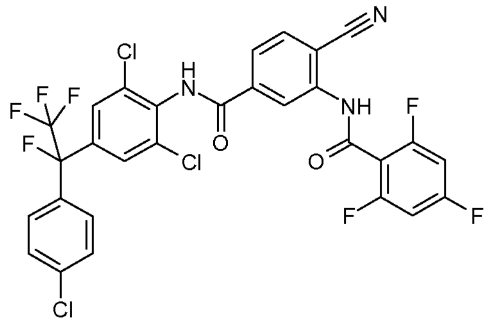

Mp = 169-172°C, LC-MS (Method B, Negatif) RT 2.16 (672, M-H$^+$).

<u>Example V: Preparation of benzamides from acid chlorides which are amenable to parallel synthesis</u>

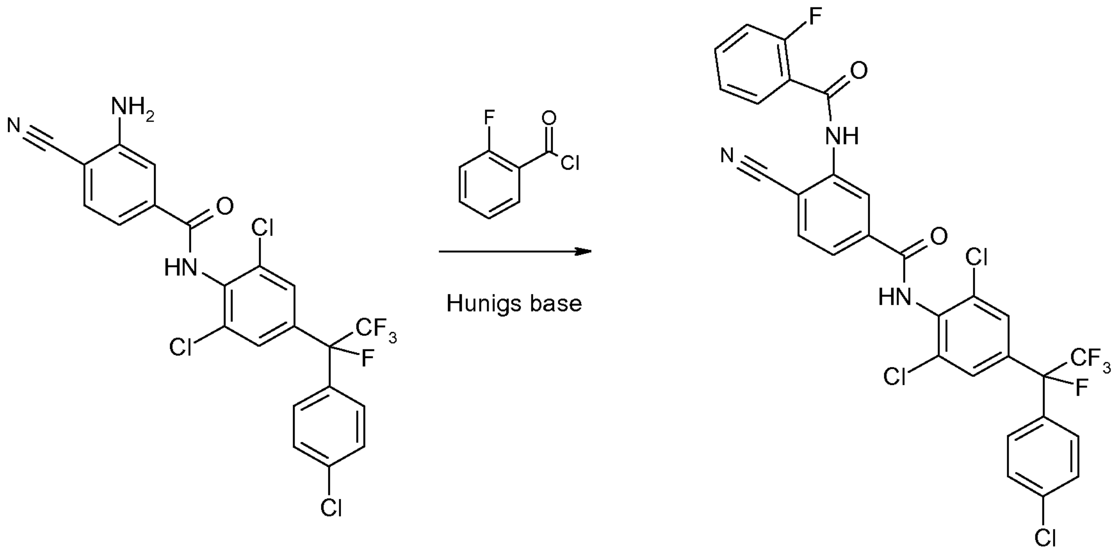


Solution A was prepared by dissolving the amino derivative (0.78 mmol), for example : 4-cyano-N-{2,6-dichloro-4-[1-(4-chlorophenyl)-1,2,2,2-tetrafluoroethyl]phenyl}-3-(2-fluorobenzoylamino)benzamide (Example IV) in the case of Compound Nos. A9 to A28 of Table A, in toluene (15.6 ml). Solution B was prepared by dissolving the acid chloride (45 μmol), for example 2-fluorobenzoyl chloride in the case of Compound No. A9 of Table A, in toluene (0.36 ml). Then Solution A (0.6 ml, 30 μmol) was put in a well and Solution B (0.36 ml, 45 μmol) and diisopropylethylamine ("Hunig's Base") (30 μl, 150 μmol) were added successively to the well. The reaction mixture was stirred at 80°C for 16 hours. The solvent was evaporated and the mixture was diluted with a mixture of acetonitrile (0.6 ml) and *N,N* dimethylacetamide (0.2 ml) and then purified by HPLC to give the desired compound.

This method was used to prepare compounds A9 to A28 of Table A in parallel.

Table A: Compounds of formula (Ia):

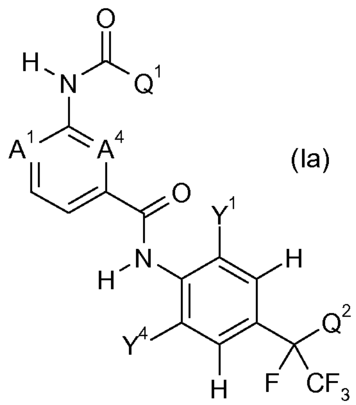

(Ia)

| No | $Y^1$ | $Y^4$ | $A^1$ | $A^4$ | $Q^2$ | $Q^1$ | RT / $MH^+$ |
|---|---|---|---|---|---|---|---|
| A1 | Me | Me | C-H | C-H | p-trifluoromethylphenyl | p-fluorophenyl | LC-MS (Method B) RT 2.15 (607, MH+, 629, M+Na+) |
| A2 | Me | Me | C-H | C-H | p-trifluoromethylphenyl | 2,3-difluorophenyl | LC-MS (Method B) RT 2.16 (625.4, MH+, 647, M+Na+) |
| A3 | Cl | Cl | C-H | COMe | p-chlorophenyl | phenyl | LC-MS (Method B, Positive) RT 2.20 (625, $MH^+$) |
| A4 | Cl | Cl | C-H | C-F | 2-thienyl | 2,4,6-trifluorophenyl | LC-MS (Method B, Negative) RT 2.09 (639, 639, 640, $M-H^+$) |
| A5 | Cl | Cl | C-H | C-F | 3- thienyl | 2,4,6-trifluorophenyl | LC-MS (Method B, Positive) RT 2.08 (637, 639, $M-H^+$) |
| A6 | Cl | Cl | C-CN | C-H | p-chlorophenyl | 2-chloro-4-fluorophenyl | LC-MS (Method A, Negative) RT 2.16 (670, 672, 673.6, $M-H^+$) |
| A7 | Cl | Cl | C-CN | C-H | p-chlorophenyl | 2-methyl-4-cyanophenyl | LC-MS (Method B, Negative) RT 2.14 (658, $M-H^+$) |
| A8 | Cl | Cl | C-CN | C-H | p-chlorophenyl | 2,4,6-trifluorophenyl | LC-MS (Method B, Negative) RT 2.16 (672, $M-H^+$) |
| A9 | Cl | Cl | C-CN | C-H | p-chlorophenyl | 3-fluorophenyl | LC-MS (Method C, Negative) RT 4.3 (636.25, $M-H^+$) |
| A10 | Cl | Cl | C-CN | C-H | p-chlorophenyl | 2-methylphenyl | LC-MS (Method C, Negative) RT 4.24 (632.31, $M-H^+$) |
| A11 | Cl | Cl | C-CN | C-H | p-chlorophenyl | 2-chlorophenyl | LC-MS (Method C, Negative) RT 4.22 (652.25, $M-H^+$) |
| A12 | Cl | Cl | C-CN | C-H | p-chlorophenyl | 4-cyano-phenyl | LC-MS (Method C, Negative) RT 4.09 (643.29, $M-H^+$) |
| A13 | Cl | Cl | C-CN | C-H | p-chlorophenyl | 4-methylphenyl | LC-MS (Method C, Negative) RT 4.31 (632.34, $M-H^+$) |
| A14 | Cl | Cl | C-CN | C-H | p-chlorophenyl | 2-methyl-4-fluorophenyl | LC-MS (Method C, Negative) RT 4.28 (650.33, $M-H^+$) |
| A15 | Cl | Cl | C-CN | C-H | p-chlorophenyl | 3-chloro-6-fluorophenyl | LC-MS (Method C, Negative) RT 4.47 (670.25, $M-H^+$) |
| A16 | Cl | Cl | C-CN | C-H | p-chlorophenyl | 2-chloro-4-nitrophenyl | LC-MS (Method C, Negative) RT 4.26 (697.27, $M-H^+$) |

(continued)

| No | $Y^1$ | $Y^4$ | $A^1$ | $A^4$ | $Q^2$ | $Q^1$ | RT / $MH^+$ |
|---|---|---|---|---|---|---|---|
| A17 | Cl | Cl | C-CN | C-H | p-chlorophenyl | furan-2-yl | LC-MS (Method C, Negative) RT 4.25 (608.3, M-$H^+$) |
| A18 | Cl | Cl | C-CN | C-H | p-chlorophenyl | 4-trifluoromethoxyphenyl | LC-MS (Method C, Negative) RT 4.45 (702.32, M-$H^+$) |
| A19 | Cl | Cl | C-CN | C-H | p-chlorophenyl | 4-fluoro-5-trifluorometh yl-phenyl | LC-MS (Method C, Negatif) RT 4.44 (704.3, M-$H^+$) |
| A20 | Cl | Cl | C-CN | C-H | p-chlorophenyl | 4-trifluoromethylphenyl | LC-MS (Method C, Negative) RT 4.41 (686.34, M-$H^+$) |
| A21 | Cl | Cl | C-CN | C-H | p-chlorophenyl | 2-trifluoromethoxyphenyl | LC-MS (Method C, Negative) RT 4.32 (702.3, M-$H^+$) |
| A22 | Cl | Cl | C-CN | C-H | p-chlorophenyl | 2-methoxy-phenyl | LC-MS (Method C, Negative) RT 4.62 (648.95, M-$H^+$) |
| A23 | Cl | Cl | C-CN | C-H | p-chlorophenyl | phenyl | LC-MS (Method C, Negative) RT 4.18 (618.37, M-$H^+$) |
| A24 | Cl | Cl | C-CN | C-H | p-chlorophenyl | 4-fluorophenyl | LC-MS (Method C, Negative) RT 4.2 (636.38, M-$H^+$) |
| A25 | Cl | Cl | C-CN | C-H | p-chlorophenyl | 2-trifluoromethylphenyl | LC-MS (Method C, Negative) RT 4.26 (686.37, M-$H^+$) |
| A26 | Cl | Cl | C-CN | C-H | p-chlorophenyl | 2,3-difluorophenyl | LC-MS (Method C, Negative) RT 4.29 (654.35, M-$H^+$) |
| A27 | Cl | Cl | C-CN | C-H | p-chlorophenyl | 2,4-difluorophenyl | LC-MS (Method C, Negatif) RT 4.34 (654.36, M-$H^+$) |
| A28 | Cl | Cl | C-CN | C-H | p-chlorophenyl | 2-fluoro-5-trifluoromethylphenyl | LC-MS (Method C, Negative) RT 4.5 (704.3, M-$H^+$) |
| A29 | Cl | Cl | C-H | C-F | 4-methoxy-phenyl | 2,4,6-trifluorophenyl | LC-MS (Method B, Negative) RT 2.1 (661, M-$H^+$) |
| A30 | Cl | Cl | C-H | C-F | 4-trifluormethoxyphenyl | 2,4,6-trifluorophenyl | LC-MS (Method B, Negative) RT 2.19 (715, M-$H^+$) |
| A31 | Cl | Cl | C-H | C-F | 3-methyl-thiophen-2-yl | 2,4,6-trifluorophenyl | LC-MS (Method B, Negative) RT 2.16 (651, M-$H^+$) |
| A32 | Cl | Cl | C-H | C-F | phenyl | 2,4,6-trifluorophenyl | LC-MS (Method B, Negative) RT 2.11 (631, M-$H^+$) |
| A33 | Cl | Cl | C-H | C-F | 3-fluoro-phenyl | 2,4,6-trifluorophenyl | LC-MS (Method B, Negative) RT 2.12 (649, M-$H^+$) |
| A34 | Cl | Cl | C-H | C-F | thiazol-2-yl | 2,4,6-trifluorophenyl | LC-MS (Method B, Negative) RT 2.03 (638, M-$H^+$) |
| A35 | Cl | Cl | C-H | C-F | 3,5-bis-trifluoromethylphenyl | 2,4,6-trifluorophenyl | LC-MS (Method B, Negative) RT 2.22 (767, M-$H^+$) |
| A36 | Cl | Cl | C-H | C-F | 4-fluorophenyl | 2,4,6-trifluorophenyl | LC-MS (Method B, Negative) RT 2.1 (649, M-$H^+$) |
| A37 | Cl | Cl | C-H | C-F | 4-chlorophenyl | 2,4,6-trifluorophenyl | LC-MS (Method B, Negative) RT 2.18 (665, M-$H^+$) |
| A38 | Cl | Cl | C-H | C-F | 3,5-dichlorophenyl | 2,4,6-trifluorophenyl | LC-MS (Method B, Negative) RT 2.25 (701, M-$H^+$) |

Biological examples

This illustrates the pesticidal/insecticidal properties of compounds of formula (I). The tests were performed as follows:

*Spodoptera littoralis* (Egyptian cotton leafworm):

Cotton leaf discs were placed on agar in a 24-well microtiter plate and sprayed with test solutions at an application rate of 200 ppm. After drying, the leaf discs were infested with 5 L1 larvae. The samples were checked for mortality, feeding behavior and growth regulation 3 days after treatment (DAT). The following compound gave at least 80% control of *Spodoptera littoralis:* A2, A3, A6 to A12, A14 to A17, A19, A21, A23 to A27, A30, A37.

*Heliothis virescens* (Tobacco budworm):

Eggs (0-24 h old) were placed in 24-well microtiter plate on artificial diet and treated with test solutions at an application rate of 200 ppm (concentration in well 18 ppm) by pipetting.

After an incubation period of 4 days, samples were checked for egg mortality, larval mortality and growth regulation. The following compounds gave at least 80% control of *Heliothis virescens:* A1, A2 & A3, A7 to A21, A23 to A28, A30, A36, A37.

*Plutella xylostella* (Diamond back moth):

A 24-well microtiter plate (MTP) with artificial diet was treated with test solutions at an application rate of 200 ppm (concentration in well 18 ppm) by pipetting. After drying, the MTP's were infested with L2 larvae (7-12 per well). After an incubation period of 6 days, samples were checked for larval mortality and growth regulation. The following compounds gave at least 80% control of *Plutella xylostella:* A1, A2 , A3, A7 to A18, A21, A23 to A27, A29, A30, A36, A37

*Diabrotica balteata* (Corn root worm):

A 24-well microtiter plate (MTP) with artificial diet was treated with test solutions at an application rate of 200 ppm (concentration in well 18 ppm) by pipetting. After drying, the MTP's were infested with L2 larvae (6-10 per well). After an incubation period of 5 days, samples were checked for larval mortality and growth regulation. The following compounds gave at least 80% control of *Diabrotica balteata:* A1, A2, A3, A6 to A21, A23 to A27, A30, A32, A33, A34, A36, A37.

*Thrips tabaci* (Onion thrips):

Sunflower leaf discs were placed on agar in a 24-well microtiter plate and sprayed with test solutions at an application rate of 200 ppm. After drying, the leaf discs were infested with an aphid population of mixed ages. After an incubation period of 7 days, samples were checked for mortality. The following compound gave at least 80% control of *Thrips tabaci:* A2, A6 to A14, A16, A18, A20, A23 to A27, A32, A33, A30, A34, A36, A37.

*Myzus persicae* (Green peach aphid):

Sunflower leaf discs were placed on agar in a 24-well microtiter plate and sprayed with test solutions at an application rate of 200 ppm. After drying, the leaf discs were infested with an aphid population of mixed ages. After an incubation period of 6 DAT, samples were checked for mortality. The following compound gave at least 80% control of *Myzus persicae:* A3.

*Tetranychus urticae* (Two-spotted spider mite):

Bean leaf discs on agar in 24-well microtiter plates were sprayed with test solutions at an application rate of 200 ppm. After drying, the leaf discs were infested with mite populations of mixed ages. 8 days later, discs were checked for egg mortality, larval mortality, and adult mortality. The following compound gave at least 80% control of *Tetranychus urticae*: A3, A6 to A8, A10 to A12, A14, A16, A20, A23, A24, A28, A27, A32, A36.

## Claims

**1.** A compound of formula (I)

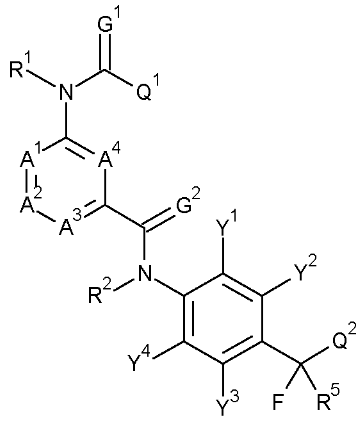

(I)

wherein

$A^1$, $A^2$, $A^3$ and $A^4$ are each independently C-X or nitrogen, wherein each X may be the same or different, and provided that no more than two of $A^1$, $A^2$, $A^3$ and $A^4$ are nitrogen;
X is hydrogen, halogen, cyano, $C_1$-$C_4$alkyl, $C_1$-$C_4$haloalkyl or $C_1$-$C_4$alkoxy;
$G^1$ and $G^2$ are each independently oxygen or sulfur;
$Y^1$ and $Y^4$ are each independently halogen, cyano, $C_1$-$C_4$alkyl, $C_1$-$C_4$haloalkyl, $C_1$-$C_4$alkoxy-$C_1$-$C_4$alkyl, $C_1$-$C_3$alkylthio, $C_1$-$C_3$haloalkylthio, $C_1$-$C_3$alkylsulfinyl, $C_1$-$C_3$haloalkylsulfinyl, $C_1$-$C_3$alkylsulfonyl or $C_1$-$C_3$haloalkylsulfonyl;
$Y^2$ and $Y^3$ are each independently hydrogen, halogen or $C_1$-$C_4$alkyl;
$Q^1$ is aryl or heterocyclyl, each optionally substituted by one to five $R^3$ substituents, which may be the same or different;
$Q^2$ is aryl or heterocyclyl, each optionally substituted by one to five $R^4$ substituents, which may be the same or different;
$R^1$ and $R^2$ are each independently hydrogen, $C_1$-$C_4$alkyl or $C_1$-$C_4$alkylcarbonyl;
$R^3$ is halogen, cyano, nitro, amino, $C_1$-$C_4$alkyl, $C_1$-$C_4$haloalkyl, $C_2$-$C_4$alkenyl, $C_2$-$C_4$haloalkenyl, $C_2$-$C_4$alkynyl, $C_2$-$C_4$haloalkynyl, $C_3$-$C_8$cycloalkyl, $C_3$-$C_8$halocycloalkyl, hydroxy, $C_1$-$C_3$alkoxy, $C_1$-$C_3$haloalkoxy, $C_1$-$C_3$alkylthio, $C_1$-$C_3$haloalkylthio, $C_1$-$C_3$alkylsulfinyl, $C_1$-$C_3$haloalkylsulfinyl, $C_1$-$C_3$alkylsulfonyl, $C_1$-$C_3$haloalkylsulfonyl, *N*-$C_1$-$C_4$alkylamino, *N,N*-di-($C_1$-$C_4$alkyl)amino, $C_1$-$C_4$alkylcarbonyl, $C_1$-$C_4$alkylcarbonyloxy, $C_1$-$C_4$alkoxycarbonyl, $C_1$-$C_4$alkylcarbonylamino, arylcarbonylamino or phenyl;
$R^4$ is halogen, cyano, nitro, $C_1$-$C_4$alkyl, $C_1$-$C_4$haloalkyl, $C_2$-$C_4$alkenyl, $C_2$-$C_4$haloalkenyl, $C_2$-$C_4$alkynyl, $C_2$-$C_4$haloalkynyl, $C_3$-$C_8$cycloalkyl, $C_3$-$C_8$halocycloalkyl, hydroxy, $C_1$-$C_3$alkoxy, $C_1$-$C_3$haloalkoxy, $C_1$-$C_3$alkylthio, $C_1$-$C_3$haloalkylthio, $C_1$-$C_3$alkylsulfinyl, $C_1$-$C_3$haloalkylsulfinyl, $C_1$-$C_3$alkylsulfonyl, $C_1$-$C_3$haloalkylsulfonyl, amino, $C_1$-$C_4$alkylamino, *N,N*-di-($C_1$-$C_4$alkyl)amino, carboxyl (-COOH), $C_1$-$C_4$alkoxycarbonyl, $C_1$-$C_4$alkylcarbonylamino, N-$C_1$-$C_4$alkylaminocarbonyl, N,N-di($C_1$-$C_4$alkyl)aminocarbonyl; and
$R^5$ is $C_1$-$C_6$perfluoroalkyl;

or a salt or *N*-oxide thereof;
provided that the compound is not

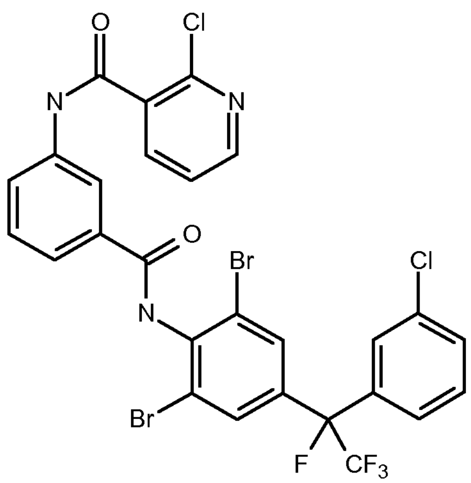

or

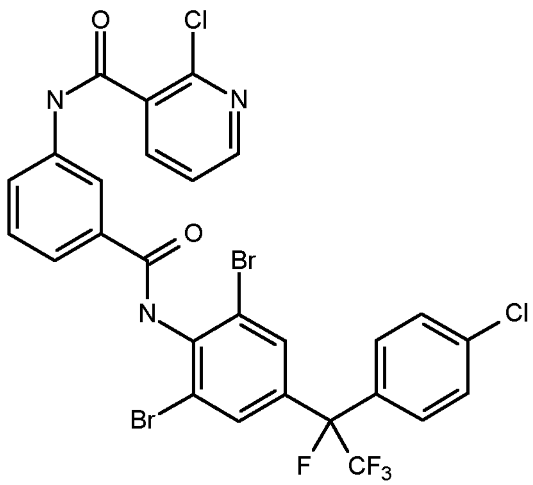

**2.** A compound according to claim 1 wherein $A^1$, $A^2$, $A^3$ and $A^4$ are C-X and each X is independently selected from hydrogen, halogen, cyano, methyl, trifluoromethyl and methoxy.

**3.** A compound according to either claim 1 or claim 2 wherein each X is independently selected from hydrogen, fluoro, cyano and methoxy.

**4.** A compound according to any one of claims 1 to 3 wherein $R^1$ is hydrogen, methyl, ethyl or acetyl.

**5.** A compound according to any one of claims 1 to 4 wherein $R^2$ is hydrogen, methyl, ethyl or acetyl.

**6.** A compound according to any one of claims 1 to 5 wherein $R^5$ is trifluoromethyl.

**7.** A compound according to any one of claims 1 to 6 wherein $G^1$ is oxygen.

**8.** A compound according to any one of claims 1 to 7 wherein $G^2$ is oxygen.

**9.** A compound according to any one of claims 1 to 8 wherein $Q^1$ is phenyl, pyridyl, furanyl, thiophenyl, pyrazolyl or 1,2,3-thiadiazolyl; each optionally substituted by one to four substituents independently selected from cyano, nitro, hydroxy, bromo, chloro, fluoro, methyl, trifluoromethyl, methoxy, trifluoromethoxy, methylthio, methylsulfinyl, methylsulfonyl and phenyl.

**10.** A compound according to claim 9 wherein $Q^1$ is phenyl or furan-2-yl; each optionally substituted by one, two or three substituents independently selected from cyano, nitro, chloro, fluoro, methyl, trifluoromethyl, methoxy and trifluoromethoxy.

**11.** A compound according to any one of claims 1 to 10 wherein $Q^2$ is phenyl, pyridyl, furanyl, thiophenyl or thiazolyl; each optionally substituted by one to three substituents independently selected from chloro, fluoro, methyl, trifluoromethyl, methoxy and trifluoromethoxy.

**12.** A compound according to claim 11 wherein $Q^2$ is phenyl or thiophenyl; each optionally substituted by one, two or three substituents independently selected from chloro, fluoro, methyl, trifluoromethyl, methoxy and trifluoromethoxy.

**13.** A compound according to any one of claims 1 to 12 wherein $Y^1$ and $Y^4$ are each independently selected from bromo, chloro, cyano, methyl, ethyl, methoxymethyl, trifluoromethyl, trifluoromethylthio, trifluoromethylsulfinyl or trifluoromethylsulfonyl.

**14.** A compound according to claim 13 wherein $Y^1$ and $Y^4$ are each independently chloro or methyl.

**15.** A compound according to any one of claims 1 to 14 wherein $Y^2$ and $Y^3$ are hydrogen.

**16.** A compound of formula (III)

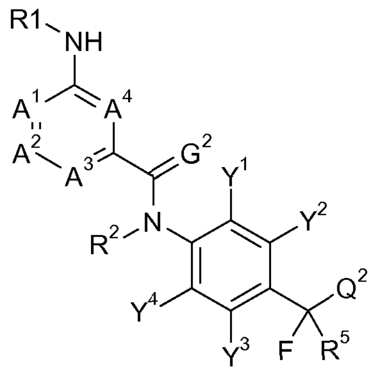

(III)

wherein $A^1$, $A^2$, $A^3$, $A^4$, $G^2$, $Y^1$, $Y^2$, $Y^3$, $Y^4$, $Q^2$, $R^1$, $R^2$ and $R^5$ are as defined in defined in claim 1; or a salt or *N*-oxide thereof; or
a compound of formula (II')

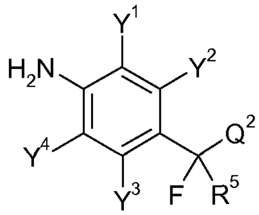

(II')

wherein $R^5$, $Q^2$, $Y^1$, $Y^2$, $Y^3$ and $Y^4$ are as defined in claim 1; or a salt or *N*-oxide thereof; or
a compound of formula (IV)

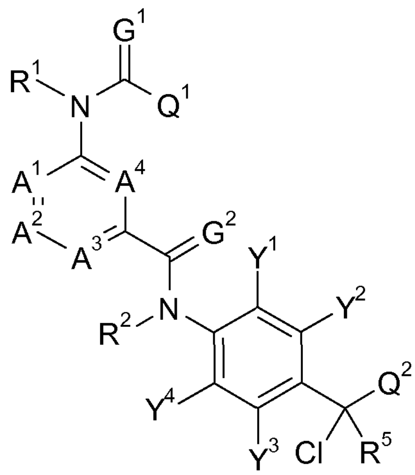

(IV)

$A^1$, $A^2$, $A^3$, $A^4$, $G^1$, $G^2$, $Y^1$, $Y^2$, $Y^3$, $Y^4$, $Q^1$, $Q^2$, $R^1$, $R^2$ and $R^5$ are as defined in claim 1; or a salt or *N*-oxide thereof; provided that the compound or formula (IV) is not

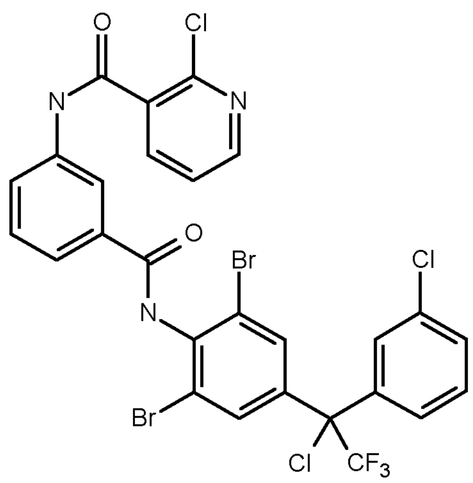

or

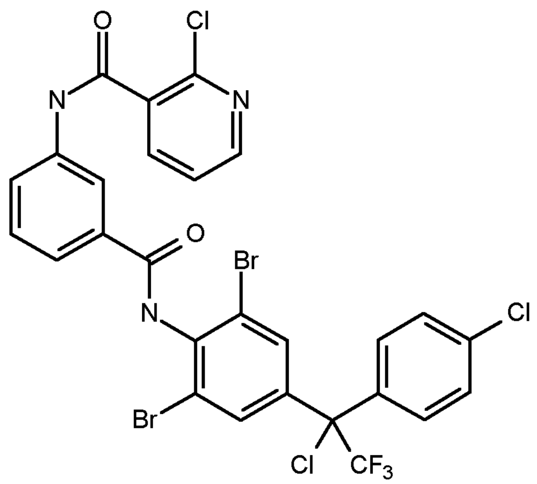

**17.** A method of combating and controlling insects, acarines, nematodes or molluscs which comprises applying to a pest, to a locus of a pest, or to a plant susceptible to attack by a pest an insecticidally, acaricidally, nematicidally or molluscicidally effective amount of a compound of formula (I) as defined in any one of claims 1 to 15.

**18.** An insecticidal, acaricidal, nematicidal or molluscicidal composition comprising an insecticidally, acaricidally, nematicidally or molluscicidally effective amount of a compound of formula (I) as defined in any one of claims 1 to 15.

## Patentansprüche

**1.** Verbindung der Formel (I)

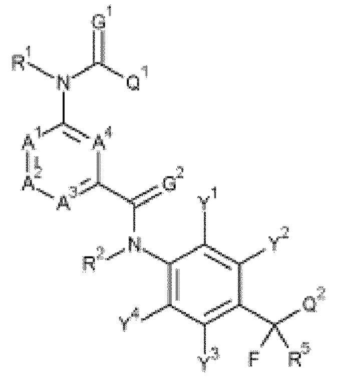

(I)

,

wobei

$A^1$, $A^2$, $A^3$ und $A^4$ jeweils unabhängig voneinander für C-X oder Stickstoff stehen, wobei X jeweils gleich oder verschieden sein kann, mit der Maßgabe, dass nicht mehr als zwei von $A^1$, $A^2$, $A^3$ und $A^4$ für Stickstoff stehen,
X für Wasserstoff, Halogen, Cyano, $C_1$-$C_4$-Alkyl, $C_1$-$C_4$-Halogenalkyl oder $C_1$-$C_4$-Alkoxy steht,
$G^1$ und $G^2$ jeweils unabhängig voneinander für Sauerstoff oder Schwefel stehen,
$Y^1$ und $Y^4$ jeweils unabhängig voneinander für Halogen, Cyano, $C_1$-$C_4$-Alkyl, $C_1$-$C_4$-Halogenalkyl, $C_1$-$C_4$-Alkoxy-$C_1$-$C_4$-alkyl, $C_1$-$C_3$-Alkylthio, $C_1$-$C_3$-Halogenalkylthio, $C_1$-$C_3$-Alkylsulfinyl, $C_1$-$C_3$-Halogenalkylsulfinyl, $C_1$-$C_3$-Alkylsulfonyl oder $C_1$-$C_3$-Halogenalkylsulfonyl stehen,
$Y^2$ und $Y^3$ jeweils unabhängig voneinander für Wasserstoff, Halogen oder $C_1$-$C_4$-Alkyl stehen,
$Q^1$ für Aryl oder Heterocyclyl steht, jeweils gegebenenfalls substituiert durch einen bis fünf $R^3$-Substituenten, die gleich oder verschieden sein können,
$Q^2$ für Aryl oder Heterocyclyl steht, jeweils gegebenenfalls substituiert durch einen bis fünf $R^4$-Substituenten, die gleich oder verschieden sein können,
$R^1$ und $R^2$ jeweils unabhängig voneinander für Wasserstoff, $C_1$-$C_4$-Alkyl oder $C_1$-$C_4$-Alkylcarbonyl stehen,

$R^3$ für Halogen, Cyano, Nitro, Amino, $C_1$-$C_4$-Alkyl, $C_1$-$C_4$-Halogenalkyl, $C_2$-$C_4$-Alkenyl, $C_2$-$C_4$-Halogenalkenyl, $C_2$-$C_4$-Alkinyl, $C_2$-$C_4$-Halogenalkinyl, $C_3$-$C_8$-Cycloalkyl, $C_3$-$C_8$-Halogencycloalkyl, Hydroxy, $C_1$-$C_3$-Alkoxy, $C_1$-$C_3$-Halogenalkoxy, $C_1$-$C_3$-Alkylthio, $C_1$-$C_3$-Halogenalkylthio, $C_1$-$C_3$-Alkylsulfinyl, $C_1$-$C_3$-Halogenalkylsulfinyl, $C_1$-$C_3$-Alkylsulfonyl, $C_1$-$C_3$-Halogenalkylsulfonyl, *N*-$C_1$-$C_4$-Alkylamino, *N,N*-Di-($C_1$-$C_4$-alkyl)amino, $C_1$-$C_4$-Alkylcarbonyl, $C_1$-$C_4$-Alkylcarbonyloxy, $C_1$-$C_4$-Alkoxycarbonyl, $C_1$-$C_4$-Alkylcarbonylamino, Arylcarbonylamino oder Phenyl steht,

$R^4$ für Halogen, Cyano, Nitro, $C_1$-$C_4$-Alkyl, $C_1$-$C_4$-Halogenalkyl, $C_2$-$C_4$-Alkenyl, $C_2$-$C_4$-Halogenalkenyl, $C_2$-$C_4$-Alkinyl, $C_2$-$C_4$-Halogenalkinyl, $C_3$-$C_8$-Cycloalkyl, $C_3$-$C_8$-Halogencycloalkyl, Hydroxy, $C_1$-$C_3$-Alkoxy, $C_1$-$C_3$-Halogenalkoxy, $C_1$-$C_3$-Alkylthio, $C_1$-$C_3$-Halogenalkylthio, $C_1$-$C_3$-Alkylsulfinyl, $C_1$-$C_3$-Halogenalkylsulfinyl, $C_1$-$C_3$-Alkylsulfonyl, $C_1$-$C_3$-Halogenalkylsulfonyl, Amino, $C_1$-$C_4$-Alkylamino, *N,N*-Di-($C_1$-$C_4$-alkyl)amino, Carboxyl(-CO-OH), $C_1$-$C_4$-Alkoxycarbonyl, $C_1$-$C_4$-Alkylcarbonylamino, *N*-$C_1$-$C_4$-Alkylaminocarbonyl, *N,N*-Di-($C_1$-$C_4$-alkyl)aminocarbonyl steht und

$R^5$ für $C_1$-$C_6$-Perfluoralkyl steht,

oder ein Salz oder N-Oxid davon,
mit der Maßgabe, dass es sich bei der Verbindung nicht um

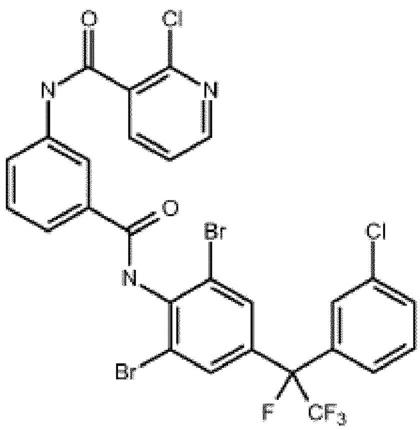

oder

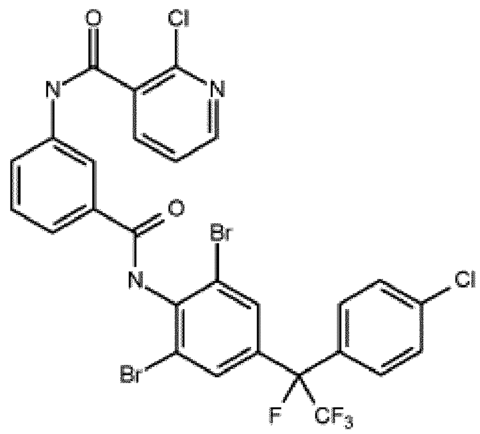

handelt.

**2.** Verbindung nach Anspruch 1, wobei $A^1$, $A^2$, $A^3$ und $A^4$ für C-X stehen und X jeweils unabhängig aus Wasserstoff, Halogen, Cyano, Methyl, Trifluormethyl und Methoxy ausgewählt ist.

**3.** Verbindung nach Anspruch 1 oder 2, wobei X jeweils unabhängig aus Wasserstoff, Fluor, Cyano und Methoxy ausgewählt ist.

**4.** Verbindung nach einem der Ansprüche 1 bis 3, wobei $R^1$ für Wasserstoff, Methyl, Ethyl oder Acetyl steht.

**5.** Verbindung nach einem der Ansprüche 1 bis 4, wobei $R^2$ für Wasserstoff, Methyl, Ethyl oder Acetyl steht.

**6.** Verbindung nach einem der Ansprüche 1 bis 5, wobei $R^5$ für Trifluormethyl steht.

**7.** Verbindung nach einem der Ansprüche 1 bis 6, wobei $G^1$ für Sauerstoff steht.

**8.** Verbindung nach einem der Ansprüche 1 bis 7, wobei $G^2$ für Sauerstoff steht.

**9.** Verbindung nach einem der Ansprüche 1 bis 8, wobei $Q^1$ für Phenyl, Pyridyl, Furanyl, Thiophenyl, Pyrazolyl oder 1,2,3-Thiadiazolyl steht, jeweils gegebenenfalls substituiert durch einen bis vier Substituenten unabhängig voneinander ausgewählt aus Cyano, Nitro, Hydroxy, Brom, Chlor, Fluor, Methyl, Trifluormethyl, Methoxy, Trifluormethoxy, Methylthio, Methylsulfinyl, Methylsulfonyl und Phenyl.

**10.** Verbindung nach Anspruch 9, wobei $Q^1$ für Phenyl oder Furan-2-yl steht, jeweils gegebenenfalls substituiert durch

einen, zwei oder drei Substituenten unabhängig voneinander ausgewählt aus Cyano, Nitro, Chlor, Fluor, Methyl, Trifluormethyl, Methoxy und Trifluormethoxy.

**11.** Verbindung nach einem der Ansprüche 1 bis 10, wobei $Q^2$ für Phenyl, Pyridyl, Furanyl, Thiophenyl oder Thiazolyl steht, jeweils gegebenenfalls substituiert durch einen bis drei Substituenten unabhängig voneinander ausgewählt aus Chlor, Fluor, Methyl, Trifluormethyl, Methoxy und Trifluormethoxy.

**12.** Verbindung nach Anspruch 11, wobei $Q^2$ für Phenyl oder Thiophenyl steht, jeweils gegebenenfalls substituiert durch einen, zwei oder drei Substituenten unabhängig voneinander ausgewählt aus Chlor, Fluor, Methyl, Trifluormethyl, Methoxy und Trifluormethoxy.

**13.** Verbindung nach einem der Ansprüche 1 bis 12, wobei $Y^1$ und $Y^4$ jeweils unabhängig voneinander aus Brom, Chlor, Cyano, Methyl, Ethyl, Methoxymethyl, Trifluormethyl, Trifluormethylthio, Trifluormethylsulfinyl und Trifluormethylsulfonyl ausgewählt sind.

**14.** Verbindung nach Anspruch 13, wobei $Y^1$ und $Y^4$ jeweils unabhängig voneinander für Chlor oder Methyl stehen.

**15.** Verbindung nach einem der Ansprüche 1 bis 14, wobei $Y^2$ und $Y^3$ für Wasserstoff stehen.

**16.** Verbindung der Formel (III)

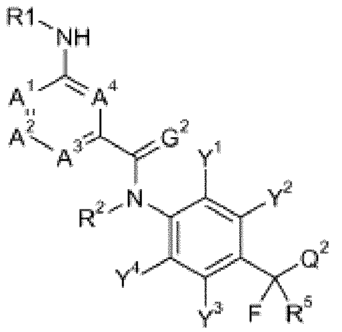

(III)

,

wobei $A^1$, $A^2$, $A^3$, $A^4$, $G^2$, $Y^1$, $Y^2$, $Y^3$, $Y^4$, $Q^2$, $R^1$, $R^2$ und $R^5$ wie in Anspruch 1 definiert sind, oder ein Salz oder N-Oxid davon, oder
eine Verbindung der Formel (II')

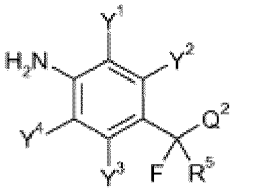

(II')

,

wobei $R^5$, $Q^2$, $Y^1$, $Y^2$, $Y^3$ und $Y^4$ wie in Anspruch 1 definiert sind, oder ein Salz oder N-Oxid davon, oder eine Verbindung der Formel (IV)

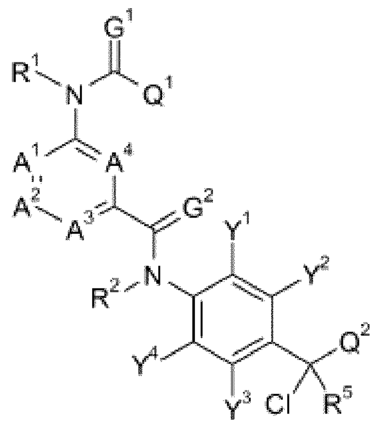

(IV)

,

in welcher $A^1$, $A^2$, $A^3$, $A^4$, $G^1$, $G^2$, $Y^1$, $Y^2$, $Y^3$, $Y^4$, $Q^1$, $Q^2$, $R^1$, $R^2$ und $R^5$ wie in Anspruch 1 definiert sind, oder ein Salz oder N-Oxid davon,
mit der Maßgabe, dass es sich bei der Verbindung der Formel (IV) nicht um

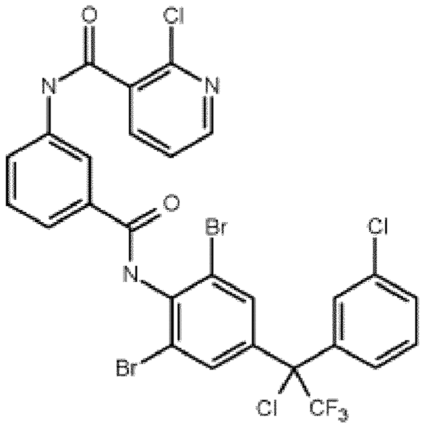

oder

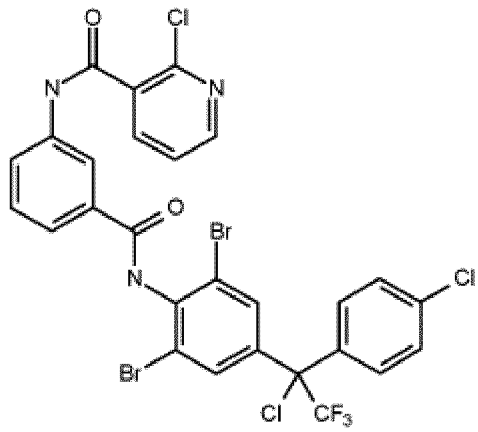

handelt.

**17.** Verfahren zur Bekämpfung und Kontrolle von Insekten, Akarina, Nematoden oder Mollusken, bei dem man eine insektizid, akarizid, nematizid bzw. molluskizid wirksame Menge einer wie in einem der Ansprüche 1 bis 15 definierten Verbindung der Formel (I) auf einen Schädling, einen Lebensraum eines Schädlings oder eine gegenüber Befall durch einen Schädling empfindliche Pflanze aufbringt.

**18.** Insektizide, akarizide, nematizide oder molluskizide Zusammensetzung, umfassend eine insektizid, akarizid, nematizid bzw. molluskizid wirksame Menge einer wie in einem der Ansprüche 1 bis 15 definierten Verbindung der Formel (I).

## Revendications

**1.** Composé de formule (I)

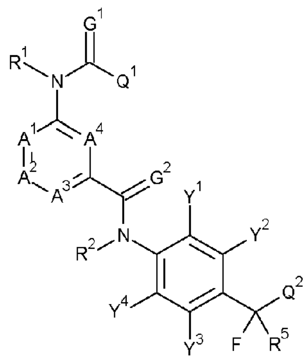

(I)

dans lequel

$A^1$, $A^2$, $A^3$, et $A^4$ sont chacun indépendamment C-X ou azote, où chaque X peut être identique ou différent, et à condition que pas plus de deux parmi $A^1$, $A^2$, $A^3$ et $A^4$ soient azote ;
X est hydrogène, halogène, cyano, $C_1$-$C_4$alkyle, $C_1$-$C_4$halogénoalkyle ou $C_1$-$C_4$alcoxy ;
$G^1$ et $G^2$ sont chacun indépendamment oxygène ou soufre ;
$Y^1$ et $Y^4$ sont chacun indépendamment halogène, cyano, $C_1$-$C_4$alkyle, $C_1$-$C_4$halogénoalkyle, $C_1$-$C_4$alcoxy-$C_1$-$C_4$alkyle, $C_1$-$C_3$alkylthio, $C_1$-$C_3$halogénoalkylthio, $C_1$-$C_3$-alkylsulfinyle, $C_1$-$C_3$halogénoalkylsulfinyle, $C_1$-$C_3$-alkylsulfonyle ou $C_1$-$C_3$halogénoalkylsulfonyle ;
$Y^2$ et $Y^3$ sont chacun indépendamment hydrogène, halogène ou $C_1$-$C_4$alkyle ;
$Q^1$ est aryle ou hétérocyclyle, chacun étant éventuellement substitué par de un à cinq substituants $R^3$, qui peuvent être identiques ou différents ;
$Q^2$ est aryle ou hétérocyclyle, chacun étant éventuellement substitué par de un à cinq substituants $R^4$, qui peuvent être identiques ou différents ;
$R^1$ et $R^2$ sont chacun indépendamment hydrogène, $C_1$-$C_4$alkyle ou $C_1$-$C_4$alkylcarbonyle ;
$R^3$ est halogène, cyano, nitro, amino, $C_1$-$C_4$alkyle, $C_1$-$C_4$halogénoalkyle, $C_2$-$C_4$alcényle, $C_2$-$C_4$halogéno-alcényle, $C_2$-$C_4$alcynyle, $C_2$-$C_4$halogénoalcynyle, $C_3$-C8-cycloalkyle, $C_3$-$C_8$halocycloalkyle, hydroxy, $C_1$-$C_3$alcoxy, $C_1$-$C_3$halogénoalcoxy, $C_1$-$C_3$alkylthio, $C_1$-$C_3$halogéno-alkylthio, $C_1$-$C_3$alkylsulfinyle, $C_1$-$C_3$halogéno-alkylsulfinyle, $C_1$-$C_3$alkylsulfonyle, $C_1$-$C_3$halogéno-alkylsulfonyle, N-$C_1$-$C_4$alkylamino, N,N-di-($C_1$-$C_4$alkyl)-amino, $C_1$-$C_4$alkylcarbonyle, $C_1$-$C_4$alkylcarbonyloxy, $C_1$-$C_4$alcoxycarbonyle, $C_1$-$C_4$alkylcarbonylamino, aryl-carbonylamino ou phényle ;
$R^4$ est halogène, cyano, nitro, $C_1$-$C_4$alkyle, $C_1$-$C_4$halogénoalkyle, $C_2$-$C_4$alcényle, $C_2$-$C_4$halogénoalcényle, $C_2$-$C_4$alcynyle, $C_2$-$C_4$halogénoalcynyle, $C_3$-$C_8$cycloalkyle, $C_3$-$C_8$halocycloalkyle, hydroxy, $C_1$-$C_3$alcoxy, $C_1$-$C_3$halogénoalcoxy, $C_1$-$C_3$alkylthio, $C_1$-$C_3$halogénoalkylthio, $C_1$-$C_3$alkylsulfinyle, $C_1$-$C_3$halogénoalkylsulfinyle, $C_1$-$C_3$alkylsulfonyle, $C_1$-$C_3$halogénoalkylsulfonyle, amino, $C_1$-$C_4$alkylamino, N,N-di-($C_1$-$C_4$alkyl)amino, carboxyle (-COOH), $C_1$-$C_4$alcoxycarbonyle, $C_1$-$C_4$alkylcarbonylamino, N-$C_1$-$C_4$alkylaminocarbonyle, N,N-di($C_1$-$C_4$alkyl)-aminocarbonyle ; et
$R^5$ est $C_1$-$C_6$perfluoroalkyle ;

ou un sel ou N-oxyde de celui-ci ;
à condition que le composé ne soit pas

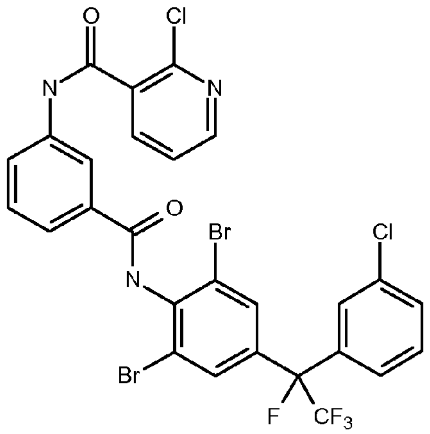

ou

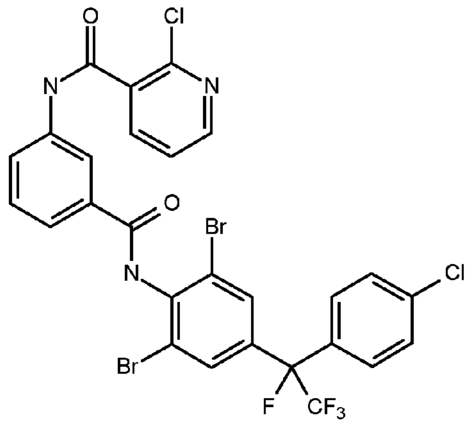

2. Composé selon la revendication 1, dans lequel $A^1$, $A^2$, $A^3$ et $A^4$ sont C-X et chaque X est choisi indépendamment parmi hydrogène, halogène, cyano, méthyle, trifluorométhyle et méthoxy.

3. Composé selon la revendication 1 ou bien la revendication 2, dans lequel chaque X est choisi indépendamment parmi hydrogène, fluoro, cyano et méthoxy.

4. Composé selon l'une quelconque des revendications 1 à 3, dans lequel $R^1$ est hydrogène, méthyle, éthyle ou acétyle.

5. Composé selon l'une quelconque des revendications 1 à 4, dans lequel $R^2$ est hydrogène, méthyle, éthyle ou acétyle.

6. Composé selon l'une quelconque des revendications 1 à 5, dans lequel $R^5$ est trifluorométhyle.

7. Composé selon l'une quelconque des revendications 1 à 6, dans lequel $G^1$ est oxygène.

8. Composé selon l'une quelconque des revendications 1 à 7, dans lequel $G^2$ est oxygène.

9. Composé selon l'une quelconque des revendications 1 à 8, dans lequel $Q^1$ est phényle, pyridyle, furanyle, thiophényle, pyrazolyle ou 1,2,3-thiadiazolyle ; chacun éventuellement substitué par de un à quatre substituants choisis indépendamment parmi cyano, nitro, hydroxy, bromo, chloro, fluoro, méthyle, trifluorométhyle, méthoxy, trifluorométhoxy, méthylthio, méthylsulfinyle, méthylsulfonyle et phényle.

10. Composé selon la revendication 9, dans lequel $Q^1$ est phényle ou furan-2-yle ; chacun éventuellement substitué par un, deux ou trois substituants choisis indépendamment parmi cyano, nitro, chloro, fluoro, méthyle, trifluorométhyle, méthoxy et trifluorométhoxy.

11. Composé selon l'une quelconque des revendications 1 à 10, dans lequel $Q^2$ est phényle, pyridyle, furanyle, thiophényle ou thiazolyle ; chacun éventuellement substitué par de un à trois substituants choisis indépendamment parmi chloro, fluoro, méthyle, trifluorométhyle, méthoxy et trifluorométhoxy.

12. Composé selon la revendication 11, dans lequel $Q^2$ est phényle ou thiophényle ; chacun éventuellement substitué par un, deux ou trois substituants choisis indépendamment parmi chloro, fluoro, méthyle, trifluorométhyle, méthoxy et trifluorométhoxy.

13. Composé selon l'une quelconque des revendications 1 à 12, dans lequel $Y^1$ et $Y^4$ sont choisis chacun indépendamment parmi bromo, chloro, cyano, méthyle, éthyle, méthoxyméthyle, trifluorométhyle, trifluorométhylthio, trifluorométhylsulfinyle ou trifluorométhylsulfonyle.

14. Composé selon la revendication 13, dans lequel $Y^1$ et $Y^4$ sont chacun indépendamment chloro ou méthyle.

15. Composé selon l'une quelconque des revendications 1 à 14, dans lequel $Y^2$ et $Y^3$ sont hydrogène.

16. Composé de formule (III)

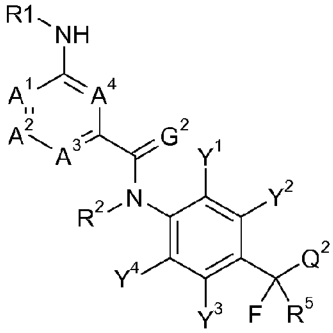

(III)

dans lequel $A^1$, $A^2$, $A^3$, $A^4$, $G^2$, $Y^1$, $Y^2$, $Y^3$, $Y^4$, $Q^2$, $R^1$, $R^2$ et $R^5$ sont tels que définis selon la revendication 1 ; ou un sel ou N-oxyde de celui-ci ; ou
un composé de formule (II')

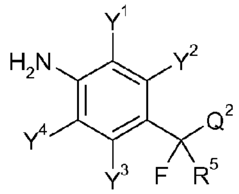

(II')

dans lequel $R^5$, $Q^2$, $Y^1$, $Y^2$, $Y^3$ et $Y^4$ sont tels que définis selon la revendication 1 ;
ou un sel ou N-oxyde de celui-ci ; ou
un composé de formule (IV)

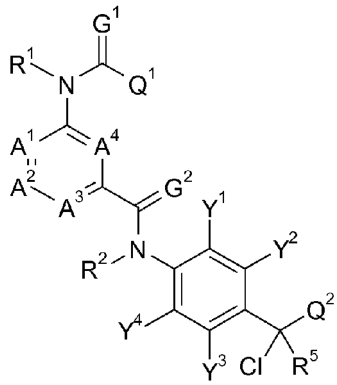

(IV)

$A^1$, $A^2$, $A^3$, $A^4$, $G^1$, $G^2$, $Y^1$, $Y^2$, $Y^3$, $Y^4$, $Q^1$, $Q^2$, $R^1$, $R^2$ et $R^5$ étant tels que définis selon la revendication 1 ;
ou un sel ou N-oxyde de celui-ci ;
à condition que le composé de formule (IV) ne soit pas

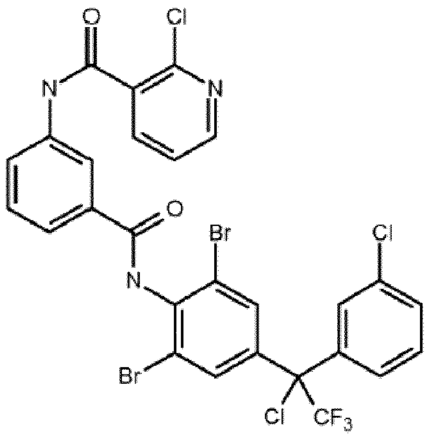

ou

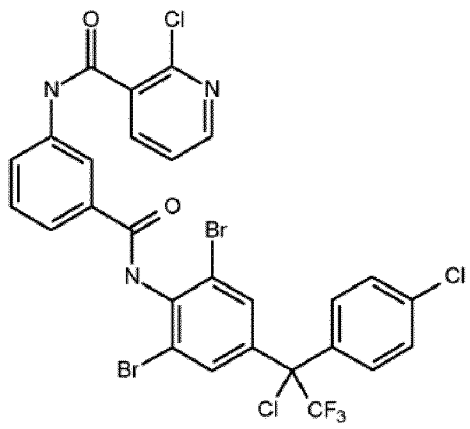

**17.** Méthode de lutte ou de contrôle d'insectes, d'acariens, de nématodes ou de mollusques, comprenant l'application à un nuisible, à un lieu où se développe le nuisible, ou à une plante susceptible d'être attaquée par un nuisible, d'une quantité efficace sur le plan insecticide, acaricide, nématicide ou molluscicide d'un composé de formule (I) tel que défini selon l'une quelconque des revendications 1 à 15.

**18.** Composition insecticide, acaricide, nématicide ou molluscicide comprenant une quantité efficace sur le plan insecticide, acaricide, nématicide ou molluscicide d'un composé de formule (I) tel que défini selon l'une quelconque des revendications 1 à 15.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010015355 A
- EP 1714958 A
- JP 2006306771 B
- WO 06137376 A
- WO 06137395 A
- WO 07017075 A
- WO 2009049845 A
- JP 2008174552 B
- WO 03015518 A
- WO 04067528 A
- WO 06022225 A
- WO 07112844 A
- WO 2008074427 A


### Non-patent literature cited in the description


- *Journal fuer Praktische Chemie (Leipzig),* 1937, vol. 148, 161-9
- *J. Med. Chem.,* 2004, vol. 47 (8), 1969-1986
- *J. Med. Chem.,* 2002, vol. 45 (17), 3692-3702
- *J. Med. Chem.,* 1989, vol. 32 (3), 575-83
- *Bioorganic & Medicinal Chemistry Letters,* 2007, vol. 17 (7), 1908
- The Pesticide Manual. The Pesticide Manual - A World Compendium. The British Crop Protection Council
- The Pesticide Manual. BCPC, 2000